# EUROPEAN PATENT SPECIFICATION

(11) **EP 3 377 176 B1**
(45) Date of publication and mention of the grant of the patent: **20.10.2021**
(21) Application number: 17757069.4
(22) Date of filing: 21.02.2017
(51) Int. Cl.: A61K 31/277, A61K 31/5377, A61P 7/02, A61P 9/12

(54) **METHOD OF TREATING PATIENTS COADMINISTERED RIVAROXABAN AND VERAPAMIL**
VERFAHREN ZUR BEHANDLUNG VON PATIENTEN, DENEN RIVAROXABAN UND VERAPAMIL VERABREICHT WERDEN
MÉTHODE DE TRAITEMENT DE PATIENTS AUXQUELS SONT ADMINISTRÉS CONJOINTEMENT DU RIVAROXABAN ET DU VÉRAPAMIL

(30) Priority: 23.02.2016 US 201662298555 P
(43) Date of publication of application: 26.09.2018
(73) Proprietor: Morgandane Scientific, LLC, Corona Del Mar, CA 92625 (US)
(72) Inventor: PATEL, Maulik, Burlingame, CA 94010 (US); SRINIVASAN, Sundar, Corona Del Mar, CA 92625 (US); CHOW, Christina, Seattle Washington 98112 (US)
(74) Representative: Cooley (UK) LLP
(86) International application number: PCT/US2017/018727
(87) International publication number: WO 2017/147074

(56) References cited:
- US-A1- 2012 095 019
- US-A1- 2012 095 019
- US-A1- 2014 010 876
- TAKI GALANIS ET AL: "Prophylaxis for Deep Vein Thrombosis and Pulmonary Embolism in the Surgical Patient", ADVANCES IN SURGERY, vol. 45, no. 1, 2011, pages 361-390, XP028258567, ISSN: 0065-3411, DOI: 10.1016/J.YASU.2011.05.001 [retrieved on 2011-05-20]
- WOLFGANG MUECK ET AL: "Clinical Pharmacokinetic and Pharmacodynamic Profile of Rivaroxaban", CLINICAL PHARMACOKINETICS., vol. 53, no. 1, 3 September 2013 (2013-09-03), pages 1-16, XP055298782, NZ ISSN: 0312-5963, DOI: 10.1007/s40262-013-0100-7
- TAMAYO SALLY ET AL: "Characterizing major bleeding in patients with nonvalvular atrial fibrillation: a pharmacovigilance study of 27 467 patients taking rivaroxaban.", CLINICAL CARDIOLOGY FEB 2015, vol. 38, no. 2, February 2015 (2015-02), pages 63-68, XP002792066, ISSN: 1932-8737
- MUECK, W et al.: "Clinical pharmacokinetic and pharmacodynamic profile of rivaroxaban", Clinical Pharmacokinetics, 3 September 2013 (2013-09-03), XP055298782,
- TAMAYO, S et al.: "Characterizing major bleeding in patients with nonvalvular atrial fibrillation: a pharmacovigilance study of 27467 patients taking rivaroxaban", Clinical Cardiology, 2015, XP002792066,
- Anonymous: "Verapamil, Wikipedia the free encyclopedia", WIKIPEDIA, 5 June 2019 (2019-06-05), XP055602307, Retrieved from the Internet: URL:https://en.wikipedia.org/wiki/Verapami l
- DATABASE MEDLINE [Online] US NATIONAL LIBRARY OF MEDICINE (NLM), BETHESDA, MD, US; February 2015 (2015-02), TAMAYO SALLY ET AL: "Characterizing major bleeding in patients with nonvalvular atrial fibrillation: a pharmacovigilance study of 27 467 patients taking rivaroxaban.", Database accession no. NLM25588595 & TAMAYO SALLY ET AL: "Characterizing major bleeding in patients with nonvalvular atrial fibrillation: a pharmacovigilance study of 27 467 patients taking rivaroxaban.", CLINICAL CARDIOLOGY FEB 2015, vol. 38, no. 2, February 2015 (2015-02), pages 63-68, ISSN: 1932-8737
- DATABASE MEDLINE [Online] US NATIONAL LIBRARY OF MEDICINE (NLM), BETHESDA, MD, US; January 2014 (2014-01), MUECK WOLFGANG ET AL: "Clinical pharmacokinetic and pharmacodynamic profile of rivaroxaban.", Database accession no. NLM23999929 & MUECK WOLFGANG ET AL: "Clinical pharmacokinetic and pharmacodynamic profile of rivaroxaban.", CLINICAL PHARMACOKINETICS JAN 2014, vol. 53, no. 1, January 2014 (2014-01), pages 1-16, ISSN: 1179-1926

## Description

### FIELD

In various embodiments, the present invention relates to the field of blood coagulation. In particular embodiments, the present invention relates to novel methods of administration and dosing of rivaroxaban in the treatment of patients who require anticoagulant therapy, for example treatment with a Factor Xa inhibitor.

### BACKGROUND

Maintenance of normal haemostasis-between bleeding and thrombosis-is subject to complex regulatory mechanisms. Uncontrolled activation of the coagulant system or defective inhibition of the activation processes may cause formation of local thrombi or embolisms in vessels (arteries, veins) or in heart cavities. This may lead to serious thromboembolic disorders, such as myocardial infarct, angina pectoris (including unstable angina), reocclusions and restenoses after angioplasty or aortocoronary bypass, stroke, transitory ischaemic attacks, peripheral arterial occlusive disorders, pulmonary embolisms or deep venous thromboses.

Anticoagulant drugs target various procoagulant factors in the coagulation pathway. Presently there are numerous anticoagulant drugs which are widely available. One new class of anticoagulant drugs is direct Factor Xa inhibitors, which play a central role in the cascade of blood coagulation by inhibiting Factor Xa directly.

Rivaroxaban is a Factor Xa inhibitor drug used to treat disorders including deep vein thrombosis (DVT) and pulmonary embolism (PE). It is also used to help prevent strokes or serious blood clots in people who have atrial fibrillation.

Use of rivaroxaban, however, presents considerable risk to patients whereby higher than expected blood concentrations of rivaroxaban can cause serious adverse events, most notably elevated rates of internal bleeding/hemorrhage. Conversely, any under dosing of the drug leaves a patient at risk of potentially fatal clotting events. Taki Galanis et al., Advances In Surgery 45:1 (2011) 361-390 is a review article describing the cause and risk factors for venous thromboembolism (VTE) and guidelines for prevention in surgical patients.

Accordingly, it is one object of the present invention to provide novel methods of rivaroxaban dosage control.

### SUMMARY OF THE DISCLOSURE

The invention is defined according to the appended claims. The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

In one embodiment, the invention is directed to rivaroxaban for use in a method of reducing the risk of stroke and systemic embolism in patients with nonvalvular atrial fibrillation comprising administering about 100 to about 480 mg verapamil daily to the patient; and administering a dose of rivaroxaban to the patient which is less than the dose recommended for that patient if the patient was not concomitantly administered verapamil, wherein the dose of rivaroxaban recommended for an otherwise identical patient who is not concomitantly administered verapamil is 20 mg and the method comprises administering a dose of about 10 mg, about 12.5 mg or about 15 mg rivaroxaban to the patient; wherein the patient has a CL_{Cr} of 50-79 mL/min.

In another embodiment, the invention is directed to rivaroxaban for use in a method of reducing the risk of stroke and systemic embolism in patients with nonvalvular atrial fibrillation comprising administering about 100 to about 480 mg verapamil daily to the patient; and administering a dose of rivaroxaban to the patient which is less than the dose recommended for that patient if the patient was not concomitantly administered verapamil, wherein the dose of rivaroxaban recommended for an otherwise identical patient who is not concomitantly administered verapamil is 15 mg and the method comprises administering a dose of about 7.5 mg, about 10 mg or about 12.5 mg rivaroxaban to the patient; wherein the patient has a CL_{Cr} of 15-49 mL/min.

In any of the various embodiments disclosed herein, the maximum prothrombin time of said patient concomitantly administered rivaroxaban and verapamil ranges from about 20 to about 30 seconds.

In any of the various embodiments disclosed herein, the Cₘₐₓ plasma concentration of rivaroxaban for the patient concomitantly administered rivaroxaban and verapamil is no more than about 317 ng/mL.

In any of the various embodiments disclosed herein, the AUC of rivaroxaban for the patient concomitantly administered rivaroxaban and verapamil is no more than about 3792 µg•hr/L.

### BRIEF DESCRIPTIONS OF THE FIGURES

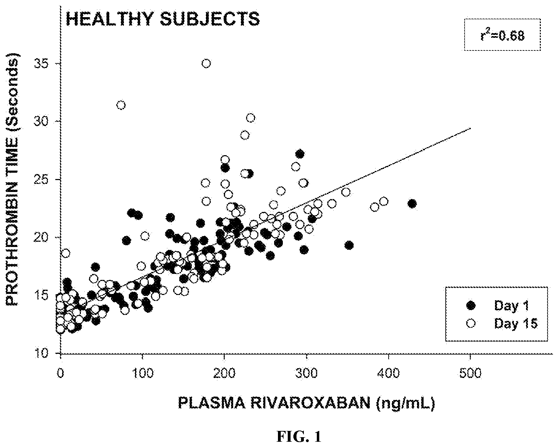

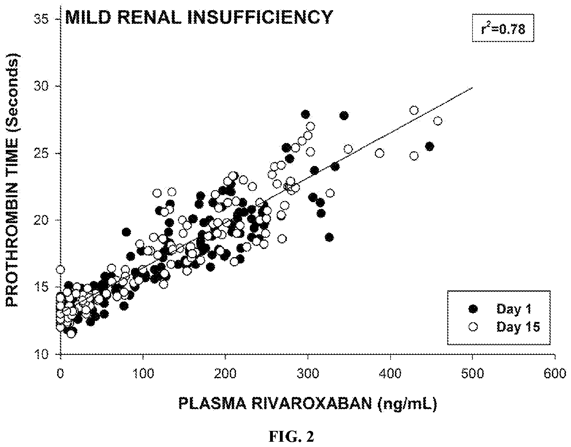

**FIG. 1** shows a correlation between plasma concentrations of rivaroxaban and Prothrombin Time ("PT") in subjects with no renal insufficiency. Data were measured on Day 1 and Day 15 of the study. **FIG. 2** shows a correlation between plasma concentrations of rivaroxaban and PT in subjects with mild renal insufficiency. Data were measured on Day 1 and Day 15 of the study.

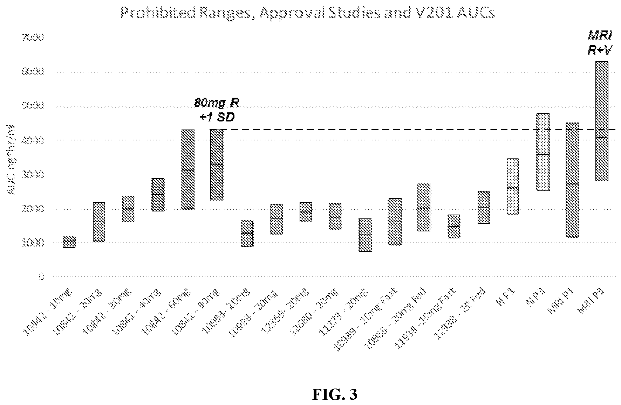

**FIG. 3** shows the ranges of steady state rivaroxaban AUC values for various single dose rivaroxaban treatments (10842, 10993, 10999, 12359, 12680, 11273, 10989, 11938, studies), and single dose rivaroxaban plus Verapamil treatments. Abbreviations in the figure are N: Normal renal function; MRI: mild renal impairment; P1: 20 mg rivaroxaban only; P3: 20 mg rivaroxaban and 360 mg Verapamil. The lines represent the geometric mean and for the numbered studies the top and bottom represent +/- 1 standard deviation. For the Normal and MRI groups by study period, the lines represent the geometric mean and the top and bottom represent the sample maximum and minimum.

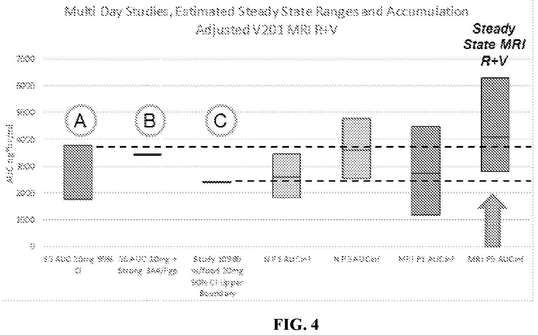

**FIG. 4** shows the 90% confidence level rivaroxaban AUC value (single 20 mg dose) (Label "A"), steady state rivaroxaban AUC value as described in the Clinical Pharmacology approval documents for Rivaroxaban and separately the 10 mg steady state dose in the presence of a strong CYP3A4/Pgp inhibitor (Label "B") from the same report. The third item, (Label "C") shows the upper 90% confidence interval value for rivaroxaban administered under fed conditions as determined in clinical study 10989. These values are compared to the ranges of AUC measured by the present inventors under various conditions. Abbreviations in the figure are SS: steady state; CI: confidence interval; AUC inf: area under the curve extrapolated to infinity; N: Normal renal function; MRI: mild renal impairment; P1: a single 20 mg rivaroxaban only; P3: a single 20 mg rivaroxaban plus 360 mg Verapamil.

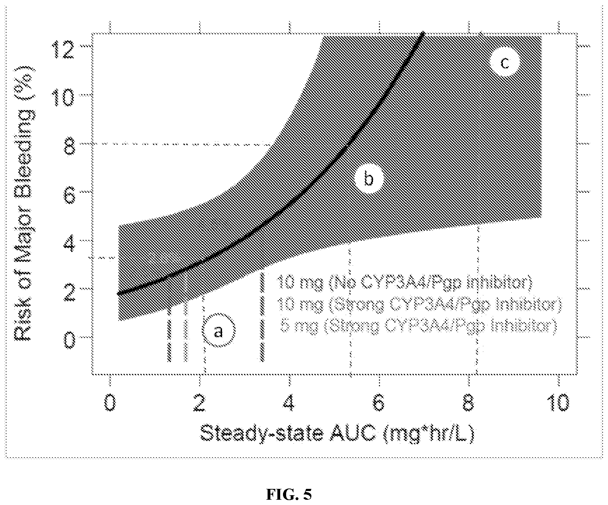

**FIG. 5** shows the relationship between the steady state AUC values and the risk of major bleeding.

### DETAILED DESCRIPTION

While the following terms are believed to be well understood by one of ordinary skill in the art, the following definitions are set forth to facilitate explanation of the presently disclosed subject matter. The following aspects of the disclosure are not claimed, but are illustrative for the purposes of reproducing the claimed invention.

Throughout the disclosure, singular forms such as "a," "an," and "the" are often used for convenience. However, it should be understood that the singular forms are intended to include the plural, except when context or an explicit statement indicates that the singular alone is intended.

Throughout this application, the term "about" is used to indicate that a value includes the inherent variation of error for the device or the method being employed to determine the value, or the variation that exists among the samples being measured. In some embodiments, the term "about" means within 5% of the reported numerical value. When used in conjunction with a range or series of values, the term "about" applies to the endpoints of the range or each of the values enumerated in the series, unless otherwise indicated.

As used herein, the term "concomitant" refers to the co-administration of two or more drugs. In some embodiments, concomitant administration includes the administration of two or more drugs at substantially the same time, either as a mixture (e.g., in a coformulation), or as separate doses. In some embodiments, concomitant administration also includes the sequential administration of two or more drugs, wherein both drugs are simultaneously present at clinically relevant levels in a patient's plasma.

As used herein, the term "pharmacokinetics" refers to a drug's movement into, through, and out of the body, including: the drug's absorption, bioavailability, tissue distribution, metabolism, and excretion.

As used herein, Cₘₐₓ refers to peak plasma concentration of a therapeutic agent as it relates to a particular drug dose (e.g. rivaroxaban dose).

As used herein, Tₘₐₓ refers to the time required to reach peak concentration.

As used herein, Cₐᵥₑ refers to average plasma concentration of a therapeutic agent under a particular drug dosing schedule (e.g. rivaroxaban dosing schedule).

As used herein, Cₘᵢₙ refers to the minimum plasma concentration of a therapeutic agent under a particular drug dosing schedule (e.g. rivaroxaban dosing schedule).

Pharmacokinetic effects of a drug can be compared based on area under the curve (AUC) values. The pharmacokinetic AUC of a drug is calculated by taking the integral (area under the curve) of the concentration of a drug against time.

As used herein, "treating" in the context of treating a condition refers to reducing or eliminating the symptoms of a condition, reducing the risk of, or preventing the patient from experiencing the symptoms of a condition. For example, treatment of a condition with rivaroxaban can reduce the risk of stroke or systemic embolism in patients, reducing the risk of recurrence of deep vein thrombosis and pulmonary embolism, the prophylaxis of deep vein thrombosis, etc.

As used herein, AUC_{0-∞} or AUC_{inf} refers to the area under the curve extrapolated to infinity, after administration of a single dose of drug. AUCₛₛ refers to the AUC under steady state conditions, and approximates AUC_{inf}.

As used herein, the term "pharmacodynamics" refers to a drug's effect(s) on the body, including: receptor binding, postreceptor effects, and chemical interactions. In some embodiments, a drug's pharmacodynamics determines the onset, duration, and intensity of that drug's effect.

As used herein, the term "prothrombin time" (PT) refers to a blood test that measures how long it takes blood to clot. In some embodiments, a prothrombin time test can be used to measure of a patient's haemostasis. In some embodiments, PT can be used to measure the effectiveness or exposure to a blood clotting drug. Persons having skill in the art sometimes interchangeably refer to a PT test as an INR. However, since the INR is calibrated specifically for warfarin, PT measurements suitable for rivaroxaban would not be comparable to the INR standards for warfarin. Additionally, PT measurements are subject to variability based on the test reagent and laboratory test ranges determined by each institution.

Rivaroxaban was developed with the premise that it would not require the type of monitoring required for conventional anticoagulant drugs such as warfarin (Coumadin). PT/INR tests are conventionally used specifically for monitoring vitamin K antagonists such as warfarin. Alternative tests, such as the activated partial thromboplastin time (APTT) are conventionally used to monitor the use of unfractionated heparin. However, as discussed in Favaloro et al., Biochemia Medica 2012; 22(3): 329-41, the PT/INR and APTT tests are not suitable for monitoring patients treated with rivaroxaban because inconsistent results are obtained for rivaroxaban depending on factors such as the reagents used, and the tests are either too sensitive or insensitive to be useful. In addition, such tests are not calibrated for rivaroxaban, and therefore there is no standardized measure for the activity of rivaroxaban in such patients. While the literature suggests that suitable tests could be developed, presently there are no validated or generally recognized methods suitable for monitoring the use of rivaroxaban in hospital laboratories or available to healthcare providers. See also, Xarelto® Package Insert (Revised 8/2016, section 5.7). Consequently, there are no current methods available for monitoring the dose titration of rivaroxaban. Indeed, the sponsor of the rivaroxaban NDA maintains that dose titration is not appropriate in most patient populations (Application Number: 022406Orig1s000 Clinical Pharmacology and Biopharmaceutics Review(s), Addendum to April 6, 2009 Review). See Hillarp et al., Journal of Thrombosis and Haemostasis, 9: 133-139; Favarolo et al., Biochemia Medica 2012; 22(3): 329-41; Nielsen et al., Clin. Res. Cardiol. 22 November 2014 (published online); and Mueck et al., Clin. Pharmacokinet (2014) 53:1-16.

As used herein, Eₘₐₓ refers to peak effect of a therapeutic agent related to a particular drug dose (e.g. rivaroxaban dose).

As used herein, the term "AUEC" refers to area under the effect curve. In some embodiments, the pharmacodynamic AUEC of an effect for a particular drug is calculated by taking the integral (area under the curve) of the drug's measured effect against time.

In some embodiments, the AUEC of rivaroxaban is calculated based on the integral of the prothrombin time effect a rivaroxaban dose has on a patient over time.

As used herein, "renal impairment" means the individual has a clinically relevant level of renal function which is less than levels of renal function generally considered to be normal. Levels of renal function, renal sufficiency or renal impairment can be determined using any of the methods known in the art or described herein. Currently, one common method for determining the level of renal sufficiency in an individual is determining creatinine clearance (CL_{Cr}) in the individual using the Cockcroft-Gault equation. In some embodiments, the present disclosure teaches alternative methods of measuring renal impairment, for example those described in U.S. Published App. 2012/022787.

As disclosed herein, the individual's actual body weight, ideal body weight, or otherwise adjusted body weight can be used in the equation. In some embodiments, the following are criteria for determining the level of renal sufficiency using creatinine clearance (CL_{Cr}) and the Cockcroft-Gault equation:
Normal renal function > or = 80 mL/min
Mild renal impairment = 50-79 mL/min
Moderate renal impairment = 30-49 mL/min
Severe renal impairment < 30 mL/min
Newer criteria for determining the level of renal sufficiency using CL_{Cr} define the cutoffs between normal, and mild-severe renal impairment using slightly different CL_{Cr} ranges:
Normal renal function > or = 90 mL/min
Mild renal impairment = 60-89 mL/min
Moderate renal impairment = 30-59 mL/min
Severe renal impairment = 15-29 mL/min
Kidney failure < 15 mL/min
A "patient" is a living organism, typically a human.

For nearly all FDA approved drugs, the "recommended" dose (or doses) of the drug are determined based on the plasma level (or range of plasma levels) of the drug required to provide the desired clinical effect(s) and/or avoid undesirable side effects. The recommended dose(s) of a particular drug are those recognized in the art as suitable for treating a patient with particular physical characteristics (or within a range of particular characteristics), and are thus the dose(s) provided in the package insert for the drug. Thus, in various embodiments, the methods of the present disclosure are directed to adjustments or changes in the dosing of rivaroxaban relative to the FDA "recommended" dose, e.g., in the package insert for rivaroxaban, as suitable for treating a patient with particular physical characteristics. Thus, as used herein, the "recommended dose" for rivaroxaban is distinct from doses which may be disclosed by particular physicians for particular patients. Depending on the specific pharmacokinetics and pharmacodynamics of the drug, the recommended dose may vary depending on one or more physical characteristics of the patient, for example age, gender, weight, body mass index, liver metabolic enzyme status (e.g., poor or extensive metabolizer status), disease state, etc. Xarelto® (rivaroxaban) is currently sold in three FDA-approved doses: 10 mg (e.g., NDC 50458-580-30), 15 mg (e.g., NDC-50458-578-30), and 20 mg (e.g., NDC 50458-579-30).

As provided in the rivaroxaban package insert (as revised 08/2016), the 15 mg and 20 mg tablets should be taken with food, and the 10 mg tablets taken with or without food. For patients with nonvalvular atrial fibrillation, with CL_{Cr} >50 mL (normal to mild renal impairment), the 20 mg dose with the evening meal is recommended. For such patients with CL_{Cr} 15-50 (severe to moderate renal impairment), the 15 mg dose with the evening meal is recommended. For patients with DVT, PE, and to reduce the risk of recurrence of DVT and PE, it is recommended that patients be administered 15 mg twice daily with food for the first 21 days, then 20 mg once daily with food for the remaining treatment. For the prophylaxis of DVT following hip or knee replacement surgery, 10 mg once daily is recommended with or without food.

With respect to drug-drug interactions, the rivaroxaban package insert recommends avoiding concomitant use of rivaroxaban with combined P-gp and strong CYP3A4 inhibitors and inducers - that is, rivaroxaban and the combined P-gp and strong CYP3A4 inhibitors or inducers should not be coadministered; rather, one or the other drug should be discontinued. Although concomitant administration of rivaroxaban with drugs such as erythromycin, which is considered in the Xarelto® package insert to be a combined P-gp and moderate CYP3A4 inhibitor, has been observed to increase exposure to rivaroxaban, the package insert indicates that no precautions are necessary in such coadministration if the change in exposure is not expected to affect bleeding risk (see section 7.1 of the package insert), but that otherwise rivaroxaban should not be used unless the potential benefit justifies potential risk (see section 7.5 of the package insert). However, various studies discussed herein have shown that there are as yet no suitable assays for monitoring rivaroxaban dosing or assessing potential risk. (See e.g., Favarolo et al., Biochemia Medica 2012; 22(3): 329-41; Xarelto® Package Insert). Also, concomitant administration of rivaroxaban with combined P-gp and strong CYP3A4 inhibitors should be avoided - i.e., one or the other drugs should be eliminated.

Thus, although drug-drug interactions with rivaroxaban are known (e.g., elevated exposure to rivaroxaban in the presence of combined P-gp and CYP3A4 inhibitors), only in the case of coadministration of rivaroxaban and combined P-gp and strong CYP3A4 inhibitors is it necessary to eliminate (i.e., avoid) the use of rivaroxaban. Indeed, until the present disclosure, those of skill in the art have not identified clinically relevant interactions between rivaroxaban and commonly prescribed medications by virtue of rivaroxaban's multiple elimination pathways. (See W. Mueck et al., Clin Pharmacokinet (2014) 53:1-16). Thus, to-date, studies of drug-drug interactions with rivaroxaban have only identified coadministration of rivaroxaban with combined P-gp and strong CYP3A4 inhibitors (or strong inducers) as requiring any modification of rivaroxaban dosing, and in such instances it is recommended that the rivaroxaban (or alternatively the strong inhibitor/inducer) be eliminated entirely from the treatment.

As discussed herein, the methods of the present disclosure provide for an adjustment of the rivaroxaban dose relative to the recommended dose for that patient if the patient was not concomitantly administered verapamil. Alternatively as discussed herein, the methods of the present disclosure provide for an adjustment of the rivaroxaban dose relative to the recommended dose for an otherwise identical patient not concomitantly administered verapamil. As used herein, the term an "otherwise identical patient who is not concomitantly administered verapamil" refers to a patient whose physical characteristics relevant to drug (e.g., rivaroxaban) dosing are expected to be substantially the same as that of the patient being treated with a reduced rivaroxaban dose according to the presently disclosed methods - except for the concomitant administration of verapamil. In some embodiments, the otherwise identical patient will be of substantially the same age, sex, and body weight. In some embodiments, the substantially identical patient will also have substantially identical renal function and drug metabolism. In some embodiments, the recommended dose of the otherwise identical patient who is not concomitantly administered verapamil is the dose that would have been recommended to that same patient, had that patient not been concomitantly administered verapamil.

All terms not specifically defined herein should be given the ordinary meaning that a person of skill in the art at the time of the invention would ascribe to them.

In some aspects, the present disclosure is directed to methods of treating a patient in need of treatment with a Factor Xa inhibitor such as rivaroxaban. In some aspects, a nonlimiting list of the medical conditions which require treatment with a Factor Xa inhibitor (e.g., rivaroxaban) include: atrial fibrillation; deep vein thrombosis; patients undergoing major orthopedic surgery; deep vein thrombosis prophylaxis, deep vein thrombosis prophylaxis after abdominal surgery; deep vein thrombosis prophylaxis after hip replacement surgery; deep vein thrombosis prophylaxis after knee replacement surgery; deep vein thrombosis recurring event; heart attack; prevention of thromboembolism in atrial fibrillation; pulmonary embolism; pulmonary embolism recurring event; thromboembolic stroke prophylaxis; venous thromboembolism; prevention of ischemic stroke; recurring myocardial infarction; antiphospholipid antibody syndrome; sickle cell disease; prevention and treatment of venous thromboembolism in cancer patients; cancer associated thrombosis; cancer patients with central line associated clots in the upper extremity; reducing post-discharge venous thromboembolism risk in medically ill patients; treating young children with venous thrombosis (6 months - 5 years); treatment of arterial or venous thrombosis in children from birth to less than 6 months; thrombophylaxis in pediatric patients 2 to 8 years of age after the fontan procedure; valvular heart disease and atrial fibrillation; patients with atrial fibrillation with bioprosthetic mitral valves; treatment of symptomatic isolated distal deep vein thrombosis; superficial vein thrombosis; prevention of thrombosis after replacement of the aortic valve with a biological valve prosthesis; prevention of recurrence of stent thrombosis and cardiovascular events in patients with atrial fibrillation complicated with stable coronary artery disease; treatment of splanchnic vein thrombosis; prevention of recurrent thrombosis in patients with chronic portal vein thrombosis; prevention of recurrent symptomatic venous thromboembolism in patients with symptomatic deep vein thrombosis or pulmonary embolism; acute ischemic stroke with atrial fibrillation; venous thromboembolism prophylaxis in patients undergoing non-major orthopedic surgery; reducing the risk of major thrombotic vascular events in subjects with peripheral artery disease undergoing peripheral revascularization procedures of the lower extremities; prevention of cardiovascular events in patients with nonvalvular atrial fibrillation scheduled for cardioversion; reducing the risk of death, myocardial infarction, or stroke in participants with heart failure and coronary artery disease following an episode of decompensated heart failure; preventing major cardiovascular events in coronary or peripheral artery disease; reducing the risk of cardiovascular death, myocardial infarction, or stroke in patients with recent acute coronary syndrome; prevention of the composite of stroke or systemic embolism in patients with rheumatic valvular heart disease (RVHD) with atrial fibrillation or flutter who are unsuitable for vitamin K antagonist therapy, or in patients with RVHD without AF or Flutter with at least one of the following: Left atrial enlargement ≥ 5.5 cm, Left atrial spontaneous echo contrast, left atrial thrombus, frequent ectopic atrial activity (>1000/24 hours) on Holter ECG; prevention of restenosis after infrainguinal percutaneous transluminal angioplasty for critical limb ischemia; and decreasing the risk of cardiovascular disease, myocardial infarction, revascularization, ischemic stroke, and systemic embolism.

Atrial fibrillation (AF) is a common cardiac disorder that is characterized by a disruption of the normal electrical impulses generated by the sinoatrial (SA) node. The resulting disorganized electrical impulses lead to an irregular heartbeat and abnormal blood flow.

AF is also a significant stroke risk factor. The characteristic lack of coordinated atrial contraction in AF can result in clot formation in the atrium, and particularly the left atrial appendage of the heart. The increased stasis of blood in the atrium due to loss of mechanical function (i.e. contraction), combined with poorly understood changes in the thrombogenicity of the atrial endocardial surface in AF is thought to be the primary basis for clot formation in the left atrium and left atrial appendage in AF.

If the blood clot leaves the atria and becomes lodged in an artery in the brain, a stroke results. Diagnosed AF is associated with a four- to five-fold increase in stroke risk (Wolf P A, Abbott R D, Kannel W B, Arch Intern Med. 1987 September; 147(9):1561-4; Stroke. 1991 August; 22(8):983-8). Approximately 15% of strokes in the U.S. occur in individuals previously diagnosed with AF. It is believed that even more strokes are associated with undiagnosed AF (Tayal, et al., Neurology. 2008 Nov. 18; 71(21):1696-701).

Patients diagnosed with AF often receive dual treatment of calcium channel blockers for their heart arrhythmia and anticoagulants to reduce the risk of stroke.

Rivaroxaban (Formula I), which is commercially available under the trade name Xarelto®, is disclosed in US7157456 (B2), US7585860 (B2), and US7592339 (B2).

Rivaroxaban is a Factor Xa inhibitor drug used to treat thrombosis-related disorders, among other conditions as disclosed herein. Rivaroxaban activity is mediated though its inhibition of Factor Xa, which in turn reduces the conversion of prothrombin to thrombin. It is also used to help prevent strokes or serious blood clots in people who have atrial fibrillation.

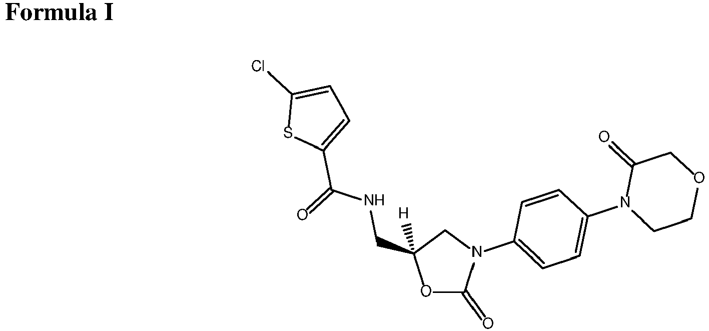

Use of rivaroxaban however, presents risks related to improper dosing. The FDA Draft Guidance on Rivaroxaban (Recommended Sept 2015) states that rivaroxaban demonstrates a "steep exposure-response relationship for both efficacy and safety" and therefore should "refer to the guidance on warfarin sodium." In other words, rivaroxaban is recognized as a drug having a narrow therapeutic index (e.g., meeting nearly all if not all the proposed FDA definitional terms of a Narrow Therapeutic Index drug; [Quality and Bioequivalence Standards for Narrow Therapeutic Index Drugs, Lawrence X Yu, Office of Generic Drugs, GPhA 2011 Fall Technical Workshop]), such that higher than expected plasma concentrations of rivaroxaban can cause serious adverse events, including internal bleeding/hemorrhage, and underdosing leaves a patient at risk of potentially fatal clotting events. Careful monitoring of patients treated with rivaroxaban is made even more difficult by the current lack of specific antidotes against rivaroxaban overdosing, the lack of specific treatments with proven efficacy for severe bleeding linked with the use of rivaroxaban, and the lack of a routine coagulation test suitable for monitoring patients treated with rivaroxaban (as discussed herein). Conventional methods for reducing plasma levels which are useful for other anticoagulants such as dabigatran (e.g. haemodialysis and FDA-approved specific antidote idarucizumab) are not effective for rivaroxaban, because rivaroxaban cannot be eliminated from the body through dialysis and there is no specific antidote for rivaroxaban. (Bleeding with dabigatran, rivaroxaban, apixaban. No antidote, and little clinical experience." Prescrire Int. 2013 Jun;22(139):155-9; PRAXBIND® Product Insert).

The risks associated with incorrectly dosing rivaroxaban have caused the drug to be placed on the Institute for Safe Medication Practices (ISMP) list of high-alert medications as a drug with "heightened risk of causing significant potential harm." (Institute for Safe Medication Practices (ISMP) "ISMP List of High-Alert Medications in Acute Care Settings" https://www.ismp.org/tools/highalertmedications.pdf (Accessed October 2015)).

In some embodiments, rivaroxaban dosing is compared in terms of its pharmacokinetic responses in different patients or patient populations, and in response to different situations. For example, in some embodiments, the present disclosure teaches methods of adjusting rivaroxaban dosing in patients concomitantly administered rivaroxaban and verapamil, relative to otherwise identical (or the same) patients not concomitantly administered verapamil, in order to provide a particular rivaroxaban AUC_{inf}, AUCₛₛ, Cₘₐₓ, C_{max ss} or Cₐᵥₑ (or a range of values of such parameters) in said patient or patient population.

In other embodiments, rivaroxaban dosing is compared in terms of its pharmacokinetic and pharmacodynamics effects. For example, in some embodiments, the present disclosure teaches methods of adjusting rivaroxaban dosing in patients concomitantly administered rivaroxaban and verapamil, relative to otherwise identical (or the same) patients not concomitantly administered verapamil, in order to provide a particular prothrombin time or decrease the incidence rate of adverse events (or range of values of such parameters) in said patient or patient population.

In some aspects, the present disclosure teaches that rivaroxaban pharmacokinetics and pharmacodynamics are at least partially a consequence of the drug's excretion/secretion and metabolism, particularly when coadministered with verapamil.

Approximately one-third (36%) of each rivaroxaban dose is eliminated in the patient's urine as unprocessed active drug. Of this 36% elimination, 30% is eliminated through active renal secretion, while the remaining 6% is eliminated though glomerular filtration. (Weinz C, Schwarz T, Kubitza D, et al. Metabolism and excretion of rivaroxaban, an oral, direct Factor Xa inhibitor, in rats, dogs and humans. Drug Metab Dispos. 2009;37:1056-64; Kubitza et al. Effects of renal impairment on the pharmacokinetic, pharmacodynamics and safety of rivaroxaban, an oral, direct Factor Xa inhibitor. Brit J of Clinical Pharma 70:5 703-712).

Renal impairment can also have effects on the pharmacodynamics of rivaroxaban. Even moderate changes in rivaroxaban AUC and half-life (t_{1/2}) can lead to significant changes in haemostasis. In subjects with mild, moderate, and severe renal impairment administered a 10 mg dose of rivaroxaban, the AUEC for Factor Xa inhibition was 1.5-fold, 1.86-fold, and 2.0-fold higher than in healthy subjects, respectively. (Kubitza et al. Effects of renal impairment on the pharmacokinetic, pharmacodynamics and safety of rivaroxaban, an oral, direct Factor Xa inhibitor. Brit J of Clinical Pharma 70:5 703-712). In this work Kubitza et al. did not evaluate patients administered 20 mg doses, or evaluate drug-drug interactions. However, even in such studies, Kubitza et al. found that such increases in rivaroxaban exposure were unlikely to be clinically relevant, and that safety profiles were similar in patients with differing levels of renal impairment. Thus, Kubitza et al. did not recognize any need to adjust rivaroxaban doses in such patients.

However, the present applicant has found that the recommended rivaroxaban dose should be adjusted in patients in need of Factor Xa inhibitor treatment who are coadministered verapamil. Thus in some aspects, the present disclosure is directed to methods of treating patients in need of Factor Xa inhibitor treatment, said method comprising adjusting rivaroxaban dosing in response to changes in a patient's renal function.

Renal secretion of rivaroxaban is partially regulated by the Permeability glycoprotein (P-gp) pathway. P-gp transporters are found in the luminal membrane of several tissues, including the blood-brain barrier, the small intestine excretory cells, hepatocytes, and kidney proximal tubule epithelia. P-gp expression on intestinal epithelial cells regulates cellular uptake and absorption of drugs into enterocytes, whereas expression of P-gp transporters on the surface of hepatocytes and renal tubular cells regulates the elimination of drugs into the bile and urine (Wessler et al. The P-glycoprotein transport system and cardiovascular drugs. J. American College of Cardiology Vol 61:25).

In some aspects, the present disclosure teaches that P-gp induction or inhibition can result in different pharmacokinetic and pharmacodynamic responses to fixed rivaroxaban doses. For example, in some aspects, the present disclosure teaches that P-gp inhibition can lead to rivaroxaban overdosing due to decreased renal excretion of the drug.

P-gp inhibition is thought to involve modulation by 1 of 4 pathways: direct inhibition of binding sites that block the transport of substrates, ATP binding inhibition, ATP hydrolysis, or coupling of ATP hydrolysis to the translocation of the substrate.

Unfortunately, molecules that are modulators of P-gp substrates do not share any obvious structural characteristics, and it is thus difficult to predict the effect of concomitant administration of a drug on P-gp function (Wessler et al. The P-glycoprotein transport system and cardiovascular drugs. J. American College of Cardiology Vol 61:25.)

Moreover, different drugs may interact differentially with P-gp, which results in a distinct mechanism of modulation of P-gp activity. As shown by Polli et al., Journal of Pharmacology and Experimental Therapeutics, vol. 299(2), 620-628 (2001), various assays of P-gp inhibition can give widely varying results. Polli et al. describe three different P-gp assays: the monolayer efflux assay (using Caco-2 cells), the ATPase and calcein-AM assays. Of the three, the monolayer efflux assay is considered the most reliable because it is a direct measurement of efflux. Polli et al. demonstrated that verapamil does not show efflux (i.e., is not a P-gp substrate) in the monolayer efflux assay, but provides positive results in the ATPase and calcein-AM assays.

Gnoth et al., Journal of Pharmacology and Experimental Therapeutics, vol. 338(1), 372-380 (2011) evaluated the P-gp transport characteristics of rivaroxaban, and rivaroxaban in the presence of potential P-gp inhibitors such as clarithromycin and erythromycin. The authors observed that erythromycin did not inhibit P-gp-mediated efflux of rivaroxaban across cell monolayers *in vitro* - in fact, erythromycin increased the efflux of rivaroxaban in a statistically significant manner. Thus, erythromycin and verapamil utilize distinct mechanisms in their interaction with and regulation of P-gp activity, which could lead to differing inhibitory effects. Gnoth et al. concluded that the impact of P-gp inhibition, alone, had only a marginal effect on the pharmacokinetics of rivaroxaban, and that only strong P-gp inhibitors at high doses might result in drug-drug interactions.

Approximately two-thirds of each rivaroxaban dose is subject to metabolic degradation via the liver's oxidative and hydrolytic pathways.

At least three functional CYP3A proteins exist in humans. The CYP3A4 monooxygenase is the predominant cytochrome P450 in human liver and small bowel. The protein displays a broad substrate specificity and it metabolizes more than 60% of all drugs that are currently in use, including contraceptive steroids, antidepressants, benzodiazepines, immunosuppressive agents, imidazole antimicotics, and macrolide antibiotics (Cholerton, Trends Pharmacol Sci 13 (1992), 434-9; Ketter, J Clin Psychopharmacol 15 (1995), 387-98).

Rivaroxaban metabolic clearance is catalyzed by CYP3A4/5, CYP2J2, and CYP-independent mechanisms. (Kubitza et al. Effects of renal impairment on the pharmacokinetic, pharmacodynamics and safety of rivaroxaban, an oral, direct Factor Xa inhibitor. Brit J of Clinical Pharma 70:5 703-712). The CYP3A4 isozyme in particular, accounts for approximately 18% of the rivaroxaban clearance. (Mueck W, Kubitza D, Becka M. Co-administration of rivaroxaban with drugs that share its elimination pathways: pharmacokinetic effects in healthy subjects. Br J Clin Pharmacol.2013;76:455-66).

In some aspects, the present disclosure teaches that variations in CYP3A4 activity can result in different pharmacokinetic and pharmacodynamic responses to fixed rivaroxaban doses. A considerable variation in the CYP3A4 content and catalytic activity has been described in the general population. For example, studies have shown that the metabolic clearance of the CYP3A4 substrates exhibit a unimodal distribution with up to 20-fold variability between individual patients. This result is also born out in direct activity studies which demonstrated that the activities of the CYP3A4 protein in liver biopsies vary up to 30-fold between individuals (Westlind, Biochem Biophys Res Commun 259 (1999), 201-205).

In order to assess the effects of combined P-gp and CYP3A4 inhibitors on the metabolism of rivaroxaban, a study was conducted to examine the effects of co-administration of rivaroxaban with erythromycin, a combined moderate CYP3A4 inhibitor and P-gp inhibitor. Erythromycin is known to be eliminated mainly in the bile, and 2-15% renally (unchanged). However, based on this study, no dose adjustment for rivaroxaban in the presence of such a combined inhibitor was found to be necessary. Similar results were obtained for clarithromycin and fluconazole, suggesting significant interactions with other drugs in this category are unlikely. (Mueck et al. Co-administration of rivaroxaban with drugs that share its elimination pathways: pharmacokinetic effects in healthy subjects. British J. of Clinical Pharma 76:3 455-466).

On the contrary, the FDA-approved rivaroxaban label states that rivaroxaban use should be avoided (i.e., should not be administered at all) in patients receiving "concomitant combined P-gp and moderate CYP3A4 inhibitors" unless "the potential benefit justifies the potential risk." However, since, as discussed herein there is no clinically standardized test methodology for evaluating the pharmacodynamics of rivaroxaban as there are for other, conventional anticoagulants such as warfarin and heparin (i.e., PT/INR or APTT tests), there is no clinically acceptable or useful methodology for evaluating the potential risk.

In some aspects, the present disclosure is directed to methods of adjusting rivaroxaban doses in response to CYP3A4 induction or inhibition. In some aspects, the present invention thus teaches that the concomitant administration of other drugs can alter CYP3A4 expression levels (*via* induction or repression). For example, some known inducers of CYP3A4 expression include drugs such as the glucocorticoid dexamethasone, the antibiotic rifampicin, and the antimycotic clotrimazole. Like the regulation of P-gp secretion however, the effect of any drug on CYP3A4 activity is difficult to predict.

In addition to metabolism by CYP3A4, 14% of rivaroxaban is metabolized by CYP2J2 (((Mueck W, Kubitza D, Becka M. Co-administration of rivaroxaban with drugs that share its elimination pathways: pharmacokinetic effects in healthy subjects. Br. J. Clin. Pharmacol. 2013;76:455-66). It has been shown that verapamil can inhibit greater than 95% of metabolism by CYP2J2 *in vitro*, while erythromycin did not demonstrate significant inhibition of CYP2J2 activity (Lee CA et al. "Identifying a Selective Substrate and Inhibitor Pair for the Evaluation of CYP2J2 Activity." Drug Metabolism and Disposition 2012 vol. 40 pp943-951). The clinical implications of the potential inhibition of CYP2J2-mediated metabolism of rivaroxaban by verapamil are as yet unknown.

Thus in some aspects, the present disclosure is directed to methods of treating patients in need of Factor Xa inhibitor treatment, said method comprising adjusting rivaroxaban dosing (as described herein) in response to changes in a patient's altered liver drug metabolism.

Verapamil is a calcium channel blocker used in the treatment of angina, arrhythmia, and essential hypertension among other uses, for example as disclosed herein. Verapamil is extensively metabolized to a number of metabolites, at least one of which (norverapamil) retains significant activity and is itself a P-gp inhibitor. Approximately 70% is excreted in the urine (mainly as metabolites, but about 4% unchanged), and approximately 16% in the feces.

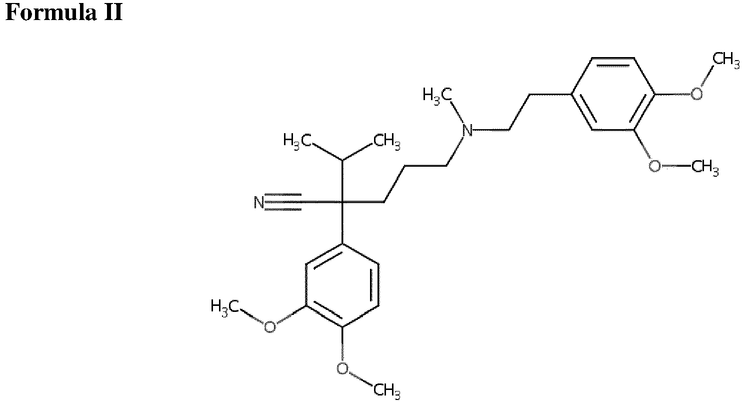

Verapamil is a popular drug, used daily by millions of older patients with heart issues. The prevalent use of the drug has caused it to be listed in the World Health Organizations (WHO) list of essential medicines (found at http://www.who.int/medicines/publications/ essentialmedicines/en/).

In some aspects, the present disclosure is directed to treatment of a patient with a medical condition requiring treatment with a calcium channel blocker such as verapamil (in conjunction with a Factor Xa inhibitor), including management of essential hypertension, treatment of hypertension, treatment of pulmonary hypertension, prevention and treatment of recurrent and paroxysmal supraventricular tachycardia, management of supraventricular tachycardia, treatment of atrial tachycardia and junctional tachycardia, treatment of cerebral vasospasm, treatment of hypertrophic cardiomyopathy, treatment of chronic stable angina pectoris, treatment of unstable angina pectoris, management of Prinzmetal variant angina, ventricular rate control in atrial fibrillation/flutter, prevention of cluster headache, prevention of migraine, prevention of myocardial infarction in patients with preserved left ventricular function, management of manic manifestations of bipolar disorder, treatment of Raynaud's disease, treatment of coronary artery disease, treatment of subarachnoid hemorrhage, treatment of Dravet Syndrome, beta cell survival therapy in Type I diabetes, treatment of vestibular migraine, treatment of chronic subjective dizziness, treatment of erectile dysfunction, prevention of keloid recurrence, treatment of refractory epilepsy, treatment of refractory meningioma, treatment of chronic heart failure secondary to non-ischemic cardiomyopathy, treatment of relapsed or refractory Hodgkin lymphoma, treatment of Marfan Syndrome, treatment of treatment-resistant mania, prevention of kidney disease in diabetic patients, treatment of Metabolic Syndrome, and treatment of hypoglycemia following gastric bypass.

In particular, verapamil has become an effective treatment for atrial fibrillation. Verapamil has been shown to prolong the effective refractory period within the AV node to slow Atrioventricular (AV) conduction in a dose-dependent manner. This property accounts for the ability of verapamil to slow the ventricular rate in patients with chronic atrial flutter or atrial fibrillation, reducing the subjective sensation of palpitations (Johansson and Olsson. 1984 Long-term oral treatment with high doses of verapamil in lone atrial fibrillation. Clin. Cardiol. 7, 163-170). Typical daily doses of verapamil for such patients range from about 40 mg to about 480 mg, often divided in 3 to 4 equal doses during the day. Alternatively, extended release verapamil formulations can be administered once a day, in daily doses of 100 mg to 400 mg, (including 40 mg, 100 mg, 200 mg, 300 mg, and 400 mg). Verapamil is available in various dosage forms including without limitation extended-release capsules (100, 120, 180, 200, 240, 300, and 360 mg), extended-release tablets (120, 180, and 240 mg), immediate release tablets (40, 80, and 120 mg doses), IV solutions (5 mg/2 mL and 10 mg/4 mL), and as combination formulations (e.g., extended release trandolapril/verapamil HCl tablets, 1 mg/240 mg, 2 mg/180 mg, 2 mg/240 mg, 4 mg/240 mg).

Thus patients with atrial fibrillation are often prescribed verapamil to treat heart palpitations, and rivaroxaban to reduce the risk of stroke.

The present disclosure is at least partially based on the inventors' discovery that the concomitant administration of verapamil and rivaroxaban leads to an unexpectedly increased risk of rivaroxaban overdosing and adverse drug effects. Without wishing to be bound by any one theory, the present inventors believe that administration of verapamil and rivaroxaban leads to an unexpected clinically significant drug-drug interaction that negatively impacts the metabolic and excretion clearance of rivaroxaban and can lead to increased adverse events. Thus, in at least some aspects, the present disclosure is a method of reducing the side effects (increased bleeding, such as gastrointestinal bleeding; or alternatively reducing the risk of clotting if the rivaroxaban dosing is discontinued) when verapamil is coadministered with rivaroxaban, by adjusting the rivaroxaban dose to less than the currently recommended dose (i.e., the dose(s) recommended on the Xarelto® package insert as amended 8/2016) as described herein. For example, when the recommended rivaroxaban dose is 20 mg, the adjusted dose of rivaroxaban ranges from 0 mg to about 19.9 mg (e.g., about 0 mg, about 1 mg, about 2 mg, about 2.5 mg, about 3 mg, about 4 mg, about 5 mg, about 6 mg, about 7 mg, about 7.5 mg, about 8 mg, about 9 mg, about 10 mg, about 11 mg, about 12 mg, about 12.5 mg, about 13 mg, about 14 mg, about 15 mg, about 16 mg, about 17 mg, about 17.5 mg, about 18 mg, about 19 mg, or about 19.9 mg); or when the recommended rivaroxaban dose is 15 mg, the adjusted dose of rivaroxaban ranges from about 0 mg to about 14.9 mg (e.g., about 0 mg, about 1 mg, about 2 mg, about 2.5 mg, about 3 mg, about 4 mg, about 5 mg, about 6 mg, about 7 mg, about 7.5 mg, about 8 mg, about 9 mg, about 10 mg, about 11 mg, about 12 mg, about 12.5 mg, about 13 mg, about 14 mg, or about 14.9 mg); or when the recommended rivaroxaban is 10 mg, the adjusted dose of rivaroxaban ranges from about 0 mg to about 9.9 mg (e.g., about 0 mg, about 1 mg, about 2 mg, about 2.5 mg, about 3 mg, about 4 mg, about 5 mg, about 6 mg, about 7 mg, about 7.5 mg, about 8 mg, about 9 mg, or about 9.9 mg).

Likewise, for any of the adjusted rivaroxaban doses enumerated above, the daily verapamil dose ranges from about 100 mg to about 480 mg per day (e.g., about 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 310 mg, 320 mg, 330 mg, 340 mg, 350 mg, 360 mg, 370 mg, 380 mg, 390 mg, 400 mg, 410 mg, 420 mg, 430 mg, 440 mg, 450 mg, 460 mg, 470 mg or about 480 mg), whether administered as a divided dose (e.g., 2-4 times daily) or a single extended release dose. For some patients treated according to the present disclosure, the patient is not concomitantly administered verapamil (i.e., the verapamil dose is 0 mg/day).

Verapamil is classified as an inhibitor of cytochrome P450 CYP3A4 and also as an inhibitor of the transporter permeability-glycoprotein (P-gp). Verapamil has been classified by the FDA as a moderate inhibitor of CYP3A4, but has less of an inhibitory effect on excretion of edoxaban, a similar Factor Xa inhibitor, than erythromycin, which is also viewed by the FDA as a moderate CYP3A4 inhibitor. Furthermore, erythromycin and verapamil are metabolized (cleared) from the body of a patient in substantially differently ways. Erythromycin is mainly metabolized by demethylation in the liver by the CYP3A4 enzyme, and is eliminated primarily in the bile, with little renal excretion (2-15% unchanged drug). In contrast, as discussed above, verapamil undergoes extensive hepatic metabolism, and its metabolites are excreted primarily in the urine. Additionally, some metabolites (i.e., norverapamil and metabolite D-703) also have inhibitory potential toward P-gp (Pauli-Magnus C et al. "Characterization of the Major Metabolites of Verapamil as Substrates and Inhibitors of P-glycoprotein." J Pharmacol Exp Ther 2000; 293:376-382). The clinical relevance of inhibition of P-gp by the metabolites of verapamil is as yet unknown. Thus, verapamil and erythromycin are quite different in their respective metabolic characteristics, particularly renal clearance and the mechanisms by which they interact with P-gp, and thus studies of rivaroxaban/erythromycin co-administration would not be expected to provide clinical insights directly relevant to any drug-drug interactions between rivaroxaban and verapamil.

In some aspects, the present disclosure teaches that the concomitant administration of verapamil leads to a reduction in the body's clearance (metabolism and excretion) of rivaroxaban. Moreover, atrial fibrillation is a disease associated with older patients, with a median age of about 70 years old (Fuster V., et al., Circulation. 2006). Thus in some aspects, the present disclosure teaches that reduced rivaroxaban clearance due to concomitant administration of verapamil is also likely exacerbated by age-related impairment and/or renal impairment of patients receiving the drug.

In some aspects, the present disclosure teaches that the concomitant administration of verapamil and rivaroxaban is associated with higher proportion of adverse events, for example compared to otherwise identical (or the same) patient populations in which verapamil is not administered.

In some aspects, the present disclosure teaches that rivaroxaban pharmacokinetics are at least partially affected by changes in a patient's CYP3A4 metabolism and P-gp secretion. The present disclosure demonstrates however, that general knowledge about the secretion and metabolism pathways of rivaroxaban are not sufficiently clear to predict whether any particular drug would have undesirable drug interaction(s) with rivaroxaban, or that such undesirable interaction(s) could be addressed effectively by the specific dose adjustments of rivaroxaban provided herein, rather than, e.g., eliminating one or both drugs from the patient's treatment. Indeed, the general knowledge in the art indicates that, except for strong combined CYP3A4/P-gp inhibitors, any noted change in rivaroxaban exposure is not clinically relevant, and coadministration of rivaroxaban and strong combined CYP3A4/P-gp inhibitors is to be avoided entirely.

This document has already described that P-gp inhibitors do not share any obvious structural characteristics that could be used to predict a drug's effect on P-gp secretion (Wessler et al. The P-glycoprotein transport system and cardiovascular drugs. J. American College of Cardiology Vol 61:25). Similarly (despite many structural modeling efforts), the molecular structure of a drug is not dispositive of a drug's effect on CYP3A4 inhibition (Sridhar et al. Insights on cytochrome P450 enzymes and inhibitors obtained through QSAR studies. Molecules. 17(8): 9283-9305). It would therefore be difficult, if not impossible, for a person having ordinary skill in the art to predict a drug's effect on liver metabolism or renal secretion solely based on its structure.

Moreover, in some aspects, the present disclosure also teaches that even empirical evidence of a drug's inhibition of CYP3A4 metabolism and/or P-gp secretion, is still not predictive of whether the drug will have a clinically relevant effect on rivaroxaban exposure.

Previous studies have demonstrated that concomitant administration of other CYP3A4 and P-gp inhibitors did not produce clinically relevant effects on rivaroxaban exposure or the prevalence of adverse events. For example, other studies have shown that concomitant administration of rivaroxaban and erythromycin (a moderate CYP3A4 and strong P-gp inhibitor) produced a 34% rivaroxaban exposure increase. Similarly, concomitant administration of rivaroxaban with clarithromycin (another strong CYP3A4 inhibitor and moderate P-gp inhibitor) produced a 54% rivaroxaban exposure increase. The concomitant administration of rivaroxaban and fluconazole (a moderate CYP3A4 inhibitor) produced a 42% rivaroxaban exposure increase (Mueck et al. Co-administration of rivaroxaban with drugs that share its elimination pathways: pharmacokinetic effects in healthy subjects. Br J Clin Pharmacol. 2013;76:455-66). Thus the present disclosure teaches that not all CYP3A4 and P-gp inhibitors exhibit clinically relevant drug-drug interactions with rivaroxaban.

Verapamil and erythromycin are also dosed quite differently clinically. Erythromycin is an antibiotic useful for the treatment of various bacterial infections, and is usually administered for relatively short periods of time, e.g. 1-2 weeks. In contrast, verapamil is typically administered to treat chronic conditions such as hypertension, angina pectoris, cardiac arrhythmias, etc. and is administered over much longer periods of time. These conditions are common comorbidities and indications for use in patients that would also require treatment with rivaroxaban.

The observation that patients concomitantly administered rivaroxaban represented 30% of the reported rivaroxaban serious bleeding adverse events, despite accounting for only 22% of the total population receiving rivaroxaban (see Example 1) was unexpected in view of the teachings of the prior art which suggested no clinically relevant drug-drug interactions existed between verapamil and rivaroxaban. For example, Xarelto's® (rivaroxaban) own product insert concludes that concomitant use of rivaroxaban with verapamil did not result in increased patient bleeding (see ROCKET AF trial in section 7.5 of Xarelto® product insert (revised 08/2016). Additionally, a poster presented on November 8, 2015 at the American Heart Association 2015 Scientific Sessions concluded that use of non-dihydropyridine calcium channel blockers such as verapamil with rivaroxaban was not associated with an increased risk of non-major clinically relevant or major bleeding compared to subjects on non-dihydropyridine calcium channel blockers and warfarin (see Poster S4081 - Efficacy and Safety of Rivaroxaban versus Warfarin in Patients Taking Non-dihydropyridine Calcium Channel Blockers: Results from the ROCKET-AF Trial, presented November 8, 2015, American Heart Association, Scientific Sessions 2015, and published at http://www.abstractsonline.com/pp8/#!/3795/presentation/37668).

Moreover, the present disclosure also teaches that the increased incidence of adverse events was not common to all CYPA34 and P-gp inhibitors, but was rather unexpectedly specific to verapamil. For example, in some aspects, the present disclosure teaches that concomitant administration of rivaroxaban and erythromycin (another moderate CYP3A4 inhibitor and strong P-gp inhibitor) is not associated with increased serious bleeding adverse events.

The present invention's discovery of the need to adjust the dose of rivaroxaban when coadministered with verapamil was also unexpected based on, among other factors, the lack of any perceived clinically relevant drug-drug interactions between rivaroxaban and substrates of CYP enzymes (e.g., Mueck et al.), the expected weaker P-gp inhibition of verapamil compared to erythromycin, verapamil's previous history of safe concomitant administration with other anticoagulants, including other direct Factor Xa inhibitors. For example, previous studies reviewing the interaction between edoxaban (an oral direct Factor Xa inhibitor) and verapamil, concluded that their co-administration did not lead to any increased bleeding or other adverse events (Mendell et al., Drug-drug interaction studies of cardiovascular drugs involving p-glycoprotein, an efflux transporter, on the pharmacokinetics of edoxban, an oral Factor Xa inhibitor. Am. J. Cardiovasc Drugs (2013) 13:331-342). Accordingly, the label for SAVAYSA™ (edoxaban) does not suggest reduced dosing for patients concomitantly administered with verapamil (SAVAYSA™ product insert, Highlights of prescribing information (revised 09/2016), found at http://dsi.com/prescribing-information-portlet/ getPIContent?productName=Savaysa). Moreover, the SAVAYSA™ product insert shows that concomitant administration of edoxaban and verapamil has a smaller impact on edoxaban exposure compared to concomitant administration edoxaban with erythromycin, as shown by the lower GMR values for the Cₘₐₓ and AUC parameters of verapamil compared to erythromycin (e.g., Figure 12.1 of the SAVAYSA™ product insert). Thus, to the extent edoxaban/verapamil coadministration may be predictive of the drug-drug interactions expected for coadministered rivaroxaban/verapamil, the effects of verapamil coadministration would be expected to be appreciably less than for erythromycin coadministration. However, the SAVAYSA™ product insert does not recommend dose adjustment of edoxaban when coadministered with either erythromycin or verapamil.

Similarly, the only clinically significant drug-drug interactions identified in the ELIQUIS® (apixaban) package insert (revised 9/2015) are with strong dual CYP3A4 and P-gp inhibitors such as ketoconazole, itraconazole, and, and clarithromycin.

In addition, the dabigatran (PRADAXA®) label, another anticoagulant, states that interaction of dabigatran with various P-gp inhibitors (e.g., verapamil, amiodarone, quinidine, clarithromycin and ticagrelor) does not require a dose adjustment of PRADAXA®, and that this conclusion should not be extrapolated to other P-gp inhibitors, further indicating that P-gp inhibition is unpredictable and one of skill in the art would not use the study of a particular drug to directly inform dosing instructions for another drug. For this reason, studies of interactions between, for example, rivaroxaban and erythromycin is not a good predictor for interactions between e.g. rivaroxaban and verapamil.

In addition, to the extent that the Xarelto® (rivaroxaban) Package Insert indicates that concomitant use of combined P-gp and CYP3A4 inhibitors is contraindicated or should be avoided (due to reduced elimination of rivaroxaban), there are a number of known alternative anticoagulants, such as apixaban, edoxaban, or dabigatran, better safety and/or efficacy profiles in patients with reduced clearance. See for example Nielsen et al., Clin. Res. Cardiol. 22 November 2014 (published online) which refers to apixaban as a first line choice for patients with impaired renal elimination due to its comparable efficacy and favorable safety profile.

The current FDA approved dosing scheme for rivaroxaban includes a once-daily 10-20 mg dose. Specifically, patients with nonvalvular atrial fibrillation, deep vein thrombosis, or pulmonary embolism, are recommended to take a once-daily 20 mg oral dose with the evening meal, while patients with deep vein thrombosis following hip or knee replacement surgery are recommended to take a once-daily 10 mg dose with or without food. Xarelto's® (rivaroxaban) product insert does not currently recommend a dose reduction with verapamil (https://www.xareltohcp.com/shared /product/xarelto/prescribing-information.pdf).

In some aspects, the present disclosure is directed to methods of treating a patient in need of treatment with a Factor Xa inhibitor and who is concomitantly administered verapamil, said method comprising administering a reduced dose of Factor Xa inhibitor relative to the recommended dose of Factor Xa inhibitor for an otherwise identical (or the same) patient who is not concomitantly administered verapamil. In particular aspects, the Factor Xa inhibitor is rivaroxaban.

In some aspects, the present disclosure is directed to the administration of a reduced rivaroxaban dose, in which the reduced rivaroxaban dose is about 0%, about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, 99%, or 99.5% of the rivaroxaban dose recommended for an otherwise identical (or the same) patient who is not concomitantly administered verapamil, including all ranges therebetween.

Thus in some aspects, the present disclosure is directed to the administration of a reduced rivaroxaban dose, in which the reduced rivaroxaban dose is between about 0%-99.5% of the recommended dose for an otherwise identical (or the same) patient who is not concomitantly administered verapamil, and all ranges and subranges within as described herein. For example, in some aspects, the present disclosure is directed to the administration of a reduced rivaroxaban dose (relative to the recommended dose for an otherwise identical (or the same) patient not concomitantly administered verapamil as described herein), in which the reduced rivaroxaban dose is between about 20-99.5% of the recommended dose for an otherwise identical (or the same) patient who is not concomitantly administered verapamil.

Thus in some aspects, the present disclosure is directed to a method of treating a patient in need of a Factor Xa inhibitor, said method comprising the step of administering one or more reduced rivaroxaban dose(s) (relative to the recommended dose for an otherwise identical (or the same) patient not concomitantly administered verapamil as described herein) of about 0 mg, about 1 mg, about 2 mg, about 2.5 mg, about 3 mg, about 4 mg, about 5 mg, about 6 mg, about 7 mg, about 7.5 mg, about 8 mg, about 9 mg, about 10 mg, about 11 mg, about 12 mg, about 12.5 mg, about 13 mg, about 14 mg, about 15 mg, about 16 mg, about 17 mg, about 17.5 mg, about 18 mg, about 19 mg, or less than about 20 mg.

Thus in some aspects, the present disclosure is directed to the administration of a reduced rivaroxaban dose (relative to the recommended dose for an otherwise identical (or the same) patient not concomitantly administered verapamil as described herein), in which the reduced rivaroxaban dose is between about 0 mg to less than about 20 mg, and all ranges and subranges therebetween as described herein. For example, in some aspects, the present disclosure is directed to the administration of a reduced rivaroxaban dose (relative to the recommended dose for an otherwise identical (or the same) patient not concomitantly administered verapamil as described herein), in which the reduced rivaroxaban dose is between about 0-10 mg, such as about 0 mg, about 2.5 mg, about 5 mg, about 7.5 mg, or about 10 mg.

In some aspects, the present disclosure is directed to a method of treating a patient in need of a Factor Xa inhibitor, said method comprising the step of administering one or more reduced rivaroxaban dose(s) (relative to the recommended dose for an otherwise identical (or the same) patient not concomitantly administered verapamil as described herein) of about 10 µg/Kg, 20 µg/Kg, 30 µg/Kg, 40 µg/Kg, 50 µg/Kg, 60 µg/Kg, 70 µg/Kg, 80 µg/Kg, 90 µg/Kg, 100 µg/Kg, 110 µg/Kg, 120 µg/Kg, 130 µg/Kg, 140 µg/Kg, 150 µg/Kg, 160 µg/Kg, 170 µg/Kg, 180 µg/Kg, 190 µg/Kg, 200 µg/Kg, 210 µg/Kg, 220 µg/Kg, 230 µg/Kg, 240 µg/Kg, 250 µg/Kg, 260 µg/Kg, 270 µg/Kg, 280 µg/Kg, 290 µg/Kg, 300 µg/Kg, 310 µg/Kg, 320 µg/Kg, 330 µg/Kg, 340 µg/Kg, 350 µg/Kg, 360 µg/Kg, 370 µg/Kg, 380 µg/Kg, 390 µg/Kg, 400 µg of rivaroxaban per Kilogram of body weight.

Thus in some aspects, the present disclosure is directed to the administration of a reduced rivaroxaban dose, in which the reduced rivaroxaban dose (relative to the recommended dose for an otherwise identical (or the same) patient not concomitantly administered verapamil as described herein) is between about 10 µg/kg-300 µg/kg of body weight, inclusive of all ranges and subranges therebetween. For example, in some aspects, the present disclosure is directed to the administration of a reduced rivaroxaban dose (relative to the recommended dose for an otherwise identical (or the same) patient not concomitantly administered verapamil as described herein), in which the reduced rivaroxaban dose is reduced to between about 100 µg/kg-150 µg/kg of body weight.

In various aspects, the present disclosure is directed to a method of treating a patient with reduced dose of rivaroxaban relative to the recommended dose of rivaroxaban for an otherwise identical (or the same) patient not concomitantly administered verapamil. Persons having skill in the art will readily recognize that a clinically effective dose of rivaroxaban for a patient may be dependent on various factors including patient age, sex, body weight, disease progression, overall heath, pathological state, tolerance to the drug, dosing frequency, route of administration, etc. However, the "recommended dose" is the dose recommended in the art, specifically in the package insert for rivaroxaban, as suitable for a particular patient or patient population based on recognized, clinically relevant physical criteria as established during e.g. clinical trials upon which FDA approval was based. Thus in some aspects, the present disclosure teaches that an "otherwise identical" patient who is not concomitantly administered verapamil is a patient that has substantially identical physical and biophysical characteristics as the treated patient, other than the administration of verapamil. Thus in some aspects, an identical patient could be an experimental control patient in studies evaluating the reduced rivaroxaban dosing treatments of the present disclosure.

In the methods of the present disclosure, the rivaroxaban can be administered by any suitable method or mode. For example the compound of the formula (I) can be administered in various forms as described herein, e.g., capsules, tablets, oral solutions or suspensions, dry powders, or parenteral dosage forms such as injectable or IV solutions or suspensions.

In some aspects, the present disclosure is directed to methods of treating a patient in need of treatment with a Factor Xa inhibitor and who is concomitantly administered verapamil, wherein said method comprises administering more than one dose of rivaroxaban per day. Thus in some aspects the present disclosure is directed to methods of administering, 1, 2, or 3 doses of rivaroxaban per day. In some aspects, the present disclosure is directed to administering 2 daily doses of rivaroxaban with food. In various of these aspects, said multiple doses are reduced doses according to the present disclosure.

In some aspects, the present disclosure is directed to methods of treating a patient in need of treatment with a Factor Xa inhibitor and who is concomitantly administered verapamil, said method comprising administering a reduced amount of the recommended dose, as described herein, for an otherwise identical (or the same) patient who is not concomitantly administered verapamil, wherein the patient experiences the same or lower Cₘₐₓ of rivaroxaban compared to the otherwise identical (or the same) patient who is not concomitantly administered verapamil and is receiving the recommended dose of rivaroxaban. In some aspects, the present disclosure is directed to administering a reduced rivaroxaban dose (relative to the recommended dose for an otherwise identical (or the same) patient not concomitantly administered verapamil as described herein), wherein the rivaroxaban Cₘₐₓ (including steady state Cₘₐₓ, C_{max ss}) of said patient for the inventive dosing regimen is less than about 500 ng/mL, 495 ng/mL, 490 ng/mL, 485 ng/mL, 480 ng/mL, 475 ng/mL, 470 ng/mL, 465 ng/mL, 460 ng/mL, 455 ng/mL, 450 ng/mL, 445 ng/mL, 440 ng/mL, 435 ng/mL, 430 ng/mL, 425 ng/mL, 420 ng/mL, 415 ng/mL, 410 ng/mL, 405 ng/mL, 400 ng/mL, 395 ng/mL, 390 ng/mL, 385 ng/mL, 380 ng/mL, 375 ng/mL, 370 ng/mL, 365 ng/mL, 360 ng/mL, 355 ng/mL, 350 ng/mL, 345 ng/mL, 340 ng/mL, 335 ng/mL, 330 ng/mL, 325 ng/mL, 320 ng/mL, 317 ng/mL, 315 ng/mL, 310 ng/mL, 305 ng/mL, 300 ng/mL, 295 ng/mL, 290 ng/mL, 285 ng/mL, 280 ng/mL, 275 ng/mL, 270 ng/mL, 265 ng/mL, 260 ng/mL, 255 ng/mL, 250 ng/mL, 245 ng/mL, 240 ng/mL, 235 ng/mL, 230 ng/mL, 225 ng/mL, 220 ng/mL, 215 ng/mL, 210 ng/mL, 205 ng/mL, 200 ng/mL, 195 ng/mL, 190 ng/mL, 185 ng/mL, 180 ng/mL, 175 ng/mL, 170 ng/mL, 165 ng/mL, 160 ng/mL, 158 ng/mL, 155 ng/mL, 150 ng/mL, 145 ng/mL, 140 ng/mL, 135 ng/mL, 130 ng/mL, 125 ng/mL, 120 ng/mL, 115 ng/mL, 110 ng/mL, 105 ng/mL, 100 ng/mL, 95 ng/mL, 90 ng/mL, 85 ng/mL, 80 ng/mL, 75 ng/mL, 70 ng/mL, 65 ng/mL, 60 ng/mL, 55 ng/mL, or about 50 ng/mL, inclusive of all ranges and subranges therebetween.

Thus in some aspects, the present disclosure is directed to the administration of a reduced rivaroxaban dose (relative to the recommended dose for an otherwise identical (or the same) patient not concomitantly administered verapamil as described herein), in which the reduced rivaroxaban dose produces a rivaroxaban Cₘₐₓ (including steady state Cₘₐₓ, C_{max ss}) between about 158 ng/mL-317 ng/mL, inclusive of all ranges and subranges therebetween. For example, in some aspects, the present disclosure is directed to the administration of a reduced rivaroxaban dose, in which the reduced rivaroxaban dose produces a rivaroxaban Cₘₐₓ (including steady state Cₘₐₓ, C_{max ss}) of between about 250 ng/mL-300 ng/mL.

In some aspects, the present disclosure is directed to methods in which the rivaroxaban dose of patients who are concomitantly administered verapamil is reduced (relative to the recommended dose for an otherwise identical (or the same) patient not concomitantly administered verapamil as described herein), wherein the reduced rivaroxaban dose provides the same or lower AUC of rivaroxaban compared to an otherwise identical (or the same) patient who is not concomitantly administered verapamil and is receiving the recommended rivaroxaban dose. In some aspects, the present disclosure is directed to the administration of a reduced rivaroxaban dose (relative to the recommended dose for an otherwise identical (or the same) patient not concomitantly administered verapamil as described herein), wherein the rivaroxaban AUC (AUC_{inf} or AUCₛₛ) of said patient is lower than about 200 µg•hr/l, 225 µg•hr/L, 250 µg•hr/L, 275 µg•hr/L, 300 µg•hr/L, 325 µg•hr/L, 350 µg•hr/L, 375 µg•hr/L, 400 µg•hr/L, 425 µg•hr/L, 450 µg•hr/L, 475 µg•hr/L, 500 µg•hr/L, 525 µg•hr/L, 550 µg•hr/L, 575 µg•hr/L, 600 µg•hr/L, 625 µg•hr/L, 650 µg•hr/L, 675 µg•hr/L, 700 µg•hr/L, 725 µg•hr/L, 750 µg•hr/L, 775 µg•hr/L, 800 µg•hr/L, 825 µg•hr/L, 850 µg•hr/L, 875 µg•hr/L, 900 µg•hr/L, 925 µg•hr/L, 950 µg•hr/L, 975 µg•hr/L, 1000 µg•hr/L, 1025 µg•hr/L, 1050 µg•hr/L, 1075 µg•hr/L, 1100 µg•hr/L, 1125 µg•hr/L, 1150 µg•hr/L, 1175 µg•hr/L, 1200 µg•hr/L, 1225 µg•hr/L, 1250 µg•hr/L, 1275 µg•hr/L, 1300 µg•hr/L, 1325 µg•hr/L, 1350 µg•hr/L, 1375 µg•hr/L, 1400 µg•hr/L, 1425 µg•hr/L, 1450 µg•hr/L, 1475 µg•hr/L, 1500 µg•hr/L, 1525 µg•hr/L, 1550 µg•hr/L, 1575 µg•hr/L, 1600 µg•hr/L, 1625 µg•hr/L, 1650 µg•hr/L, 1668 µg•hr/L, 1675 µg•hr/L, 1700 µg•hr/L, 1725 µg•hr/L, 1750 µg•hr/L, 1775 µg•hr/L, 1800 µg•hr/L, 1825 µg•hr/L, 1850 µg•hr/L, 1875 µg•hr/L, 1900 µg•hr/L, 1925 µg•hr/L, 1950 µg•hr/L, 1975 µg•hr/L, 2000 µg•hr/L, 2025 µg•hr/L, 2050 µg•hr/L, 2075 µg•hr/L, 2100 µg•hr/L, 2125 µg•hr/L, 2150 µg•hr/L, 2175 µg•hr/L, 2200 µg•hr/L, 2225 µg•hr/L, 2250 µg•hr/L, 2275 µg•hr/L, 2300 µg•hr/L, 2325 µg•hr/L, 2350 µg•hr/L, 2375 µg•hr/L, 2400 µg•hr/L, 2425 µg•hr/L, 2450 µg•hr/L, 2475 µg•hr/L, 2500 µg•hr/L, 2525 µg•hr/L, 2550 µg•hr/L, 2575 µg•hr/L, 2600 µg•hr/L, 2625 µg•hr/L, 2650 µg•hr/L, 2675 µg•hr/L, 2700 µg•hr/L, 2725 µg•hr/L, 2750 µg•hr/L, 2775 µg•hr/L, 2800 µg•hr/L, 2825 µg•hr/L, 2850 µg•hr/L, 2875 µg•hr/L, 2900 µg•hr/L, 2925 µg•hr/L, 2950 µg•hr/L, 2975 µg•hr/L, 3000 µg•hr/L, 3025 µg•hr/L, 3050 µg•hr/L, 3075 µg•hr/L, 3100 µg•hr/L, 3125 µg•hr/L, 3150 µg•hr/L, 3175 µg•hr/L, 3200 µg•hr/L, 3225 µg•hr/L, 3250 µg•hr/L, 3275 µg•hr/L, 3300 µg•hr/L, 3325 µg•hr/L, 3350 µg•hr/L, 3375 µg•hr/L, 3400 µg•hr/L, 3425 µg•hr/L, 3450 µg•hr/L, 3475 µg•hr/L, 3500 µg•hr/L, 3525 µg•hr/L, 3550 µg•hr/L, 3575 µg•hr/L, 3600 µg•hr/L, 3625 µg•hr/L, 3650 µg•hr/L, 3675 µg•hr/L, 3700 µg•hr/L, 3725 µg•hr/L, 3750 µg•hr/L, 3775 µg•hr/L, 3792 µg•hr/L, or about 3800 µg•hr/L, inclusive of all ranges and subranges there between.

Thus in some aspects, the present disclosure is directed to the administration of a reduced rivaroxaban dose (relative to the recommended dose for an otherwise identical (or the same) patient not concomitantly administered verapamil as described herein), in which the reduced rivaroxaban dose provides an AUC between about 1668 µg•hr/L to about 3792 µg•hr/L, inclusive of all ranges and subranges therebetween. In some aspects, the present disclosure is directed to the administration of a reduced rivaroxaban dose (relative to the recommended dose for an otherwise identical (or the same) patient not concomitantly administered verapamil as described herein), in which the reduced rivaroxaban dose produces an AUC between about 2000 µg•hr/L-2400 µg•hr/L.

In some aspects, the present disclosure is directed to methods of reducing the rivaroxaban dose of patients who are concomitantly administered verapamil (relative to the recommended dose for an otherwise identical (or the same) patient not concomitantly administered verapamil as described herein), wherein the reduced dose causes the patient to maintain the approximately the same maximum prothrombin time compared to an otherwise identical (or the same) patient who is not concomitantly administered verapamil and is receiving the recommended dose of rivaroxaban. Thus in some aspects, the present disclosure is directed to a reduced rivaroxaban dose (relative to the recommended dose for an otherwise identical (or the same) patient not concomitantly administered verapamil as described herein), wherein the patient's maximum prothrombin time is lower than about 20s, 21s, 22s, 23s, 24s, 25s, 26s, 27s, 28s, 29s, or about 30s.

In some aspects, the present disclosure is directed to methods of reducing the rivaroxaban dose of patients who are concomitantly administered verapamil (relative to the recommended dose for an otherwise identical (or the same) patient not concomitantly administered verapamil as described herein), wherein the reduced dose of rivaroxaban provides a % risk of major bleeding, for example using the relation shown in **FIG. 5**, which is less than about 12%, less than about 11%, less than about 10%, less than about 9%, less than about 8%, less than about 7%, less than about 6%, less than about 5%, less than about 4.5%, less than about 4%, less than about 3.5%, less than about 3%, less than about 2.5%, less than about 2%, less than about 1.5%, or less than about 1%. In still other aspects, said patients hereinabove may have normal renal function, or mild, moderate, or severe renal impairment.

In some aspects, the present disclosure is directed to methods of reducing the rivaroxaban dose of patients with mild renal impairment relative to the recommended dose for an otherwise identical patient having normal renal function, wherein the reduced dose of rivaroxaban provides a % risk of major bleeding, for example using the relation shown in **FIG. 5**, which is less than about 12%, less than about 11%, less than about 10%, less than about 9%, less than about 8%, less than about 7%, less than about 6%, less than about 5%, less than about 4.5%, less than about 4%, less than about 3.5%, less than about 3%, less than about 2.5%, less than about 2%, less than about 1.5%, or less than about 1%.

Thus in some aspects, the present disclosure is directed to the administration of a reduced rivaroxaban dose (relative to the recommended dose for an otherwise identical (or the same) patient not concomitantly administered verapamil as described herein), in which the reduced rivaroxaban dose causes said patient to exhibit a maximum prothrombin time between about 20s-30s, inclusive of all ranges and subranges therebetween. For example, in some aspects, the present disclosure is directed to the administration of a reduced rivaroxaban dose (relative to the recommended dose for an otherwise identical (or the same) patient not concomitantly administered verapamil as described herein), in which the reduced rivaroxaban dose produces a maximum prothrombin time between about 20s-30s.

The present disclosure is also directed to a method of treating a patient in need of treatment with a Factor Xa inhibitor and a calcium channel blocker (such as verapamil), wherein the treatment is to reduce the risk of stroke and systemic embolism in a patient with non valvular atrial fibrillation, the treatment of deep vein thrombosis (DVT), pulmonary embolism (PE), and for the reduction of the risk of recurrence of DVT and of PE, and for the prophylaxis of DVT which may lead to PE in patients undergoing knee or hip replacement surgery. In some embodiments, the method can include administering a daily dose of about 100, 120, 180, 240 300, 360, or about 480 mg verapamil, including all ranges and subranges therebetween to the patient and administering a dose of rivaroxaban (for example with the evening meal) which is less than the dose recommended for an otherwise identical (or the same) patient who is not concomitantly administered verapamil. In some embodiments, the patient has mild, moderate, or severe renal impairment.

The present disclosure is also directed to a method of treating a person in need of a calcium channel blocker comprising administering a dose of rivaroxaban (for example with the evening meal) which is less than the dose recommended for an otherwise identical (or the same) patient who is not concomitantly administered verapamil. The present disclosure is also directed to a method of treating essential hypertension in a patient. In some embodiments, the method can include administering a daily dose of about 100, 120, 180, 240, about 360, or about 480 mg verapamil, including all ranges and subranges therebetween to the patient, and administering a dose of rivaroxaban (for example with the evening meal) which is less than the dose recommended for an otherwise identical (or the same) patient who is not concomitantly administered verapamil. In some embodiments, the patient can have mild, moderate, or severe renal impairment and is in need of anticoagulant therapy.

The present disclosure also directs to a method of treating a patient in need of treatment with a factor Xa inhibitor and a calcium channel blocker (such as verapamil), wherein the treatment is to reduce the risk of stroke and systemic embolism in a patient with non valvular atrial fibrillation, the treatment of deep vein thrombosis (DVT), pulmonary embolism (PE), and for the reduction of the risk of recurrence of DVT and of PE, and for the prophylaxis of DVT which may lead to PE in patients undergoing knee or hip replacement surgery. In some aspects, the method can include administering a daily dose of a calcium channel blocker to the patient and administering a dose of rivaroxaban (for example with the evening meal) which is less than the dose recommended for an otherwise identical (or the same) patient who is not concomitantly administered verapamil. In some aspects, the patient has mild, moderate, or severe renal impairment.

The present disclosure is also directed to a method of treating essential hypertension in a patient. In some aspects, the method can include administering a calcium channel blocker to the patient, and administering a dose of rivaroxaban (for example with the evening meal) which is less than the dose recommended for an otherwise identical (or the same) patient who is not concomitantly administered verapamil. In some aspects, the patient can have mild, moderate, or severe renal impairment and is in need of anticoagulant therapy.

In some aspects, the patient has a CL_{Cr} of greater than or equal to about 90 mL/min, greater than or equal to about 80 mL/min, about 50-79 mL/min, about 60-89 mL/min, about 30-49 mL/min, about 30-59 mL/min, less than about 30 mL/min, about 15-29 mL/min, or less than about 15 mL/min, including all ranges and all subranges therebetween. In some aspects, the dose of rivaroxaban is less than the dose recommended for an otherwise identical (or the same) patient who is not concomitantly administered verapamil, and about 0%, at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or at least about 99.5% and all ranges and subranges therebetween of the dose recommended for an otherwise identical (or the same) patient who is not concomitantly administered verapamil.

In some aspects, the patient has a CL_{Cr} of greater than or equal to about 90 mL/min, greater than or equal to about 80 mL/min, about 50-79 mL/min, about 60-89 mL/min, about 30-49 mL/min, about 30-59 mL/min, less than about 30 mL/min, about 15-29 mL/min, or less than about 15 mL/min, including all ranges and all subranges therebetween and the dose of rivaroxaban is less than: about 19.9 mg, about 19 mg, about 18 mg, about 17.5 mg, about 17 mg, about 16 mg, about 15 mg, about 14 mg, about 13 mg, about 12.5 mg, about 12 mg, about 11 mg, about 10 mg, about 9 mg, about 8 mg, about 7.5 mg, about 7 mg, about 6 mg, about 5 mg, about 4 mg, about 3 mg, about 2.5 mg, about 2 mg, about 1 mg, or about 0 mg, including all ranges and subranges therebetween. In some aspects, the dose of rivaroxaban is less than the dose recommended for an otherwise identical (or the same) patient who is not concomitantly administered verapamil, and about 0 mg, at least about 1 mg, at least about 2 mg, at least about 2.5 mg, at least about 3 mg, at least about 4 mg, at least about 5 mg, at least about 6 mg, at least about 7 mg, at least about 7.5 mg, at least about 8 mg, at least about 9 mg, at least about 10 mg, at least about 11 mg, at least about 12 mg, at least about 12.5 mg, at least about 13 mg, at least about 14 mg, or less than about 15 mg, including all ranges and subranges therebetween. In some embodiments, the daily dose of verapamil is 100 mg. In some embodiments, the daily dose of verapamil is 120 mg. In some embodiments, the daily dose of verapamil is 180mg. In some embodiments, the daily dose of verapamil is 240 mg. In some embodiments, the daily dose of verapamil is 360 mg. In some embodiments, the daily dose of verapamil is 480 mg.

The present disclosure is also directed to a method of treating coronary artery disease in a patient. In some aspects, the present disclosure is directed to a method of treating peripheral artery disease in a patient. In some aspects, the present disclosure is directed to a method of treating atrial fibrillation. In some aspects, the present disclosure is directed to a method of treating deep vein thrombosis. In some aspects, the present disclosure is directed to a method of treating patients undergoing major orthopedic surgery. In some aspects, the present disclosure is directed to a method of deep vein thrombosis prophylaxis. In some aspects, the present disclosure is directed to a method of deep vein thrombosis prophylaxis after abdominal surgery. In some aspects, the present disclosure is directed to a method of deep vein thrombosis prophylaxis after hip replacement surgery. In some aspects, the present disclosure is directed to a method of deep vein thrombosis prophylaxis after knee replacement surgery. In some aspects, the present disclosure is directed to a method of treating a deep vein thrombosis recurring event. In some aspects, the present disclosure is directed to a method of treating a heart attack patient. In some aspects, the present disclosure is directed to a method of prevention of thromboembolism in atrial fibrillation. In some aspects, the present disclosure is directed to a method of treating pulmonary embolism. In some aspects, the present disclosure is directed to a method of treating a pulmonary embolism recurring event. In some aspects, the present disclosure is directed to thromboembolic stroke prophylaxis. In some aspects, the present disclosure is directed to a method of treating venous thromboembolism. In some aspects, the present disclosure is directed to a method of prevention of ischemic stroke. In some aspects, the present disclosure is directed to a method of treating recurring myocardial infarction. In some aspects, the present disclosure is directed to a method of treating antiphospholipid antibody syndrome. In some aspects, the present disclosure is directed to a method of treating sickle cell disease. In some aspects, the present disclosure is directed to a method of prevention and treatment of venous thromboembolism in cancer patients. In some aspects, the present disclosure is directed to a method of treating cancer associated thrombosis. In some aspects, the present disclosure is directed to a method of treating cancer patients with central line associated clots in the upper extremity. In some aspects, the present disclosure is directed to a method of reducing post-discharge venous thromboembolism risk in medically ill patients. In some aspects, the present disclosure is directed to a method of treating young children with venous thrombosis (6 months - 5 years). In some aspects, the present disclosure is directed to a method of treating arterial or venous thrombosis in children from birth to less than 6 months. In some aspects, the present disclosure is directed to a method of treating thrombophylaxis in pediatric patients 2 to 8 years of age after the fontan procedure. In some aspects, the present disclosure is directed to a method of treating valvular heart disease and atrial fibrillation. In some aspects, the present disclosure is directed to a method of treating patients with atrial fibrillation with bioprosthetic mitral valves. In some aspects, the present disclosure is directed to a method of treating symptomatic isolated distal deep vein thrombosis. In some aspects, the present disclosure is directed to a method of treating superficial vein thrombosis. In some aspects, the present disclosure is directed to a method of prevention of thrombosis after replacement of the aortic valve with a biological valve prosthesis. In some aspects, the present disclosure is directed to a method of prevention of recurrence of stent thrombosis and cardiovascular events in patients with atrial fibrillation complicated with stable coronary artery disease. In some aspects, the present disclosure is directed to a method of treating splanchnic vein thrombosis. In some aspects, the present disclosure is directed to a method of prevention of recurrent thrombosis in patients with chronic portal vein thrombosis. In some aspects, the present disclosure is directed to a method of prevention of recurrent symptomatic venous thromboembolism in patients with symptomatic deep vein thrombosis or pulmonary embolism. In some aspects, the present disclosure is directed to a method of treating acute ischemic stroke with atrial fibrillation. In some aspects, the present disclosure is directed to a method of venous thromboembolism prophylaxis in patients undergoing non-major orthopedic surgery. In some aspects, the present disclosure is directed to a method of reducing the risk of major thrombotic vascular events in subjects with peripheral artery disease undergoing peripheral revascularization procedures of the lower extremities. In some aspects, the present disclosure is directed to a method of prevention of cardiovascular events in patients with nonvalvular atrial fibrillation scheduled for cardioversion. In some aspects, the present disclosure is directed to a method of reducing the risk of death, myocardial infarction, or stroke in participants with heart failure and coronary artery disease following an episode of decompensated heart failure. In some aspects, the present disclosure is directed to a method of preventing major cardiovascular events in coronary or peripheral artery disease. In some aspects, the present disclosure is directed to a method of reducing the risk of cardiovascular death, myocardial infarction, or stroke in patients with recent acute coronary syndrome. In some aspects, the present disclosure is directed to a method of prevention of the composite of stroke or systemic embolism in patients with rheumatic valvular heart disease (RVHD) with atrial fibrillation or flutter who are unsuitable for vitamin K antagonist therapy, or in patients with RVHD without AF or flutter with at least one of the following: left atrial enlargement ≥ 5.5 cm, left atrial spontaneous echo contrast, left atrial thrombus, or frequent ectopic atrial activity (>1000/24 hours) on Holter ECG. In some aspects, the present disclosure is directed to a method of prevention of restenosis after infrainguinal percutaneous transluminal angioplasty for critical limb ischemia. In some aspects, the present disclosure is directed to a method of decreasing the risk of cardiovascular disease, myocardial infarction, revascularization, ischemic stroke, and systemic embolism.

In some aspects, the present disclosure is directed to treatment of a patient with a medical condition requiring treatment with a calcium channel blocker such as verapamil (in conjunction with a Factor Xa inhibitor), including management of essential hypertension. In some aspects, the present disclosure is directed to treatment of hypertension. In some aspects, the present disclosure is directed to treatment of pulmonary hypertension. In some aspects, the present disclosure is directed to prevention and treatment of recurrent and paroxysmal supraventricular tachycardia. In some aspects, the present disclosure is directed to management of supraventricular tachycardia. In some aspects, the present disclosure is directed to treatment of atrial tachycardia. In some aspects, the present disclosure is directed to treatment of junctional tachycardia. In some aspects, the present disclosure is directed to treatment of cerebral vasospasm. In some aspects, the present disclosure is directed to treatment of hypertrophic cardiomyopathy. In some aspects, the present disclosure is directed to treatment of chronic stable angina pectoris. In some aspects, the present disclosure is directed to treatment of unstable angina pectoris. In some aspects, the present disclosure is directed to management of Prinzmetal variant angina. In some aspects, the present disclosure is directed to ventricular rate control in atrial fibrillation/flutter. In some aspects, the present disclosure is directed to prevention of cluster headache. In some aspects, the present disclosure is directed to prevention of migraine. In some aspects, the present disclosure is directed to prevention of myocardial infarction in patients with preserved left ventricular function. In some aspects, the present disclosure is directed to management of manic manifestations of bipolar disorder. In some aspects, the present disclosure is directed to treatment of Raynaud's disease. In some aspects, the present disclosure is directed to treatment of coronary artery disease. In some aspects, the present disclosure is directed to treatment of subarachnoid hemorrhage. In some aspects, the present disclosure is directed to treatment of Dravet Syndrome. In some aspects, the present disclosure is directed to beta cell survival therapy in Type I diabetes. In some aspects, the present disclosure is directed to treatment of vestibular migraine. In some aspects, the present disclosure is directed to treatment of chronic subjective dizziness. In some aspects, the present disclosure is directed to treatment of erectile dysfunction. In some aspects, the present disclosure is directed to prevention of keloid recurrence. In some aspects, the present disclosure is directed to treatment of refractory epilepsy. In some aspects, the present disclosure is directed to treatment of refractory meningioma. In some aspects, the present disclosure is directed to treatment of chronic heart failure secondary to non-ischemic cardiomyopathy. In some aspects, the present disclosure is directed to treatment of relapsed or refractory Hodgkin lymphoma. In some aspects, the present disclosure is directed to treatment of Marfan Syndrome. In some aspects, the present disclosure is directed to treatment of treatment-resistant mania. In some aspects, the present disclosure is directed to prevention of kidney disease in diabetic patients. In some aspects, the present disclosure is directed to treatment of Metabolic Syndrome. In some aspects, the present disclosure is directed to treatment of hypoglycemia following gastric bypass.

In some aspects, the method can include administering rivaroxaban only to the patient. In some aspects, the administering can be once daily. In some aspects, the single dose of rivaroxaban is about 19.9 mg, about 19 mg, about 18 mg, about 17.5 mg, about 17 mg, about 16 mg, about 15 mg, about 14 mg, about 13 mg, about 12.5 mg, about 12 mg, about 11 mg, about 10 mg, about 9 mg, about 8 mg, about 7.5 mg, about 7 mg, about 6 mg, about 5 mg, about 4 mg, about 3 mg, about 2.5 mg, about 2 mg, about 1 mg, about 0 mg, or any ranges and subranges therebetween. In some aspects, the daily dose of rivaroxaban is 0 mg, 2.5 mg, 5 mg, 7.5 mg, 10 mg, 12.5 mg, or 15 mg. In some aspects, the method can include administering rivaroxaban with aspirin daily. In some aspects, the frequency of administering rivaroxaban is twice daily. In some aspects, the frequency of administering aspirin is once daily.

In some aspects, when aspirin is coadministered with rivaroxaban as described herein, the single dose of aspirin can be about 150 mg, about 140 mg, about 130 mg, about 120 mg, about 110 mg, about 100 mg, about 90 mg, about 80 mg, about 70 mg, or any ranges and subranges therebetween. In some aspects, the daily dose of aspirin is 100 mg. In some aspects, the method can include administering aspirin only to the patient. In some aspects, the administering can be once daily. In some aspects, the daily dose of aspirin can be 150 mg, about 140 mg, about 130 mg, about 120 mg, about 110 mg, about 100 mg, about 90 mg, about 80 mg, about 70 mg, or any ranges and subranges therebetween. In some aspects, the daily dose of aspirin is 100 mg.

In some aspects, the methods described herein can prevent major adverse cardiac events. In some aspects, the adverse cardiac events can include, but are not limited to cardiovascular death, myocardial infarction and stroke. In some embodiments, the methods can be used to prevent stroke and systemic embolism in adult patients. In some embodiments, the patients have non-valvular atrial fibrillation (AF) with one or more risk factors. In some embodiments, the methods can be used to prevent or treat pulmonary embolism (PE) in adults. In some embodiments, the methods can be used to prevent or treat deep vein thrombosis (DVT) in adults. In some embodiments, the methods can be used to concurrently prevent or treat recurrent deep vein thrombosis (DVT) and pulmonary embolism (PE) in adults. In some embodiments, the methods can be used to prevent or treat venous thromboembolism (VTE) in adult patients. In other embodiments, the adult patients undergo elective hip replacement surgery.

In some aspects, the methods can be used to prevent or treat venous thromboembolism (VTE) in adult patients. In other aspects, the adult patients undergo elective knee replacement surgery. In some aspects, the methods can be used to prevent or treat atherothrombotic events. In some aspects, the atherothrombotic events can include but are not limited to cardiovascular death, myocardial infarction or stroke. In some aspects, the prevention or treatment can be after an Acute Coronary Syndrome in adult patients. In some aspects, the adult patients have elevated cardiac biomarkers. In other aspects, the adult patients have no prior stroke or transient ischaemic attack (TIA) when co-administered with acetylsalicylic acid (ASA) alone. In some aspects, the adult patients have no prior stroke or transient ischaemic attack (TIA) when co-administered with acetylsalicylic acid (ASA) plus clopidogrel. In some aspects, the adult patients have no prior stroke or transient ischaemic attack (TIA) when co-administered with acetylsalicylic acid (ASA) plus or ticlopidine.

### EXAMPLES

### Example 1 - Oral Verapamil and Rivaroxaban Drug-Drug Interaction

A volunteer patient trial using both healthy and mildly renally impaired subjects is conducted in order to assess the pharmacokinetic and pharmacodynamic consequences of rivaroxaban and verapamil concomitant administration. This initial study is a non-randomized, open label controlled study of the rivaroxaban-verapamil drug-drug interaction.

During the initial portion of the study, patients are administered a single 20 mg dose of rivaroxaban with their breakfast without co-administration of verapamil. These patients are subjected to various one-time and longitudinal pharmacokinetic (PK) and pharmacodynamic (PD) analyses. Patient plasma levels of rivaroxaban are tracked for at least 72 hours after receiving a rivaroxaban dose. Cₘₐₓ and AUC values for each dose are calculated. Patients also undergo a general physical exam to determine overall health and well-being.

Additional pharmacodynamic metrics are also gathered for each patient, including, but not limited to: prothrombin time (using the STA® Neoplastin® method) and Factor Xa activity. Protocols for each of these analyses have been described in (Nielsen et al 2015. Renal Function and non-vitamin K oral anticoagulants in comparison with warfarin on safety and efficacy outcomes in atrial fibrillation patients: a systemic review and meta-regression analysis. Clin Res Cardiol May 104(5): 418-429).

During the second portion of the study, patients are administered a verapamil dose of 180 mg on Day 8, 240 mg on Day 9, and 360 mg on Days 10-15 to achieve steady state plasma concentrations of verapamil. On Day 15, patients will receive 360 mg verapamil together with the 20 mg rivaroxaban doses described for the initial portion of the study. All the pharmacokinetic and pharmacodynamics analyses are repeated on patients receiving this treatment.

PK and PD results for the rivaroxaban single treatment and rivaroxaban-verapamil concomitant treatment are compared to determine the safety profile of the resulting rivaroxaban doses.

The results show that concomitant administration of verapamil with rivaroxaban produces higher rivaroxaban plasma concentrations, prolonged PT, and increased Factor Xa inhibition.

### Example 2 - Pharmacokinetics and Plasma Rivaroxaban Concentrations in Subjects with Mild Renal Impairment and Normal Renal Function

To estimate the effect of steady-state verapamil on the pharmacokinetics, pharmacodynamics, and safety of rivaroxaban, a study was conducted on subjects with either mild renal impairment or normal renal function. Subjects with either mild renal impairment or with normal renal function were enrolled. Subjects received a single 20 mg dose of rivaroxaban ("Period I") on the first day of the study, and after a washout period, a single 20 mg dose of rivaroxaban and a 360 mg dose of verapamil at steady state ("Period III").

Renal function of the subjects was determined by measuring creatinine clearance (CLcr) rates. Subjects who had CLcr ≥90 ml/min were categorized as having normal renal function whereas subjects who had CLcr 50-79 ml/min were categorized as mild renal impaired. Blood samples were collected and processed according to the standard protocols. Plasma rivaroxaban concentrations were measured on Day 1 of the study at 0.5, 1, 2, 3, 4, 6, 8, and 12 hours, Day 2 at 24 and 36 hours, Day 3 at 48 hours, and Day 4 at 72 hours and are shown in **Tables 1A-8 below.**

| **Table 1A: Plasma Rivaroxaban Concentration-Time Data and Summary Statistics for Treatment Period I Separated by Renal Function Group (PK Analysis Set)** | | | | | | | |
|---|---|---|---|---|---|---|---|
| **Group 1: Mild Renal Impairment, (N=14)** | | | | | | | |
| Subject | Time Point | | | | | | |
| | Day 1, | Day 1, | Day 1, | Day 1, | Day 1, | Day 1, | Day 1, |
| | 0 hr | 0.5 hr | 1 hr | 2 hr | 3 hr | 4 hr | 6 hr |
| 1101 | 0 | 83.8 | 151 | 251 | 241 | 218 | 114 |
| 1102 | 0 | 207 | 278 | 207 | 197 | 204 | 161 |
| 1103 | 0 | 170 | 187 | 185 | 196 | 181 | 126 |
| 1104 | 0 | 235 | 218 | 184 | 199 | 182 | 119 |
| 1106 | 0 | 24.0 | 153 | 232 | 238 | 172 | 172 |
| 1108 | 0 | 174 | 168 | 170 | 297 | 344 | 274 |
| 1110 | 0 | 201 | 306 | 248 | 206 | 219 | 157 |
| 1111 | 0 | 245 | 247 | 211 | 268 | 247 | 164 |
| 1114 | 0 | 215 | 308 | 232 | 184 | 192 | 103 |
| 1115 | 0 | 170 | 133 | 187 | 215 | 190 | 133 |
| 1116 | 0 | 130 | 132 | 120 | 97.9 | 90.6 | 55.7 |
| 1118 | 0 | 326 | 448 | 333 | 315 | 316 | 128 |
| 2101 | 0 | 208 | 217 | 208 | 176 | 165 | 114 |
| 2104 | 0 | 23.4 | 25.7 | 40.6 | 55.8 | 79.6 | 232 |
| n | 14 | 14 | 14 | 14 | 14 | 14 | 14 |
| n<LLOQ | 14 | 0 | 0 | 0 | 0 | 0 | 0 |
| CV% | NC | 48.8 | 48.3 | 33.4 | 33.6 | 35.7 | 37.3 |
| AM | 0 | 172 | 212 | 201 | 206 | 200 | 147 |
| SD | 0 | 84.0 | 102 | 66.9 | 69.2 | 71.5 | 54.6 |
| Median | 0 | 188 | 202 | 208 | 202 | 191 | 130 |
| Minimum | 0 | 23.4 | 25.7 | 40.6 | 55.8 | 79.6 | 55.7 |
| Maximum | 0 | 326 | 448 | 333 | 315 | 344 | 274 |

| | | | | | | | |
|---|---|---|---|---|---|---|---|
| Lower limit of quantitation (LLOQ) = 5 (µg/L). N: Number of subjects, n: Number of non-missing observations, hr: Hour, BLQ: Below the limit of quantitation, AM: Arithmetic mean, SD: Standard deviation. BLQ values are treated as zero. NC = Not calculated Values expressed in µg/L CV% = (SD/Mean) × 100, where SD and mean are standard deviation and arithmetic mean of untransformed data. Summary statistics for Day 1, 0 hr are based on greater than 30% imputed values. n<LLOQ = Number of BLQs imputed as zero. | | | | | | | |

| **Table 1B: Plasma Rivaroxaban Concentration-Time Data and Summary Statistics for Treatment Period I Separated by Renal Function Group (PK Analysis Set)** | | | | | | |
|---|---|---|---|---|---|---|
| Group 1: Mild Renal Impairment, (N=14) | | | | | | |
| Subject | Time Point | | | | | |
| | Day 1, | Day 1, | Day 2, | Day 2, | Day 3, | Day 4, |
| | 8 hr | 12 hr | 24 hr | 36 hr | 48 hr | 72 hr |
| 1101 | 76.8 | 52.9 | 36.5 | 14.9 | 8.49 | 0 |
| 1102 | 131 | 84.9 | 53.7 | 10.0 | 9.67 | 0 |
| 1103 | 124 | 61.5 | 52.9 | 8.92 | 0 | 0 |
| 1104 | 89.5 | 38.8 | 41.9 | 10.8 | 6.49 | 0 |
| 1106 | 157 | 92.7 | 19.9 | 12.1 | 6.13 | 0 |
| 1108 | 221 | 132 | 50.3 | 17.9 | 11.4 | 0 |
| 1110 | 143 | 89.1 | 52.5 | 21.7 | 17.0 | 0 |
| 1111 | 125 | 72.7 | 38.6 | 18.5 | 15.5 | 0 |
| 1114 | 66.4 | 66.8 | 39.1 | 14.8 | 6.71 | 0 |
| 1115 | 122 | 103 | 33.9 | 11.2 | 0 | 0 |
| 1116 | 47.6 | 29.4 | 10.1 | 0 | 0 | 0 |
| 1118 | 102 | 49.9 | 42.2 | 21.5 | 21.4 | 12.2 |
| 2101 | 90.2 | 51.9 | 21.1 | 7.44 | 7.10 | 0 |
| 2104 | 223 | 97.7 | 18.0 | 0 | 0 | 0 |
| n | 14 | 14 | 14 | 14 | 14 | 14 |
| n<LLOQ | 0 | 0 | 0 | 2 | 4 | 13 |
| CV% | 42.2 | 38.7 | 39.1 | 56.1 | 86.2 | 374 |
| AM | 123 | 73.1 | 36.5 | 12.1 | 7.85 | 0.871 |
| SD | 51.8 | 28.3 | 14.3 | 6.80 | 6.77 | 3.26 |
| Median | 123 | 69.8 | 38.8 | 11.6 | 6.90 | 0 |
| Minimum | 47.6 | 29.4 | 10.1 | 0 | 0 | 0 |
| Maximum | 223 | 132 | 53.7 | 21.7 | 21.4 | 12.2 |

| | | | | | | |
|---|---|---|---|---|---|---|
| Lower limit of quantitation (LLOQ) = 5 (µg/L). N: Number of subjects, n: Number of non-missing observations, hr: Hour, BLQ: Below the limit of quantitation, AM Arithmetic mean, SD: Standard deviation. BLQ values are treated as zero. Values expressed in µg/L CV% = (SD/Mean) × 100, where SD and mean are standard deviation and arithmetic mean of untransformed data. Summary statistics for Day 4, 72 hr are based on greater than 30% imputed values. n<LLOQ = Number of BLQs imputed as zero. | | | | | | |

| **Table 2A: Plasma Rivaroxaban Concentration-Time Data and Summary Statistics for Treatment Period I Separated by Renal Function Group Excluding Outlier Sample (PK Analysis Set)** | | | | | | | |
|---|---|---|---|---|---|---|---|
| Group 2: Normal Renal Function, (N=13) | | | | | | | |
| Subject | Time Point | | | | | | |
| | Day 1, | Day 1, | Day 1, | Day 1, | Day 1, | Day 1, | Day 1, |
| | 0 hr | 0.5 hr | 1 hr | 2 hr | 3 hr | 4 hr | 6 hr |
| 01105 | 0 | 107 | 160 | 187 | 290 | 336 | 230 |
| 01107 | 0 | 194 | 252 | 229 | 242 | 297 | 172 |
| 01109 | 0 | 117 | 249 | 204 | 307 | 276 | 162 |
| 01112 | 0 | 71.8 | 175 | 195 | 183 | 169 | 119 |
| 01113 | 0 | 169 | 178 | 163 | 183 | 148 | 91.9 |
| 01117 | 0 | 429 | 297 | 195 | 164 | 155 | 116 |
| 01119 | 0 | 197 | 175 | 208 | 263 | 352 | 180 |
| 01120 | 0 | 60.3 | 214 | 196 | 211 | 187 | 131 |
| 01121 | 0 | 218 | 195 | 151 | 133 | 115 | 67.5 |
| 02102 | 0 | 67.1 | 142 | 212 | 202 | 171 | 142 |
| 02103 | 0 | 129 | 214 | 201 | 230 | 183 | 133 |
| 02105 | 0 | 43.4 | 104 | 188 | 245 | 292 | 256 |
| 02106 | 0 | 26.2 | 80.5 | 141 | 215 | 266 | 218 |
| n | 13 | 13 | 13 | 13 | 13 | 13 | 13 |
| n<LLOQ | 13 | 0 | 0 | 0 | 0 | 0 | 0 |
| CV% | NC | 75.9 | 32.0 | 13.1 | 22.4 | 34.8 | 35.5 |
| AM | 0 | 141 | 187 | 190 | 221 | 227 | 155 |
| SD | 0 | 107 | 59.9 | 24.8 | 49.5 | 78.8 | 55.1 |
| Median | 0 | 117 | 178 | 195 | 215 | 187 | 142 |
| Minimum | 0 | 26.2 | 80.5 | 141 | 133 | 115 | 67.5 |
| Maximum | 0 | 429 | 297 | 229 | 307 | 352 | 256 |

| | | | | | | | |
|---|---|---|---|---|---|---|---|
| Lower limit of quantitation (LLOQ) = 5 (µg/L). N: Number of subjects, n: Number of non-missing observations, hr: Hour, BLQ: Below the limit of quantitation, AM Arithmetic mean, SD: Standard deviation. %CVb = 100 * SQRT [ex[(S^2)-1]], where S is the standard deviation of the data on a log scale. BLQ values are treated as zero. NC = Not calculated Values expressed in µg/L Summary statistics for Day 1, 0 hr are based on greater than 30% imputed values. n<LLOQ = Number of BLQs imputed as zero. | | | | | | | |

| **Table 2B: Plasma Rivaroxaban Concentration-Time Data and Summary Statistics for Treatment Period I Separated by Renal Function Group Excluding Outlier Sample (PK Analysis Set)** | | | | | | |
|---|---|---|---|---|---|---|
| Group 2: Normal Renal Function, (N=13) | | | | | | |
| Subject | Time Point | | | | | |
| | Day 1, | Day 1, | Day 2, | Day 2, | Day 3, | Day 4, |
| | 8 hr | 12 hr | 24 hr | 36 hr | 48 hr | 72 hr |
| 01105 | 202 | 94.7 | 27.1 | 9.61 | 0 | 0 |
| 01107 | 114 | 72.3 | 25.1 | 5.30 | 0 | 0 |
| 01109 | 138 | 110 | 23.8 | 0 | 0 | 0 |
| 01112 | 88.9 | 53.9 | 20.0 | 6.02 | 0 | 0 |
| 01113 | 71.5 | 26.5 | 21.3 | 7.48 | 0 | 0 |
| 01117 | 104 | 47.3 | 32.0 | 20.6 | 14.3 | 0 |
| 01119 | 138 | 77.3 | 24.3 | 7.19 | 93.1† | 0 |
| 01120 | 114 | 50.7 | 17.3 | 5.98 | 0 | 0 |
| 01121 | 52.7 | 38.0 | 19.4 | 9.86 | 7.00 | 0 |
| 02102 | 109 | 66.6 | 22.6 | 5.26 | 0 | 0 |
| 02103 | 134 | 42.6 | 16.6 | 8.97 | 5.53 | 0 |
| 02105 | 183 | 91.9 | 25.2 | 8.16 | 5.53 | 0 |
| 02106 | 150 | 86.9 | 35.4 | 12.4 | 8.51 | 0 |
| n | 13 | 13 | 13 | 13 | 12 | 13 |
| n<LLOQ | 0 | 0 | 0 | 1 | 7 | 13 |
| CV% | 33.7 | 38.2 | 22.7 | 58.0 | 139 | NC |
| AM | 123 | 66.1 | 23.9 | 8.22 | 3.41 | 0 |
| SD | 41.4 | 25.3 | 5.42 | 4.76 | 4.75 | 0 |
| Median | 114 | 66.6 | 23.8 | 7.48 | 0 | 0 |
| Minimum | 52.7 | 26.5 | 16.6 | 0 | 0 | 0 |
| Maximum | 202 | 110 | 35.4 | 20.6 | 14.3 | 0 |

| | | | | | | |
|---|---|---|---|---|---|---|
| Lower limit of quantitation (LLOQ) = 5 (µg/L). N: Number of subjects, n: Number of non-missing observations, hr: Hour, BLQ: Below the limit of quantitation, AM Arithmetic mean, SD: Standard deviation. %CVb = 100 * SQRT [ex[(S^2)-1]], where S is the standard deviation of the data on a log scale. BLQ values are treated as zero. NS = No sample; NC = Not calculated Values expressed in µg/L † Outlier sample excluded from summary statitics Summary statistics for Day 3, 48 hr, Day 4, 72 hr are based on greater than 30% imputed values. n<LLOQ = Number of BLQs imputed as zero. | | | | | | |

| **Table 3A: Plasma Rivaroxaban Concentration-Time Data and Summary Statistics for Treatment Period III Separated by Renal Function Group (PK Analysis Set)** | | | | | | | |
|---|---|---|---|---|---|---|---|
| Group 1: Mild Renal Impairment, (N=11) | | | | | | | |
| Subject | Time Point | | | | | | |
| | Day 1, | Day 1, | Day 1, | Day 1, | Day 1, | Day 1, | Day 1, |
| | 0 hr | 0.5 hr | 1 hr | 2 hr | 3 hr | 4 hr | 6 hr |
| 1101 | 0 | 154 | 211 | 196 | 238 | 247 | 154 |
| 1103 | 0 | 203 | 293 | 300 | 285 | 257 | 188 |
| 1104 | 0 | 278 | 260 | 268 | 276 | 214 | 164 |
| 1106 | 0 | 10.2 | 42.6 | 109 | 213 | 280 | 233 |
| 1108 | 0 | 158 | 192 | 263 | 303 | 303 | 269 |
| 1111 | 0 | 242 | 272 | 278 | 280 | 327 | 285 |
| 1114 | 0 | 249 | 218 | 279 | 250 | 226 | 170 |
| 1116 | 0 | 185 | 192 | 191 | 203 | 242 | 124 |
| 1118 | 0 | 349 | 387 | 429 | 429 | 458 | 209 |
| 2101 | 0 | 202 | 190 | 149 | 134 | 160 | 99.7 |
| 2104 | 0 | 136 | 113 | 132 | 117 | 126 | 135 |
| n | 11 | 11 | 11 | 11 | 11 | 11 | 11 |
| n<LLOQ | 11 | 0 | 0 | 0 | 0 | 0 | 0 |
| CV% | NC | 44.4 | 42.2 | 39.1 | 34.3 | 34.1 | 32.1 |
| AM | 0 | 197 | 216 | 236 | 248 | 258 | 185 |
| SD | 0 | 87.5 | 91.0 | 92.1 | 85.1 | 88.1 | 59.2 |
| Median | 0 | 202 | 211 | 263 | 250 | 247 | 170 |
| Minimum | 0 | 10.2 | 42.6 | 109 | 117 | 126 | 99.7 |
| Maximum | 0 | 349 | 387 | 429 | 429 | 458 | 285 |

| | | | | | | | |
|---|---|---|---|---|---|---|---|
| Lower limit of quantitation (LLOQ) = 5 (µg/L). N: Number of subjects, n: Number of non-missing observations, hr: Hour, BLQ: Below the limit of quantitation, AM Arithmetic mean, SD: Standard deviation. BLQ values are treated as zero. NC = Not calculated Values expressed in µg/L CV% = (SD/Mean) × 100, where SD and mean are standard deviation and arithmetic mean of untransformed data. Summary statistics for Day 1, 0 hr are based on greater than 30% imputed values. n<LLOQ = Number of BLQs imputed as zero. | | | | | | | |

| **Table 3B: Plasma Rivaroxaban Concentration-Time Data and Summary Statistics for Treatment Period III Separated by Renal Function Group (PK Analysis Set)** | | | | | | |
|---|---|---|---|---|---|---|
| Group 1: Mild Renal Impairment, (N=11) | | | | | | |
| Subject | Time Point | | | | | |
| | Day 1, | Day 1, | Day 2, | Day 2, | Day 3, | Day 4, |
| | 8 hr | 12 hr | 24 hr | 36 hr | 48 hr | 72 hr |
| 1101 | 125 | 77.7 | 36.0 | 11.1 | 8.48 | 0 |
| 1103 | 148 | 96.0 | 61.9 | 20.2 | 11.1 | 0 |
| 1104 | 194 | 88.6 | 45.5 | 25.0 | 12.9 | 8.27 |
| 1106 | 251 | 145 | 34.8 | 21.6 | 8.48 | 0 |
| 1108 | 222 | 154 | 78.5 | 33.0 | 27.4 | 10.6 |
| 1111 | 268 | 159 | 76.8 | 29.1 | 23.0 | 9.37 |
| 1114 | 126 | 55.4 | 27.6 | 14.2 | 17.0 | 8.69 |
| 1116 | 106 | 67.7 | 30.0 | 13.9 | 8.27 | 0 |
| 1118 | 179 | 134 | 68.5 | 34.9 | 31.2 | 13.1 |
| 2101 | 78.5 | 75.8 | 62.2 | 31.1 | 14.7 | 0 |
| 2104 | 118 | 95.0 | 46.6 | 27.0 | 19.1 | 7.48 |
| n | 11 | 11 | 11 | 11 | 11 | 11 |
| n<LLOQ | 0 | 0 | 0 | 0 | 0 | 5 |
| CV% | 37.8 | 35.4 | 36.2 | 34.5 | 47.9 | 99.6 |
| AM | 165 | 104 | 51.7 | 23.7 | 16.5 | 5.23 |
| SD | 62.3 | 37.0 | 18.7 | 8.19 | 7.91 | 5.21 |
| Median | 148 | 95.0 | 46.6 | 25.0 | 14.7 | 7.48 |
| Minimum | 78.5 | 55.4 | 27.6 | 11.1 | 8.27 | 0 |
| Maximum | 268 | 159 | 78.5 | 34.9 | 31.2 | 13.1 |

| | | | | | | |
|---|---|---|---|---|---|---|
| Lower limit of quantitation (LLOQ) = 5 (µg/L). N: Number of subjects, n: Number of non-missing observations, hr: Hour, BLQ: Below the limit of quantitation, AM Arithmetic mean, SD: Standard deviation. BLQ values are treated as zero. Values expressed in µg/L CV% = (SD/Mean) × 100, where SD and mean are standard deviation and arithmetic mean of untransformed data. Summary statistics for Day 4, 72 hr are based on greater than 30% imputed values. n<LLOQ = Number of BLQs imputed as zero. | | | | | | |

| **Table 4A: Plasma Rivaroxaban Concentration-Time Data and Summary Statistics for Treatment Period III Separated by Renal Function Group (PK Analysis Set)** | | | | | | | |
|---|---|---|---|---|---|---|---|
| Group 2: Normal Renal Function, (N=10) | | | | | | | |
| Subject | Time Point | | | | | | |
| | Day 1, | Day 1, | Day 1, | Day 1, | Day 1, | Day 1, | Day 1, |
| | 0 hr | 0.5 hr | 1 hr | 2 hr | 3 hr | 4 hr | 6 hr |
| 1105 | 0 | 179 | 185 | 208 | 267 | 383 | 268 |
| 1107 | 0 | 179 | 159 | 220 | 331 | 296 | 303 |
| 1109 | 0 | 198 | 236 | 302 | 264 | 292 | 205 |
| 1112 | 0 | 151 | 163 | 160 | 163 | 143 | 116 |
| 1119 | 0 | 148 | 206 | 314 | 314 | 348 | 287 |
| 1120 | 0 | 131 | 137 | 236 | 260 | 257 | 202 |
| 1121 | 0 | 178 | 201 | 208 | 178 | 154 | 103 |
| 2102 | 0 | 125 | 141 | 225 | 220 | 224 | 193 |
| 2103 | 0 | 214 | 269 | 232 | 225 | 201 | 177 |
| 2106 | 0 | 50.9 | 122 | 284 | 310 | 394 | 227 |
| n | 10 | 10 | 10 | 10 | 10 | 10 | 10 |
| n<LLOQ | 10 | 0 | 0 | 0 | 0 | 0 | 0 |
| CV% | NC | 29.9 | 25.7 | 19.9 | 22.4 | 33.2 | 32.0 |
| AM | 0 | 155 | 182 | 239 | 253 | 269 | 208 |
| SD | 0 | 46.4 | 46.7 | 47.6 | 56.8 | 89.5 | 66.6 |
| Median | 0 | 164 | 174 | 228 | 262 | 274 | 204 |
| Minimum | 0 | 50.9 | 122 | 160 | 163 | 143 | 103 |
| Maximum | 0 | 214 | 269 | 314 | 331 | 394 | 303 |

| | | | | | | | |
|---|---|---|---|---|---|---|---|
| Lower limit of quantitation (LLOQ) = 5 (µg/L). N: Number of subjects, n: Number of non-missing observations, hr: Hour, BLQ: Below the limit of quantitation, AM Arithmetic mean, SD: Standard deviation. BLQ values are treated as zero. NC = Not calculated Values expressed in µg/L CV% = (SD/Mean) × 100, where SD and mean are standard deviation and arithmetic mean of untransformed data. Summary statistics for Day 1, 0 hr are based on greater than 30% imputed values. n<LLOQ = Number of BLQs imputed as zero. | | | | | | | |

| **Table 4B: Plasma Rivaroxaban Concentration-Time Data and Summary Statistics for Treatment Period III Separated by Renal Function Group (PK Analysis Set)** | | | | | | |
|---|---|---|---|---|---|---|
| Group 2: Normal Renal Function, (N=10) | | | | | | |
| Subject | Time Point | | | | | |
| | Day 1, | Day 1, | Day 2, | Day 2, | Day 3, | Day 4, |
| | 8 hr | 12 hr | 24 hr | 36 hr | 48 hr | 72 hr |
| 1105 | 197 | 85.9 | 42.3 | 13.3 | 5.04 | 0 |
| 1107 | 182 | 121 | 41.3 | 11.7 | 6.36 | 0 |
| 1109 | 143 | 110 | 42.5 | 17.4 | 10.8 | 0 |
| 1112 | 94.9 | 78.7 | 50.9 | 12.3 | 8.87 | 0 |
| 1119 | 262 | 145 | 27.3 | 8.79 | 0 | 0 |
| 1120 | 119 | 62.8 | 27.4 | 8.99 | 0 | 0 |
| 1121 | 73.9 | 50.8 | 27.1 | 14.8 | 12.2 | 0 |
| 2102 | 160 | 102 | 48.4 | 19.5 | 10.7 | 0 |
| 2103 | 248 | 98.4 | 40.6 | 12.1 | 6.73 | 6.42 |
| 2106 | 173 | 125 | 60.2 | 22.8 | 13.0 | 9.22 |
| n | 10 | 10 | 10 | 10 | 10 | 10 |
| n<LLOQ | 0 | 0 | 0 | 0 | 2 | 8 |
| CV% | 36.9 | 29.7 | 27.0 | 31.9 | 63.3 | 215 |
| AM | 165 | 98.0 | 40.8 | 14.2 | 7.37 | 1.56 |
| SD | 61.0 | 29.1 | 11.0 | 4.52 | 4.66 | 3.36 |
| Median | 166 | 100 | 41.8 | 12.8 | 7.80 | 0 |
| Minimum | 73.9 | 50.8 | 27.1 | 8.79 | 0 | 0 |
| Maximum | 262 | 145 | 60.2 | 22.8 | 13.0 | 9.22 |

| | | | | | | |
|---|---|---|---|---|---|---|
| Lower limit of quantitation (LLOQ) = 5 (µg/L). N: Number of subjects, n: Number of non-missing observations, hr: Hour, BLQ: Below the limit of quantitation, AM Arithmetic mean, SD: Standard deviation. BLQ values are treated as zero. Values expressed in µg/L CV% = (SD/Mean) × 100, where SD and mean are standard deviation and arithmetic mean of untransformed data. Summary statistics for Day 4, 72 hr are based on greater than 30% imputed values. n<LLOQ = Number of BLQs imputed as zero. | | | | | | |

Plasma rivaroxaban pharmacokinetic data for both normal renal function groups and mild renal impairment groups who received both rivaroxaban alone (Period I) and rivaroxaban and verapamil (Period III) are presented in the Tables below:

| **Table 5: Plasma Rivaroxaban Noncompartmental Pharmacokinetic Parameters and Summary Statistics for Treatment Period I Separated by Renal Function Group (PK Analysis Set)** | | | | | | | | | |
|---|---|---|---|---|---|---|---|---|---|
| Group 1: Mild Renal Impairment, (N=14) | | | | | | | | | |
| Subject | Cmax (ng/mL) | tmax (hr) | AUC₀₋ₜ (hr*ng/mL) | AUC_{0-inf} (hr*ng/mL) | Kₑ₁ (1/hr) | t_{½} (hr) | CL/F (L/hr) | CL/F/kg ((L/hr)/kg) | V_{z}/F (L) |
| 01101 | 251 | 2.00 | 2530 | 2680 | 0.0542 | 12.8 | 7.45 | 0.113 | 138 |
| 01102 | 278 | 1.00 | 3240 | 3370 | 0.0734 | 9.44 | 5.93 | 0.0803 | 80.8 |
| 01103 | 196 | 3.00 | 2690 | 2800 | 0.0825 | 8.40 | 7.14 | 0.114 | 86.5 |
| 01104 | 235 | 0.50 | 2430 | 2510 | 0.0752 | 9.21 | 7.96 | 0.156 | 106 |
| 01106 | 238 | 3.00 | 2830 | 2960 | 0.0491 | 14.1 | 6.76 | 0.0845 | 138 |
| 01108 | 344 | 4.00 | 4350 | 4500 | 0.0778 | 8.91 | 4.45 | 0.0496 | 57.2 |
| 01110 | 306 | 1.00 | 3560 | 3840 | 0.0601 | 11.5 | 5.20 | 0.0670 | 86.5 |
| 01111 | 268 | 3.00 | 3250 | 3600 | 0.0447 | 15.5 | 5.55 | 0.0841 | 124 |
| 01114 | 308 | 1.00 | 2670 | 2760 | 0.0734 | 9.44 | 7.24 | 0.137 | 98.7 |
| 01115 | 215 | 3.00 | 2800 | 2920 | 0.0925 | 7.50 | 6.84 | 0.0946 | 74.0 |
| 01116 | 132 | 1.00 | 1070 | 1170 | 0.0954 | 7.26 | 17.0 | 0.253 | 179 |
| 01118 | 448 | 1.00 | 3880 | 4390 | 0.0240 | 28.9 | 4.56 | 0.0828 | 190 |
| 02101 | 217 | 1.00 | 2200 | 2290 | 0.0786 | 8.82 | 8.74 | NR | 111 |
| 02104 | 232 | 6.00 | 2290 | 2400 | 0.155 | 4.47 | 8.32 | NR | 53.7 |
| n | 14 | 14 | 14 | 14 | 14 | 14 | 14 | 12 | 14 |
| GM* | 252 | NC | 2720 | 2880 | 0.0683 | 10.2 | 6.95 | 0.100 | 102 |
| CVb% | 28.7 | NC | 28.3 | 29.1 | 41.1 | 52.5 | 41.9 | 49.2 | 38.0 |
| AM | 262 | NC | 2840 | 3010 | 0.0740 | 11.2 | 7.37 | 0.110 | 109 |
| SD | 75.2 | NC | 804 | 878 | 0.0304 | 5.87 | 3.09 | 0.0540 | 41.3 |
| Median | 244 | 1.50 | 2750 | 2860 | 0.0743 | 9.32 | 6.99 | 0.0896 | 102 |
| Minimum | 132 | 0.50 | 1070 | 1170 | 0.0240 | 4.47 | 4.45 | 0.0496 | 53.7 |
| Maximum | 448 | 6.00 | 4350 | 4500 | 0.155 | 28.9 | 17.0 | 0.253 | 190 |

| | | | | | | | | | |
|---|---|---|---|---|---|---|---|---|---|
| * Calculated using log transformed data. N: Number of subjects, n: Number of non-missing observations, GM: Geometric mean, AM: Arithmetic mean, SD: Standard deviation, NR = Not reported; NC = Not calculated %CVb = 100 * SQRT [ex[(S^2)-1]], where S is the standard deviation of the data on a log scale. | | | | | | | | | |

| **Table 6: Plasma Rivaroxaban Noncompartmental Pharmacokinetic Parameters and Summary Statistics for Treatment Period I Separated by Renal Function Group Excluding Outlier Sample (PK Analysis Set)** | | | | | | | | | |
|---|---|---|---|---|---|---|---|---|---|
| **Group** 2: Normal Renal Function, (N=13) | | | | | | | | | |
| Subject | Cmax (ng/mL) | tmax (hr) | AUC₀₋ₜ (hr*ng/mL) | AUC_{0-inf} (hr*ng/mL) | Kₑ₁ (1/hr) | t_{½} (hr) | CL/F (L/hr) | CL/F/kg ((L/hr)/kg) | V_{z}/F (L) |
| 01105 | 336 | 4.08 | 3370 | 3470 | 0.0955 | 7.26 | 5.76 | 0.0696 | 60.4 |
| 01107 | 297 | 4.00 | 2800 | 2850 | 0.110 | 6.29 | 7.02 | 0.0817 | 63.7 |
| 01109 | 307 | 3.00 | 2930 | 3150 | 0.108 | 6.39 | 6.35 | 0.0829 | 58.6 |
| 01112 | 195 | 2.00 | 2010 | 2070 | 0.0938 | 7.39 | 9.64 | 0.0960 | 103 |
| 01113 | 183 | 3.00 | 1700 | 1790 | 0.0839 | 8.26 | 11.2 | 0.197 | 133 |
| 01117 | 429 | 0.50 | 2670 | 3090 | 0.0336 | 20.6 | 6.46 | 0.0998 | 193 |
| 01119 | 352 | 4.10 | 3560 | 4440 | 0.106 | 6.56 | 4.50 | 0.0579 | 42.6 |
| 01120 | 214 | 1.00 | 2130 | 2200 | 0.0891 | 7.78 | 9.10 | 0.107 | 102 |
| 01121 | 218 | 0.50 | 1700 | 1840 | 0.0511 | 13.6 | 10.9 | 0.177 | 213 |
| 02102 | 212 | 2.00 | 2260 | 2310 | 0.108 | 6.44 | 8.68 | NR | 80.5 |
| 02103 | 230 | 3.00 | 2280 | 2400 | 0.0458 | 15.1 | 8.33 | NR | 182 |
| 02105 | 292 | 4.00 | 3200 | 3260 | 0.0939 | 7.38 | 6.14 | NR | 65.4 |
| 02106 | 266 | 4.00 | 3040 | 3150 | 0.0726 | 9.55 | 6.34 | NR | 87.4 |
| n | 13 | 13 | 13 | 13 | 13 | 13 | 13 | 9 | 13 |
| GM* | 263 | NC | 2520 | 2680 | 0.0793 | 8.74 | 7.47 | 0.0997 | 94.2 |
| CVb% | 26.7 | NC | 24.0 | 27.4 | 30.5 | 46.3 | 26.8 | 44.3 | 53.0 |
| AM | 272 | NC | 2590 | 2770 | 0.0839 | 9.43 | 7.72 | 0.108 | 106 |
| SD | 72.4 | NC | 621 | 760 | 0.0256 | 4.37 | 2.07 | 0.0476 | 56.4 |
| Median | 266 | 3.00 | 2670 | 2850 | 0.0938 | 7.39 | 7.02 | 0.0960 | 87.4 |
| Minimum | 183 | 0.50 | 1700 | 1790 | 0.0336 | 6.29 | 4.50 | 0.0579 | 42.6 |
| Maximum | 429 | 4.10 | 3560 | 4440 | 0.110 | 20.6 | 11.2 | 0.197 | 213 |

| | | | | | | | | | |
|---|---|---|---|---|---|---|---|---|---|
| * Calculated using log transformed data. N: Number of subjects, n: Number of non-missing observations, GM: Geometric mean, AM: Arithmetic mean, SD: Standard deviation, NR = Not reported; NC = Not calculated %CVb = 100 * SQRT [ex[(S^2)-1]], where S is the standard deviation of the data on a log scale. The Period 1, 48 hr timepoint for subject 01119 was excluded as an outlier from the analysis. | | | | | | | | | |

| **Table 7: Plasma Rivaroxaban Noncompartmental Pharmacokinetic Parameters and Summary Statistics for Treatment Period III Separated by Renal Function Group (PK Analysis Set)** | | | | | | | | | |
|---|---|---|---|---|---|---|---|---|---|
| Group 1: Mild Renal Impairment, (N=11) | | | | | | | | | |
| Subject | Cmax (ng/mL) | tmax (hr) | AUC₀₋ₜ (hr*ng/mL) | AUC_{0-inf} (hr*ng/mL) | Kₑ₁ (1/hr) | t_{½} (hr) | CL/F (L/hr) | CL/F/kg ((L/hr)/kg) | V_{z}/F (L) |
| 01101 | 247 | 4.00 | 2950 | 3070 | 0.0721 | 9.61 | 6.51 | 0.0991 | 90.3 |
| 01103 | 300 | 2.00 | 3920 | 4090 | 0.0664 | 10.4 | 4.89 | 0.0781 | 73.6 |
| 01104 | 278 | 0.50 | 3990 | 4140 | 0.0542 | 12.8 | 4.83 | 0.0949 | 89.1 |
| 01106 | 280 | 4.00 | 3870 | 3980 | 0.0820 | 8.46 | 5.03 | 0.0628 | 61.3 |
| 01108 | 303 | 3.00 | 5630 | 5840 | 0.0503 | 13.8 | 3.42 | 0.0382 | 68.1 |
| 01111 | 327 | 4.00 | 5810 | 6090 | 0.0323 | 21.4 | 3.28 | 0.0497 | 102 |
| 01114 | 279 | 2.00 | 3230 | 3530 | 0.0283 | 24.5 | 5.66 | 0.107 | 200 |
| 01116 | 242 | 4.00 | 2680 | 2820 | 0.0590 | 11.7 | 7.08 | 0.105 | 120 |
| 01118 | 458 | 4.00 | 5990 | 6300 | 0.0418 | 16.6 | 3.17 | 0.0577 | 76.0 |
| 02101 | 202 | 0.50 | 3010 | 3260 | 0.0598 | 11.6 | 6.14 | NR | 103 |
| 02104 | 136 | 0.50 | 3280 | 3480 | 0.0374 | 18.5 | 5.74 | NR | 154 |
| n | 11 | 11 | 11 | 11 | 11 | 11 | 11 | 9 | 11 |
| GM* | 267 | NC | 3880 | 4080 | 0.0504 | 13.7 | 4.90 | 0.0728 | 97.1 |
| CVb% | 28.9 | NC | 30.2 | 29.6 | 32.0 | 35.6 | 26.2 | 33.6 | 40.2 |
| AM | 277 | NC | 4030 | 4240 | 0.0530 | 14.5 | 5.07 | 0.0770 | 103 |
| SD | 80.1 | NC | 1220 | 1260 | 0.0170 | 5.16 | 1.33 | 0.0259 | 41.5 |
| Median | 279 | 3.00 | 3870 | 3980 | 0.0542 | 12.8 | 5.03 | 0.0781 | 90.3 |
| Minimum | 136 | 0.50 | 2680 | 2820 | 0.0283 | 8.46 | 3.17 | 0.0382 | 61.3 |
| Maximum | 458 | 4.00 | 5990 | 6300 | 0.0820 | 24.5 | 7.08 | 0.107 | 200 |

| | | | | | | | | | |
|---|---|---|---|---|---|---|---|---|---|
| * Calculated using log transformed data. N: Number of subjects, n: Number of non-missing observations, GM: Geometric mean, AM: Arithmetic mean, SD: Standard deviation. NR = Not reported; NC = Not calculated %CVb = 100 * SQRT [ex[(S^2)-1]], where S is the standard deviation of the data on a log scale. | | | | | | | | | |

| **Table 8: Plasma Rivaroxaban Noncompartmental Pharmacokinetic Parameters and Summary Statistics for Treatment Period III Separated by Renal Function Group (PK Analysis Set)** | | | | | | | | | |
|---|---|---|---|---|---|---|---|---|---|
| Group 2: Normal Renal Function, (N=10) | | | | | | | | | |
| Subject | Cmax (ng/mL) | tmax (hr) | AUC₀₋ₜ (hr*ng/mL) | AUC_{0-inf} (hr*ng/mL) | Kₑ₁ (1/hr) | t_{½} (hr) | CL/F (L/hr) | CL/F/kg ((L/hr)/kg) | V_{z}/F (L) |
| 01105 | 383 | 4.00 | 3780 | 3840 | 0.0884 | 7.84 | 5.21 | 0.0629 | 58.9 |
| 01107 | 331 | 3.00 | 3990 | 4060 | 0.0866 | 8.00 | 4.92 | 0.0573 | 56.9 |
| 01109 | 302 | 2.00 | 3770 | 3930 | 0.0672 | 10.3 | 5.08 | 0.0664 | 75.7 |
| 01112 | 163 | 1.00 | 2690 | 2820 | 0.0656 | 10.6 | 7.08 | 0.0705 | 108 |
| 01119 | 348 | 4.00 | 4260 | 4330 | 0.121 | 5.71 | 4.61 | 0.0594 | 38.0 |
| 01120 | 260 | 3.00 | 2690 | 2800 | 0.0810 | 8.55 | 7.14 | 0.0839 | 88.1 |
| 01121 | 208 | 2.00 | 2260 | 2530 | 0.0456 | 15.2 | 7.90 | 0.128 | 173 |
| 02102 | 225 | 2.00 | 3500 | 3670 | 0.0643 | 10.8 | 5.46 | NR | 84.9 |
| 02103 | 269 | 1.00 | 3780 | 3880 | 0.0614 | 11.3 | 5.15 | NR | 83.9 |
| 02106 | 394 | 4.00 | 4620 | 4800 | 0.0511 | 13.6 | 4.17 | NR | 81.6 |
| n | 10 | 10 | 10 | 10 | 10 | 10 | 10 | 7 | 10 |
| GM* | 278 | NC | 3460 | 3600 | 0.0705 | 9.83 | 5.56 | 0.0727 | 78.8 |
| CVb% | 26.6 | NC | 21.4 | 19.9 | 30.0 | 27.6 | 21.9 | 33.0 | 43.2 |
| AM | 288 | NC | 3530 | 3670 | 0.0732 | 10.2 | 5.67 | 0.0755 | 84.9 |
| SD | 76.8 | NC | 755 | 728 | 0.0220 | 2.82 | 1.24 | 0.0249 | 36.7 |
| Median | 286 | 2.50 | 3780 | 3860 | 0.0664 | 10.4 | 5.18 | 0.0664 | 82.7 |
| Minimum | 163 | 1.00 | 2260 | 2530 | 0.0456 | 5.71 | 4.17 | 0.0573 | 38.0 |
| Maximum | 394 | 4.00 | 4620 | 4800 | 0.121 | 15.2 | 7.90 | 0.128 | 173 |

| | | | | | | | | | |
|---|---|---|---|---|---|---|---|---|---|
| * Calculated using log transformed data. N: Number of subjects, n: Number of non-missing observations, GM: Geometric mean, AM: Arithmetic mean, SD: Standard deviation. NR = Not reported; NC = Not calculated %CVb = 100 * SQRT [ex[(S^2)-1]], where S is the standard deviation of the data on a log scale. | | | | | | | | | |

### Example 3 - Plasma Rivaroxaban Concentrations and the Risk of Major Bleeding in Subjects with Mild Renal Impairment

To evaluate the relationship between the plasma levels of rivaroxaban and the risk of major bleeding, the subjects with mild renal insufficiency were given a single 20 mg dose of rivaroxaban on the first day of the study (as described in the study in Example 2). Blood samples were collected and processed according to the standard protocols. Plasma rivaroxaban concentrations were measured on Day 1 and Day 15. As shown in **FIGs. 1** and **2**, the risk of major bleeding based on PT values, were positively correlated with increased plasma concentrations of rivaroxaban. The results showed a clear linear relationship between the pharmacokinetic and the pharmacodynamics of rivaroxaban in subjects with normal renal function and mild renal insufficiency.

The steady state area under the curve (AUC) of plasma rivaroxaban in subjects with either normal renal function or mild renal impairment under different treatment regimens was also examined. As shown in **FIG. 3**, the leftmost 6 box plots (study 10842) show the distribution of AUC values for various dosing levels of rivaroxaban (10 mg, 20 mg, 30 mg, 40 mg, 60 mg, and 80 mg, respectively) previously measured in clinical trials supporting FDA approval of Xarelto® (rivaroxaban). The next nine box plots (studies 10993, 10999, 12359, 12680, 11273, 10989, and 11938) in the middle represent subjects treated with a single 20 mg dose of rivaroxaban under normal, fasted, or fed condition. All show the geometric mean plus or minus one standard deviation. The last four box plots show the distribution of AUC values measured in the present study, for subjects with normal renal function (designated "N") and subjects with mild renal impairment (designated "MRI") who were either treated with a single 20 mg dose of rivaroxaban ("P1") or a single 20 mg dose of rivaroxaban and 360 mg verapamil ("P3"). These four show the geometric mean and the full minimum and maximum subject value range.

The results in **FIG. 3** show that the geometric mean in the subjects with mild renal impairment treated with both rivaroxaban and verapamil in the present study was about one standard deviation higher than the mean AUC levels in subjects with normal renal function treated with a single 80 mg dose of rivaroxaban. The present study shows that subjects with mild renal impairment who took both rivaroxaban and verapamil had mean AUC levels well above the AUC levels observed in subjects with normal renal function treated with a single 20 mg dose of rivaroxaban; that is, such patients coadministered rivaroxaban and verapamil have AUC values substantially higher than values currently considered acceptable by the FDA.

Another representation of these data are shown in **FIG. 4****.** Label "A" represents the upper bound of the 90% confidence interval of the steady state AUC of patients administered 20 mg of rivaroxaban, about 3,792 µg•hr/L. Label "B" is the estimated steady state AUC, about 3,404 µg•hr/L for patients administered a single 10 mg dose of rivaroxaban in combination with a strong CYP3A4/Pgp inhibitor. Label "C" is the upper boundary of the 90% confidence interval of the steady state AUC level in subjects treated with a single 20 mg dose of rivaroxaban after a meal, about 2,448µg•hr/L. The remaining 4 box plots are reproduced from FIG. 3. FIG. 4 shows that AUC_{inf} values in subjects with mild renal impairment ("MRI") treated with a single 20 mg dose of rivaroxaban and 360 mg verapamil (P3) are significantly higher than expected from previous studies. For example, the geometric mean of the steady state AUC for such patients was higher than the upper limits of safety identified in the FDA's Clinical Pharmacology review for rivaroxaban, demonstrating that a significant portion of the relevant patient population who have mild renal impairment have AUC values falling outside of the range accepted by the FDA. Further, the present study shows that a significant portion of patients with normal renal function treated with rivaroxaban and verapamil have plasma levels significantly higher than the upper boundary of the 90% confidence interval of the steady state AUC level in subjects from the rivaroxaban approval studies treated with a single 20 mg dose of rivaroxaban (Label "C"), demonstrating a higher bleeding risk than is presently recognized in the art. Additionally, the present study shows that patients with mild renal impairment have plasma levels of rivaroxaban which are significantly higher than the upper boundary of the 90% confidence interval of the steady state AUC level in subjects from the rivaroxaban approval studies treated with a single 20 mg dose of rivaroxaban (Label "C"), demonstrating a higher bleeding risk than is presently recognized in the art.

**FIG. 5** shows that the relationship between steady state rivaroxaban AUC and the risk of major bleeding. Group "a" in FIG. 5 represents the average geometric mean of fed subjects across two studies (10989 and 11938) who took a single 20 mg rivaroxaban and had an average of AUC of approximately 2,026 µg•hr/L, which corresponded to about 3% risk of major bleeding. Group "b" in the FIG. 5 represents subjects with mild renal impairment who took a single 20 mg rivaroxaban and 360 mg verapamil and had an estimated of AUC_{inf} of approximately 5,469 µg•hr/L, which shows that the mild renal impairment caused higher accumulation of rivaroxaban in the plasma and led to 2.5 times higher the risk of major bleeding compared to the populations in Group "a". Group "c" represents the average of the three subjects in Group "b" who had the highest AUC_{inf}, which was about 8,143 µg•hr/L. The risk of major bleeding of the subjects in Group "c" were at least 5 times higher compared to the populations in Group "a" and not even visible on this scale.

### Example 4 - Mathematical Models for Determining the Redosing Regimens of Rivaroxaban to Reduce the Risk of Major Bleeding in Subjects with Mild Renal Impairment

To reduce the risks of major bleeding for the various populations that are among the high risk groups identified above, Applicant created an exponential mathematical model and a logarithmic mathematical model to calculate the appropriate rivaroxaban redosing regimens for these populations to achieve the AUC and Cₘₐₓ that are within the safety recommendations.

The present study has identified various target AUC values representing different potential AUC levels above which there could be a serious risk of bleeding in patients treated with rivaroxaban as described herein. Specifically, referring to FIG. 4, AUC values above about 3,792 µg•hr/L (the upper bound of the 90% confidence interval of the steady state AUC of patients administered 20 mg of rivaroxaban, Label "A"), above about 3,404 µg•hr/L (estimated steady state AUC for patients administered a single 10 mg dose of rivaroxaban in combination with a strong CYP3A4/Pgp inhibitor, Label "B"), or about 2,448 µg•hr/L (the upper boundary of the 90% confidence interval of the steady state AUC level in subjects with normal renal function treated with a single 20 mg dose of rivaroxaban after a meal, Label "C") are all potential AUC values above which there would be an unacceptable level of bleeding risk.

It is well known that the pharmacokinetics characteristics of drugs vary among individuals. Thus, PK parameters such as AUC_{inf} and Cₘₐₓ within a patient population or subgroup are characterized statistically, using parameters such as the maximum and minimum values, mean (arithmetic and geometric), median, average of the highest 3 levels in the patient population, and the 90% confidence interval (having an upper and lower boundary or limit). When assessing risks associated with drug treatments (e.g., bleeding risk associated with rivaroxaban treatment), it may be desirable to ensure that the entire patient population has AUC (or Cₘₐₓ) values falling below the level identified as unacceptably risky (e.g., termed the "target" value. In this case, the drug should be dosed such that the maximum AUC or Cₘₐₓ value in the population or subgroup falls at or below the target value. In other estimates of risk, the drug should be dosed such that other statistical parameters characterizing the appropriate PK values of the patient population (mean, median, 90% confidence interval boundaries) fall at or below the target value. As shown below in Tables 9-124, the present applicant has calculated, based on various models, the maximum rivaroxaban dose for various patients (e.g., having varying levels of renal impairment and/or concomitant treatment with verapamil) which will provide AUC_{inf} or Cₘₐₓ values no higher than the "target" AUC_{inf} or Cₘₐₓ value, for the respective statistical parameter characterizing the PK distribution of the patient population. That is, for a particular patient population and target value (AUC or Cmax), applicant has calculated the maximum rivaroxaban dose that will provide no more than that target value for the maximum, minimum, mean (arithmetic or geometric), median, average of the 3 highest values, or upper or lower boundary of the 90% confidence interval of the PK parameter (AUC_{inf} or Cₘₐₓ) in the patient population.

An exponential model was used to estimate the rivaroxaban redosing regimen. A formula was fitted to a large interval of rivaroxaban dosing data, encompassing doses from 5mg to 80mg, a 16-fold range. The resulting formula was then indexed to the actual AUC observed in the study and used to calculate an adjusted dose recommendation. In Tables 9-11 below, three targets of the exponential redosing AUC_{inf} (area under the curve extrapolated to infinity) were identified using AUC values corresponding to "A", "B", and "C" in FIG. 4 as discussed above, for subjects with mild renal impairment, and who are administered both rivaroxaban and verapamil (Table 9) or for patients with mild renal impairment treated only with rivaroxaban (Table 10). Table 11 shows corresponding re-dosing for patients with no renal impairment ("normal") administered both rivaroxaban and verapamil.

The redosing shown below represents individual aspects of the methods of the present disclosure. The value in the respective Dose 1, Dose 2, and Dose 3 columns is the maximum rivaroxaban dose that should be administered to a patient falling within the patient class defined in the header of the table, in order to provide a PK value (i.e., AUC or Cₘₐₓ) that does not exceed the corresponding Target 1, Target 2, or Target 3 PK parameter for the respective statistical parameter. By way of example, the calculated value of 8.7 mg rivaroxaban for the "maximum" Group, Dose 1 of Table 9 represents the maximum dose of rivaroxaban that should be administered to a patient with mild renal impairment, also treated with verapamil, to ensure that the highest (i.e., maximum) AUC_{inf} value obtained in that population would be no more than 3,792 µg•hr/L. Similarly, for treatment of a patient with mild renal impairment taking only rivaroxaban, to ensure that in a population of such patients, the AUC_{inf} of the upper boundary of the 90% confidence interval would not exceed 2,448 µg•hr/L, a dose of about 12.2 mg or rivaroxaban would be the highest dose that should be administered (Table 10, Group "90% CI, Upper Boundary", Dose 3). Where the rivaroxaban dose is not listed in the table, the recommended dose of rivaroxaban (as defined by the Xarelto package insert revised 08/2016) can be used.

Thus, in various aspects, the present disclosure is directed to methods of treating a patient with a condition for which a Factor Xa inhibitor is indicated (selected from the group consisting of reducing the risk of stroke and systemic embolism in patients with non valvular atrial fibrillation, treating deep vein thrombosis (DVT), treating pulmonary embolism (PE), reducing the risk of DVT, and reducing the risk of PE, the prophylaxis of DVT which leads to PE in patients undergoing knee or hip replacement therapy), and a condition for which a calcium channel blocker is indicated (such as the management of essential hypertension), or any other condition disclosed herein, wherein said patient is administered verapamil and an amount of rivaroxaban such that the maximum, mean of the top 3, upper boundary of the 90% CI, median, arithmetic mean, geometric mean, lower boundary of the 90% CI, or minimum AUC or Cₘₐₓ is no more than about the Dose 1, Dose 2, or Dose 3 values (in mg) found in the respective portions of Tables 9, 11, 12, 14-16, 21, and 22. In various such aspects, the patient has mild renal impairment. In other aspects, the patient has normal renal function.

In various other aspects, the present disclosure is directed to methods of treating a patient with a condition for which a Factor Xa inhibitor is indicated (selected from the group consisting of reducing the risk of stroke and systemic embolism in patients with non valvular atrial fibrillation, treating deep vein thrombosis (DVT), treating pulmonary embolism (PE), reducing the risk of DVT, and reducing the risk of PE, the prophylaxis of DVT which leads to PE in patients undergoing knee or hip replacement therapy), or any other condition disclosed herein, wherein said patient is administered an amount of rivaroxaban such that the maximum, mean of the top 3, upper boundary of the 90% CI, median, arithmetic mean, geometric mean, lower boundary of the 90% CI, or minimum AUC or Cₘₐₓ is no more than about the Dose 1, Dose 2, or Dose 3 values (in mg) found in the respective portions of Tables 10, 13, 17-19, and 23-26. In various such aspects, the patient has mild renal impairment.

**Table 9: Redosing regimens of rivaroxaban in response to various target AUC values in subjects with mild renal impairment treated with both rivaroxaban and verapamil using the exponential model.**

| **Mild Renal Impairment - R+V** | | | | | | | |
|---|---|---|---|---|---|---|---|
| | | | | | | | |
| **Exponential Redosing AUC** | | | | | | | |
| | | | | | | | |
| **Group** | **Target 1** | **Target 2** | **Target 3** | | **Dose 1** | **Dose 2** | **Dose 3** |
| **Maximum** | 3,792 | 3,404 | 2,448 | | 8.7 | 7.2 | 4.1 |
| **Mean of Top 3** | 3,792 | 3,404 | 2,448 | | 9.3 | 7.7 | 4.4 |
| **90% CI, Upper Boundary** | 3,792 | 3,404 | 2,448 | | 14.1 | 11.7 | 6.6 |
| **Median** | 3,792 | 3,404 | 2,448 | | 19.3 | 16.0 | 9.1 |
| **Arithmetic Mean** | 3,792 | 3,404 | 2,448 | | 17.3 | 14.4 | 8.1 |
| **Geometric Mean** | 3,792 | 3,404 | 2,448 | | 18.5 | 15.3 | 8.7 |
| **90% CI, Lower Boundary** | 3,792 | 3,404 | 2,448 | | | 19.9 | 11.2 |
| **Minimum** | 3,792 | 3,404 | 2,448 | | | | 16.4 |

**Table 10: Redosing regimens of rivaroxaban in response to various target AUC values in subjects with mild renal impairment treated with a single dose of rivaroxaban using the exponential model.**

| **Mild Renal Impairment - R only** | | | | | | | |
|---|---|---|---|---|---|---|---|
| | | | | | | | |
| **Exponential Redosing AUC** | | | | | | | |
| | | | | | | | |
| **Group** | **Target 1** | **Target 2** | **Target 3** | | **Dose 1** | **Dose 2** | **Dose 3** |
| **Maximum** | 3,792 | 3,404 | 2,448 | | 15.6 | 12.9 | 7.3 |
| **Mean of Top 3** | 3,792 | 3,404 | 2,448 | | 17.8 | 14.8 | 8.4 |
| **90% CI, Upper Boundary** | 3,792 | 3,404 | 2,448 | | | | 12.2 |
| **Median** | 3,792 | 3,404 | 2,448 | | | | 17.0 |
| **Arithmetic Mean** | 3,792 | 3,404 | 2,448 | | | | 15.5 |
| **Geometric Mean** | 3,792 | 3,404 | 2,448 | | | | 17.1 |
| **90% CI, Lower Boundary** | 3,792 | 3,404 | 2,448 | | | | |
| **Minimum** | 3,792 | 3,404 | 2,448 | | | | |

**Table 11: Redosing regimens of rivaroxaban in response to various target AUC values in subjects with no renal impairment treated with both rivaroxaban and verapamil using the exponential model.**

| **No Renal Impairment - R + V** | | | | | | | |
|---|---|---|---|---|---|---|---|
| | | | | | | | |
| **Exponential Redosing AUC** | | | | | | | |
| | | | | | | | |
| **Group** | **Target 1** | **Target 2** | **Target 3** | | **Dose 1** | **Dose 2** | **Dose 3** |
| **Maximum** | 3,792 | 3,404 | 2,448 | | 14.0 | 11.6 | 6.6 |
| **Mean of Top 3** | 3,792 | 3,404 | 2,448 | | 17.1 | 14.2 | 8.0 |
| **90% CI, Upper Boundary** | 3,792 | 3,404 | 2,448 | | 18.6 | 15.4 | 8.7 |
| **Median** | 3,792 | 3,404 | 2,448 | | | 16.9 | 9.5 |
| **Arithmetic Mean** | 3,792 | 3,404 | 2,448 | | | 18.4 | 10.4 |
| **Geometric Mean** | 3,792 | 3,404 | 2,448 | | | 19.1 | 10.8 |
| **90% CI, Lower Boundary** | 3,792 | 3,404 | 2,448 | | | | 13.3 |
| **Minimum** | 3,792 | 3,404 | 2,448 | | | | 19.8 |

A logarithmic model was also developed and used to estimate rivaroxaban redosing using the same observed-16 fold range. This was also indexed to the observed study data and used to calculate adjusted dose recommendations. In Tables [d]-[f] below, three targets of redosing based on AUC_{inf} were identified using AUC values corresponding to "A", "B", and "C" in FIG. 4 as discussed above, for subjects with mild renal impairment, and who are administered both rivaroxaban and verapamil (Table [d]) or for patients with mild renal impairment treated only with rivaroxaban (Table [e]). Table [f] shows corresponding redosing for patients with no renal impairment ("normal") administered both rivaroxaban and verapamil.

**Table 12: Redosing regimens of rivaroxaban in response to various target AUCs in subjects with mild renal impairment treated with both rivaroxaban and verapamil using the logarithmic model.**

| **Mild Renal Impairment - R + V** | | | | | | | |
|---|---|---|---|---|---|---|---|
| | | | | | | | |
| **Logarithmic Redosing AUC** | | | | | | | |
| | | | | | | | |
| **Group** | **Target 1** | **Target 2** | **Target 3** | | **Dose 1** | **Dose 2** | **Dose 3** |
| **Maximum** | 3,792 | 3,404 | 2,448 | | 9.5 | 8.7 | 6.9 |
| **Mean of Top 3** | 3,792 | 3,404 | 2,448 | | 9.9 | 9.0 | 7.1 |
| **90% CI, Upper** | | | | | | | |
| **Boundary** | 3,792 | 3,404 | 2,448 | | 12.8 | 11.3 | 8.3 |
| **Median** | 3,792 | 3,404 | 2,448 | | 16.3 | 14.0 | 9.7 |
| **Arithmetic Mean** | 3,792 | 3,404 | 2,448 | | 14.9 | 13.0 | 9.2 |
| **Geometric Mean** | 3,792 | 3,404 | 2,448 | | 15.7 | 13.6 | 9.5 |
| **90% CI, Lower** | | | | | | | |
| **Boundary** | 3,792 | 3,404 | 2,448 | | 19.7 | 16.7 | 11.0 |
| **Minimum** | 3,792 | 3,404 | 2,448 | | | | 14.3 |

**Table 13: Redosing regimens of rivaroxaban in response to various target AUCs in subjects with mild renal impairment treated with a single dose of rivaroxaban using the logarithmic model.**

| **Mild Renal Impairment - R only** | | | | | | | |
|---|---|---|---|---|---|---|---|
| | | | | | | | |

| **Logarithmic Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 13.8 | 12.1 | 8.7 |
| **Mean of Top 3** | 3,792 | 3,404 | 2,448 | | 15.3 | 13.2 | 9.3 |
| **90% CI, Upper Boundary** | 3,792 | 3,404 | 2,448 | | | 17.9 | 11.6 |
| **Median** | 3,792 | 3,404 | 2,448 | | | | 14.7 |
| **Arithmetic Mean** | 3,792 | 3,404 | 2,448 | | | | 13.7 |
| **Geometric Mean** | 3,792 | 3,404 | 2,448 | | | | 14.7 |
| **90% CI, Lower Boundary** | 3,792 | 3,404 | 2,448 | | | | 17.5 |
| **Minimum** | 3,792 | 3,404 | 2,448 | | | | |

**Table 14: Redosing regimens of rivaroxaban in response to various target AUCs in subjects with mild renal impairment treated with both rivaroxaban and verapamil using the logarithmic model.**

| **No Renal Impairment - R + V** | | | | | | | |
|---|---|---|---|---|---|---|---|
| | | | | | | | |

| **Logarithmic Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 12.7 | 11.2 | 8.3 |
| **Mean of Top 3** | 3,792 | 3,404 | 2,448 | | 14.8 | 12.9 | 9.2 |
| **90% CI, Upper Boundary** | 3,792 | 3,404 | 2,448 | | 15.8 | 13.6 | 9.5 |
| **Median** | 3,792 | 3,404 | 2,448 | | 17.0 | 14.6 | 10.0 |
| **Arithmetic Mean** | 3,792 | 3,404 | 2,448 | | 18.4 | 15.7 | 10.5 |
| **Geometric Mean** | 3,792 | 3,404 | 2,448 | | 19.0 | 16.1 | 10.8 |
| **90% CI, Lower Boundary** | 3,792 | 3,404 | 2,448 | | | 19.4 | 12.3 |
| **Minimum** | 3,792 | 3,404 | 2,448 | | | | 16.6 |

Analogously to target AUC_{inf} values, three target Cₘₐₓ values were also used to calculate rivaroxaban redosing regimens (275, 268, and 317 ng/mL), respectively derived from the upper bound of the 90% confidence interval of the steady state Cₘₐₓ of patients administered 20 mg of rivaroxaban, the Cₘₐₓ for patients administered a single 10 mg dose of rivaroxaban in combination with a strong CYP3A4/Pgp inhibitor, and the upper boundary of the 90% confidence interval of the Cₘₐₓ level of subjects administered 20 mg rivaroxaban with food in study [11938] and applied to subjects with mild renal impairment treated with a single 20 mg dose of rivaroxaban alone, a single 20 mg dose of rivaroxaban and 360 mg verapamil, and for subjects with normal renal function on a single 20 mg dose of rivaroxaban and 360 mg verapamil. These redosing estimates are shown in Tables [e]-[1], below.

The fitted exponential Cₘₐₓ model indexed rivaroxaban redosing estimates are provided in Tables [e]-[h].

**Table 15: Redosing regimens of rivaroxaban in response to various target single dose Cₘₐₓ in subjects with mild renal impairment treated with both rivaroxaban and verapamil using the exponential model.**

| **Mild Renal Impairment - R+V** | | | | | | | |
|---|---|---|---|---|---|---|---|
| | | | | | | | |

| **Exponential Redosing Single Dose Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 5.9 | 5.6 | 8.0 |
| **Mean of Top 3** | 275 | 268 | 317 | | 9.8 | 9.3 | 13.4 |
| **90% CI, Upper Boundary** | 275 | 268 | 317 | | 13.2 | 12.6 | 18.1 |
| **Median** | 275 | 268 | 317 | | 17.2 | 16.3 | |
| **Arithmetic Mean** | 275 | 268 | 317 | | 17.4 | 16.5 | |
| **Geometric Mean** | 275 | 268 | 317 | | 18.9 | 18.0 | |
| **90% CI, Lower Boundary** | 275 | 268 | 317 | | | | |
| **Minimum** | 275 | 268 | 317 | | | | |

**Table 16: Redosing regimens of rivaroxaban in response to various target steady state Cₘₐₓ in subjects with mild renal impairment treated with both rivaroxaban and Verapamil using the exponential model.**

| **Mild Renal Impairment - R+V** | | | | | | | |
|---|---|---|---|---|---|---|---|
| | | | | | | | |

| **Exponential Redosing Steady State Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 3.4 | 3.2 | 4.6 |
| **Mean of Top 3** | 275 | 268 | 317 | | 5.6 | 5.4 | 7.7 |
| **90% CI, Upper Boundary** | 275 | 268 | 317 | | 7.6 | 7.2 | 10.4 |
| **Median** | 275 | 268 | 317 | | 9.9 | 9.4 | 13.5 |
| **Arithmetic Mean** | 275 | 268 | 317 | | 10.0 | 9.5 | 13.7 |
| **Geometric Mean** | 275 | 268 | 317 | | 10.9 | 10.4 | 14.9 |
| **90% CI, Lower Boundary** | 275 | 268 | 317 | | 15.6 | 14.8 | |
| **Minimum** | 275 | 268 | 317 | | | | |

**Table 17: Redosing regimens of rivaroxaban in response to various target single dose Cₘₐₓ in subjects with mild renal impairment treated with a single dose of rivaroxaban using the exponential model.**

| **Mild Renal Impairment - R Only** | | | | | | | |
|---|---|---|---|---|---|---|---|
| | | | | | | | |

| **Exponential Redosing Single Dose Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 6.2 | 5.9 | 8.4 |
| **Mean of Top 3** | 275 | 268 | 317 | | 9.5 | 9.0 | 13.0 |
| **90% CI, Upper Boundary** | 275 | 268 | 317 | | 15.9 | 15.1 | |
| **Median** | 275 | 268 | 317 | | | | |
| **Arithmetic Mean** | 275 | 268 | 317 | | 19.9 | 18.9 | |
| **Geometric Mean** | 275 | 268 | 317 | | | | |
| **90% CI, Lower Boundary** | 275 | 268 | 317 | | | | |
| **Minimum** | 275 | 268 | 317 | | | | |

**Table 18: Redosing regimens of rivaroxaban in response to various target steady state Cₘₐₓ in subjects with mild renal impairment treated with a single dose of rivaroxaban using the exponential model.**

| **Mild Renal Impairment - R Only** | | | | | | | |
|---|---|---|---|---|---|---|---|
| | | | | | | | |

| **Exponential Redosing Steady State Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 4.0 | 3.8 | 5.5 |
| **Mean of Top 3** | 275 | 268 | 317 | | 6.2 | 5.9 | 8.4 |
| **90% CI, Upper Boundary** | 275 | 268 | 317 | | 10.4 | 9.8 | 14.1 |
| **Median** | 275 | 268 | 317 | | 15.8 | 15.0 | |
| **Arithmetic Mean** | 275 | 268 | 317 | | 12.9 | 12.3 | 17.6 |
| **Geometric Mean** | 275 | 268 | 317 | | 14.2 | 13.5 | 19.4 |
| **90% CI, Lower Boundary** | 275 | 268 | 317 | | 18.6 | 17.7 | |
| **Minimum** | 275 | 268 | 317 | | | | |

**Table 19: Redosing regimens of rivaroxaban in response to various target single dose Cₘₐₓ in subjects with no renal impairment treated with both rivaroxaban and verapamil using the exponential model.**

| **No Renal Impairment - R Only** | | | | | | | |
|---|---|---|---|---|---|---|---|
| | | | | | | | |

| **Exponential Redosing Single Dose Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 8.1 | 7.7 | 11.1 |
| **Mean of Top 3** | 275 | 268 | 317 | | 10.3 | 9.7 | 14.0 |
| **90% CI, Upper Boundary** | 275 | 268 | 317 | | 12.1 | 11.5 | 16.5 |
| **Median** | 275 | 268 | 317 | | 16.3 | 15.5 | |
| **Arithmetic Mean** | 275 | 268 | 317 | | 16.0 | 15.2 | |
| **Geometric Mean** | 275 | 268 | 317 | | 17.3 | 16.4 | |
| **90% CI, Lower Boundary** | 275 | 268 | 317 | | | | |
| **Minimum** | 275 | 268 | 317 | | | | |

**Table 20: Redosing regimens of rivaroxaban in response to various target steady state Cₘₐₓ in subjects with no renal impairment treated with both rivaroxaban and verapamil using the exponential model.**

| **No Renal Impairment - R Only** | | | | | | | |
|---|---|---|---|---|---|---|---|
| | | | | | | | |

| **Exponential Redosing Steady State Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 5.9 | 5.6 | 8.1 |
| **Mean of Top 3** | 275 | 268 | 317 | | 7.4 | 7.1 | 10.2 |
| **90% CI, Upper Boundary** | 275 | 268 | 317 | | 8.8 | 8.3 | 12.0 |
| **Median** | 275 | 268 | 317 | | 11.9 | 11.3 | 16.2 |
| **Arithmetic Mean** | 275 | 268 | 317 | | 11.6 | 11.0 | 15.8 |
| **Geometric Mean** | 275 | 268 | 317 | | 12.5 | 11.9 | 17.1 |
| **90% CI, Lower Boundary** | 275 | 268 | 317 | | 17.9 | 17.0 | |
| **Minimum** | 275 | 268 | 317 | | | | |

The fitted logarithmic model rivaroxaban redosing regimen indexed results are shown in Tables [d]-[f] and [m]-[r], herein.

**Table 21: Redosing regimens of rivaroxaban in response to various target single dose Cₘₐₓ in subjects with mild renal impairment treated with both rivaroxaban and verapamil using the logarithmic model.**

| **Mild Renal Impairment - R + V** | | | | | | | |
|---|---|---|---|---|---|---|---|
| | | | | | | | |

| **Logarithmic Redosing Single Dose** Cₘₐₓ | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 7.4 | 7.3 | 8.8 |
| **Mean of Top 3** | 275 | 268 | 317 | | 10.0 | 9.7 | 12.4 |
| **90% CI, Upper Boundary** | 275 | 268 | 317 | | 12.3 | 11.8 | 15.8 |
| **Median** | 275 | 268 | 317 | | 15.1 | 14.5 | |
| **Arithmetic Mean** | 275 | 268 | 317 | | 15.2 | 14.6 | |
| **Geometric Mean** | 275 | 268 | 317 | | 16.4 | 15.7 | |
| **90% CI, Lower Boundary** | 275 | 268 | 317 | | | | |
| **Minimum** | 275 | 268 | 317 | | | | |

**Table 22: Redosing regimens of rivaroxaban in response to various target steady state Cₘₐₓ in subjects with mild renal impairment treated with both rivaroxaban and verapamil using the logarithmic model.**

| **Mild Renal Impairment - R + V** | | | | | | | |
|---|---|---|---|---|---|---|---|
| | | | | | | | |

| **Logarithmic Redosing Steady State** Cₘₐₓ | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 5.8 | 5.7 | 6.6 |
| **Mean of Top 3** | 275 | 268 | 317 | | 7.3 | 7.1 | 8.6 |
| **90% CI, Upper Boundary** | 275 | 268 | 317 | | 8.6 | 8.3 | 10.4 |
| **Median** | 275 | 268 | 317 | | 10.1 | 9.7 | 12.5 |
| **Arithmetic Mean** | 275 | 268 | 317 | | 10.1 | 9.8 | 12.6 |
| **Geometric Mean** | 275 | 268 | 317 | | 10.7 | 10.4 | 13.5 |
| **90% CI, Lower Boundary** | 275 | 268 | 317 | | 14.0 | 13.4 | 18.3 |
| **Minimum** | 275 | 268 | 317 | | | | |

**Table 23: Redosing regimens of rivaroxaban in response to various target single dose Cₘₐₓ in subjects with mild renal impairment treated with a single dose of rivaroxaban using the logarithmic model.**

| **Mild Renal Impairment - R Only** | | | | | | | |
|---|---|---|---|---|---|---|---|
| | | | | | | | |

| **Logarithmic Redosing Single Dose** Cₘₐₓ | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 7.6 | 7.4 | 9.1 |
| **Mean of Top 3** | 275 | 268 | 317 | | 9.8 | 9.5 | 12.1 |
| **90% CI, Upper Boundary** | 275 | 268 | 317 | | 14.2 | 13.6 | 18.6 |
| **Median** | 275 | 268 | 317 | | | 19.6 | |
| **Arithmetic Mean** | 275 | 268 | 317 | | 17.1 | 16.4 | |
| **Geometric Mean** | 275 | 268 | 317 | | 18.7 | 17.8 | |
| **90% CI, Lower Boundary** | 275 | 268 | 317 | | | | |
| **Minimum** | 275 | 268 | 317 | | | | |

**Table 24: Redosing regimens of rivaroxaban in response to various target steady state Cₘₐₓ in subjects with mild renal impairment treated with a single dose of rivaroxaban using the logarithmic model.**

| **Mild Renal Impairment - R Only** | | | | | | | |
|---|---|---|---|---|---|---|---|
| | | | | | | | |

| **Logarithmic Redosing Steady State** Cₘₐₓ | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 6.2 | 6.1 | 7.2 |
| **Mean of Top 3** | 275 | 268 | 317 | | 7.6 | 7.4 | 9.1 |
| **90% CI, Upper Boundary** | 275 | 268 | 317 | | 10.4 | 10.0 | 12.9 |
| **Median** | 275 | 268 | 317 | | 14.1 | 13.5 | 18.4 |
| **Arithmetic Mean** | 275 | 268 | 317 | | 12.1 | 11.6 | 15.4 |
| **Geometric Mean** | 275 | 268 | 317 | | 13.0 | 12.5 | 16.8 |
| **90% CI, Lower Boundary** | 275 | 268 | 317 | | 16.2 | 15.5 | |
| **Minimum** | 275 | 268 | 317 | | | | |

**Table 25: Redosing regimens of rivaroxaban in response to various target single dose Cₘₐₓ in subjects with no renal impairment treated with both rivaroxaban and verapamil using the logarithmic model.**

| **No Renal Impairment - R Only** | | | | | | | |
|---|---|---|---|---|---|---|---|
| | | | | | | | |

| **Logarithmic Redosing Single Dose** Cₘₐₓ | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 8.9 | 8.6 | 10.9 |
| **Mean of Top 3** | 275 | 268 | 317 | | 10.3 | 9.9 | 12.8 |
| **90% CI, Upper Boundary** | 275 | 268 | 317 | | 11.5 | 11.1 | 14.6 |
| **Median** | 275 | 268 | 317 | | 14.5 | 13.9 | 19.0 |
| **Arithmetic Mean** | 275 | 268 | 317 | | 14.2 | 13.7 | 18.7 |
| **Geometric Mean** | 275 | 268 | 317 | | 15.1 | 14.5 | |
| **90% CI, Lower Boundary** | 275 | 268 | 317 | | | 19.9 | |
| **Minimum** | 275 | 268 | 317 | | | | |

**Table 26: Redosing regimens of rivaroxaban in response to various target steady state Cₘₐₓ in subjects with no renal impairment treated with both rivaroxaban and verapamil using the logarithmic model.**

| **No Renal Impairment - R Only** | | | | | | | |
|---|---|---|---|---|---|---|---|
| | | | | | | | |

| **Logarithmic Redosing Steady State** Cₘₐₓ | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 7.5 | 7.3 | 8.9 |
| **Mean of Top 3** | 275 | 268 | 317 | | 8.5 | 8.2 | 10.2 |
| **90% CI, Upper Boundary** | 275 | 268 | 317 | | 9.3 | 9.0 | 11.4 |
| **Median** | 275 | 268 | 317 | | 11.4 | 10.9 | 14.4 |
| **Arithmetic Mean** | 275 | 268 | 317 | | 11.2 | 10.8 | 14.1 |
| **Geometric Mean** | 275 | 268 | 317 | | 11.8 | 11.4 | 15.0 |
| **90% CI, Lower Boundary** | 275 | 268 | 317 | | 15.6 | 15.0 | |
| **Minimum** | 275 | 268 | 317 | | | | |

### Example 5 - PBPK Model For Extending Clincal Findings and Determining Redosing Levels of Rivaroxaban to reduce the Risk of Major Bleeding

A Physiologically based Pharmacokinetic (PBPK) model was developed using the data from the previously mentioned clinical studies to confirm and extend the key findings. Specifically, once fully developed the model was used to determine from multiple simulations ranges of AUC and Cmax for normal, mild renal impairment and moderate renal impairment subjects on rivaroxaban alone and on rivaroxaban and verapamil at steady state. The model was also used to test how these groups would react at verapamil doses ranging from 0mg to 360mg inclusively. These AUC and Cmax values were used to calculate redosing levels based on the exponential and logarithmic methods described herein.

Each of the rivaroxaban redosing values specified in the following tables is an aspect of the methods of the present disclosure, wherein a patient having the specified renal status, and concomitantly (or not as indicated) administered verapamil, and who would be administered the specified recommended rivaroxaban dose, may be re-dosed at the indicated level (or to a level rounded to the nearest mg or 0.5 mg).

**Table 27: Redosing regimens of rivaroxaban in response to various target AUC values in subjects with no renal impairment where the recommended dose is 15 mg rivaroxaban, no concomitant verapamil, using the exponential model.**

| **No Renal Impairment - 15 mg Recommended R, 0 V** | | | | | | | |
|---|---|---|---|---|---|---|---|
| | | | | | | | |

| **Exponential Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | | | 8.7 |
| **90% CI, Upper Boundary** | 3,792 | 3,404 | 2,448 | | | | |
| **80% CI, Upper Boundary** | 3,792 | 3,404 | 2,448 | | | | |
| **IQR Upper** | 3,792 | 3,404 | 2,448 | | | | |
| **Median** | 3,792 | 3,404 | 2,448 | | | | |
| **Arithmetic Mean** | 3,792 | 3,404 | 2,448 | | | | |
| **Geometric Mean** | 3,792 | 3,404 | 2,448 | | | | |
| **IQR Lower** | 3,792 | 3,404 | 2,448 | | | | |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 28: Redosing regimens of rivaroxaban in response to various target AUC values in subjects with no renal impairment where the recommended dose is 15 mg rivaroxaban, 120 mg concomitant verapamil, using the exponential model.**

| **No Renal Impairment - 15 mg Recommended R, 120 V** | | | | | | | |
|---|---|---|---|---|---|---|---|
| | | | | | | | |

| **Exponential Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 11.9 | 9.9 | 5.6 |
| **90% CI, Upper Boundary** | 3,792 | 3,404 | 2,448 | | | | 9.8 |
| **80% CI, Upper Boundary** | 3,792 | 3,404 | 2,448 | | | | 10.9 |
| **IQR Upper** | 3,792 | 3,404 | 2,448 | | | | 12.6 |
| **Median** | 3,792 | 3,404 | 2,448 | | | | |
| **Arithmetic Mean** | 3,792 | 3,404 | 2,448 | | | | 14.9 |
| **Geometric Mean** | 3,792 | 3,404 | 2,448 | | | | |
| **IQR Lower** | 3,792 | 3,404 | 2,448 | | | | |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 29: Redosing regimens of rivaroxaban in response to various target AUC values in subjects with no renal impairment where the recommended dose is 15 mg rivaroxaban, 240 mg concomitant verapamil, using the exponential model.**

| **No Renal Impairment - 15 mg Recommended R, 240 V** | | | | | | | |
|---|---|---|---|---|---|---|---|
| | | | | | | | |

| **Exponential Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 10.9 | 9.0 | 5.1 |
| **90% CI, Upper Boundary** | 3,792 | 3,404 | 2,448 | | | | 8.9 |
| **80% CI, Upper Boundary** | 3,792 | 3,404 | 2,448 | | | | 9.8 |
| **IQR Upper** | 3,792 | 3,404 | 2,448 | | | | 11.4 |
| **Median** | 3,792 | 3,404 | 2,448 | | | | 13.8 |
| **Arithmetic Mean** | 3,792 | 3,404 | 2,448 | | | | 13.5 |
| **Geometric Mean** | 3,792 | 3,404 | 2,448 | | | | 13.8 |
| **IQR Lower** | 3,792 | 3,404 | 2,448 | | | | |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 30: Redosing regimens of rivaroxaban in response to various target AUC values in subjects with no renal impairment where the recommended dose is 15 mg rivaroxaban, 360 mg concomitant verapamil, using the exponential model.**

| **No Renal Impairment - 15 mg Recommended R, 360 V** | | | | | | | |
|---|---|---|---|---|---|---|---|
| | | | | | | | |

| **Exponential Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 10.3 | 8.5 | 4.8 |
| **90% CI, Upper Boundary** | 3,792 | 3,404 | 2,448 | | | 14.6 | 8.3 |
| **80% CI, Upper Boundary** | 3,792 | 3,404 | 2,448 | | | | 9.1 |
| **IQR Upper** | 3,792 | 3,404 | 2,448 | | | | 10.7 |
| **Median** | 3,792 | 3,404 | 2,448 | | | | 12.9 |
| **Arithmetic Mean** | 3,792 | 3,404 | 2,448 | | | | 12.6 |
| **Geometric Mean** | 3,792 | 3,404 | 2,448 | | | | 12.9 |
| **IQR Lower** | 3,792 | 3,404 | 2,448 | | | | |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 31: Redosing regimens of rivaroxaban in response to various target AUC values in subjects with no renal impairment where the recommended dose is 20 mg rivaroxaban, no concomitant verapamil, using the exponential model.**

| **No Renal Impairment - 20 mg Recommended R, 0 V** | | | | | | | |
|---|---|---|---|---|---|---|---|
| | | | | | | | |

| **Exponential Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 14.3 | 11.9 | 6.7 |
| **90% CI, Upper Boundary** | 3,792 | 3,404 | 2,448 | | | | 12.1 |
| **80% CI, Upper Boundary** | 3,792 | 3,404 | 2,448 | | | | 13.4 |
| **IQR Upper** | 3,792 | 3,404 | 2,448 | | | | 15.7 |
| **Median** | 3,792 | 3,404 | 2,448 | | | | 19.2 |
| **Arithmetic Mean** | 3,792 | 3,404 | 2,448 | | | | 18.9 |
| **Geometric Mean** | 3,792 | 3,404 | 2,448 | | | | 19.4 |
| **IQR Lower** | 3,792 | 3,404 | 2,448 | | | | |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 32: Redosing regimens of rivaroxaban in response to various target AUC values in subjects with no renal impairment where the recommended dose is 20 mg rivaroxaban, 120 mg concomitant verapamil, using the exponential model.**

| **No Renal Impairment - 20 mg Recommended R, 120 V** | | | | | | | |
|---|---|---|---|---|---|---|---|
| | | | | | | | |

| **Exponential Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 9.1 | 7.6 | 4.3 |
| **90% CI, Upper Boundary** | 3,792 | 3,404 | 2,448 | | 16.1 | 13.3 | 7.5 |
| **80% CI, Upper Boundary** | 3,792 | 3,404 | 2,448 | | 17.8 | 14.8 | 8.4 |
| **IQR Upper** | 3,792 | 3,404 | 2,448 | | | 17.1 | 9.7 |
| **Median** | 3,792 | 3,404 | 2,448 | | | | 11.7 |
| **Arithmetic Mean** | 3,792 | 3,404 | 2,448 | | | | 11.5 |
| **Geometric Mean** | 3,792 | 3,404 | 2,448 | | | | 11.7 |
| **IQR Lower** | 3,792 | 3,404 | 2,448 | | | | 14.1 |
| **80% CI, Lower Boundary** | 3,792 | 3,404 | 2,448 | | | | 16.8 |
| **90% CI, Lower Boundary** | 3,792 | 3,404 | 2,448 | | | | 18.8 |
| **Minimum** | 3,792 | 3,404 | 2,448 | | | | |

**Table 33: Redosing regimens of rivaroxaban in response to various target AUC values in subjects with no renal impairment where the recommended dose is 20 mg rivaroxaban, 240 mg concomitant verapamil, using the exponential model.**

| **No Renal Impairment - 20 mg Recommended R, 240 V** | | | | | | | |
|---|---|---|---|---|---|---|---|
| | | | | | | | |

| **Exponential Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 8.4 | 6.9 | 3.9 |
| **90% CI, Upper Boundary** | 3,792 | 3,404 | 2,448 | | 14.5 | 12.0 | 6.8 |
| **80% CI, Upper Boundary** | 3,792 | 3,404 | 2,448 | | 16.0 | 13.3 | 7.5 |
| **IQR Upper** | 3,792 | 3,404 | 2,448 | | 18.6 | 15.5 | 8.7 |
| **Median** | 3,792 | 3,404 | 2,448 | | | 18.7 | 10.6 |
| **Arithmetic Mean** | 3,792 | 3,404 | 2,448 | | | 18.3 | 10.4 |
| **Geometric Mean** | 3,792 | 3,404 | 2,448 | | | 18.8 | 10.6 |
| **IQR Lower** | 3,792 | 3,404 | 2,448 | | | | 12.8 |
| **80% CI, Lower Boundary** | 3,792 | 3,404 | 2,448 | | | | 15.2 |
| **90% CI, Lower Boundary** | 3,792 | 3,404 | 2,448 | | | | 17.1 |
| **Minimum** | 3,792 | 3,404 | 2,448 | | | | |

**Table 34 Redosing regimens of rivaroxaban in response to various target AUC values in subjects with no renal impairment where the recommended dose is 20 mg rivaroxaban, 360 mg concomitant verapamil, using the exponential model.**

| **No Renal Impairment - 20 mg Recommended R, 360 V** | | | | | | | |
|---|---|---|---|---|---|---|---|
| | | | | | | | |

| **Exponential Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 7.9 | 6.6 | 3.7 |
| **90% CI, Upper Boundary** | 3,792 | 3,404 | 2,448 | | 13.6 | 11.2 | 6.4 |
| **80% CI, Upper Boundary** | 3,792 | 3,404 | 2,448 | | 14.9 | 12.4 | 7.0 |
| **IQR Upper** | 3,792 | 3,404 | 2,448 | | 17.5 | 14.5 | 8.2 |
| **Median** | 3,792 | 3,404 | 2,448 | | | 17.6 | 9.9 |
| **Arithmetic Mean** | 3,792 | 3,404 | 2,448 | | | 17.1 | 9.7 |
| **Geometric Mean** | 3,792 | 3,404 | 2,448 | | | 17.6 | 9.9 |
| **IQR Lower** | 3,792 | 3,404 | 2,448 | | | | 11.9 |
| **80% CI, Lower Boundary** | 3,792 | 3,404 | 2,448 | | | | 14.2 |
| **90% CI, Lower Boundary** | 3,792 | 3,404 | 2,448 | | | | 16.2 |
| **Minimum** | 3,792 | 3,404 | 2,448 | | | | |

**Table 35: Redosing regimens of rivaroxaban in response to various target AUC values in subjects with mild renal impairment where the recommended dose is 15 mg rivaroxaban, no concomitant verapamil, using the exponential model.**

| **Mild Renal Impairment - 15 mg Recommended R, 0 V** | | | | | | | |
|---|---|---|---|---|---|---|---|
| | | | | | | | |

| **Exponential Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | | | 8.8 |
| **90% CI, Upper Boundary** | 3,792 | 3,404 | 2,448 | | | | 14.2 |
| **80% CI, Upper Boundary** | 3,792 | 3,404 | 2,448 | | | | |
| **IQR Upper** | 3,792 | 3,404 | 2,448 | | | | |
| **Median** | 3,792 | 3,404 | 2,448 | | | | |
| **Arithmetic Mean** | 3,792 | 3,404 | 2,448 | | | | |
| **Geometric Mean** | 3,792 | 3,404 | 2,448 | | | | |
| **IQR Lower** | 3,792 | 3,404 | 2,448 | | | | |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 36: Redosing regimens of rivaroxaban in response to various target AUC values in subjects with mild renal impairment where the recommended dose is 15 mg rivaroxaban, 120 mg concomitant verapamil, using the exponential model.**

| **Mild Renal Impairment - 15 mg Recommended R, 120 V** | | | | | | | |
|---|---|---|---|---|---|---|---|
| | | | | | | | |

| **Exponential Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 12.4 | 10.3 | 5.8 |
| **90% CI, Upper Boundary** | 3,792 | 3,404 | 2,448 | | | | 8.6 |
| **80% CI, Upper Boundary** | 3,792 | 3,404 | 2,448 | | | | 9.4 |
| **IQR Upper** | 3,792 | 3,404 | 2,448 | | | | 11.3 |
| **Median** | 3,792 | 3,404 | 2,448 | | | | 13.8 |
| **Arithmetic Mean** | 3,792 | 3,404 | 2,448 | | | | 13.4 |
| **Geometric Mean** | 3,792 | 3,404 | 2,448 | | | | 13.8 |
| **IQR Lower** | 3,792 | 3,404 | 2,448 | | | | |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 37: Redosing regimens of rivaroxaban in response to various target AUC values in subjects with mild renal impairment where the recommended dose is 15 mg rivaroxaban, 240 mg concomitant verapamil, using the exponential model.**

| **Mild Renal Impairment - 15 mg Recommended R, 240 V** | | | | | | | |
|---|---|---|---|---|---|---|---|
| | | | | | | | |

| **Exponential Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 11.0 | 9.1 | 5.2 |
| **90% CI, Upper Boundary** | 3,792 | 3,404 | 2,448 | | | 13.6 | 7.7 |
| **80% CI, Upper Boundary** | 3,792 | 3,404 | 2,448 | | | | 8.5 |
| **IQR Upper** | 3,792 | 3,404 | 2,448 | | | | 10.2 |
| **Median** | 3,792 | 3,404 | 2,448 | | | | 12.5 |
| **Arithmetic Mean** | 3,792 | 3,404 | 2,448 | | | | 12.2 |
| **Geometric Mean** | 3,792 | 3,404 | 2,448 | | | | 12.5 |
| **IQR Lower** | 3,792 | 3,404 | 2,448 | | | | |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 38: Redosing regimens of rivaroxaban in response to various target AUC values in subjects with mild renal impairment where the recommended dose is 15 mg rivaroxaban, 360 mg concomitant verapamil, using the exponential model.**

| **Mild Renal Impairment - 15 mg Recommended R, 360 V** | | | | | | | |
|---|---|---|---|---|---|---|---|
| | | | | | | | |

| **Exponential Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 10.1 | 8.4 | 4.7 |
| **90% CI, Upper Boundary** | 3,792 | 3,404 | 2,448 | | | 12.8 | 7.2 |
| **80% CI, Upper Boundary** | 3,792 | 3,404 | 2,448 | | | 14.1 | 8.0 |
| **IQR Upper** | 3,792 | 3,404 | 2,448 | | | | 9.5 |
| **Median** | 3,792 | 3,404 | 2,448 | | | | 11.7 |
| **Arithmetic Mean** | 3,792 | 3,404 | 2,448 | | | | 11.4 |
| **Geometric Mean** | 3,792 | 3,404 | 2,448 | | | | 11.7 |
| **IQR Lower** | 3,792 | 3,404 | 2,448 | | | | 14.2 |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 39: Redosing regimens of rivaroxaban in response to various target AUC values in subjects with mild renal impairment where the recommended dose is 20 mg rivaroxaban, no concomitant verapamil, using the exponential model.**

| **Mild Renal Impairment - 20 mg Recommended R, 0 V** | | | | | | | |
|---|---|---|---|---|---|---|---|
| | | | | | | | |

| **Exponential Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 14.4 | 12.0 | 6.8 |
| **90% CI, Upper Boundary** | 3,792 | 3,404 | 2,448 | | | 19.2 | 10.9 |
| **80% CI, Upper Boundary** | 3,792 | 3,404 | 2,448 | | | | 12.0 |
| **IQR Upper** | 3,792 | 3,404 | 2,448 | | | | 14.7 |
| **Median** | 3,792 | 3,404 | 2,448 | | | | 17.8 |
| **Arithmetic Mean** | 3,792 | 3,404 | 2,448 | | | | 17.4 |
| **Geometric Mean** | 3,792 | 3,404 | 2,448 | | | | 17.9 |
| **IQR Lower** | 3,792 | 3,404 | 2,448 | | | | |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 40: Redosing regimens of rivaroxaban in response to various target AUC values in subjects with mild renal impairment where the recommended dose is 20 mg rivaroxaban, 120 mg concomitant verapamil, using the exponential model.**

| **Mild Renal Impairment - 20 mg Recommended R, 120 V** | | | | | | | |
|---|---|---|---|---|---|---|---|
| | | | | | | | |

| **Exponential Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 9.5 | 7.9 | 4.5 |
| **90% CI, Upper Boundary** | 3,792 | 3,404 | 2,448 | | 14.0 | 11.6 | 6.6 |
| **80% CI, Upper Boundary** | 3,792 | 3,404 | 2,448 | | 15.5 | 12.8 | 7.3 |
| **IQR Upper** | 3,792 | 3,404 | 2,448 | | 18.5 | 15.3 | 8.7 |
| **Median** | 3,792 | 3,404 | 2,448 | | | 18.7 | 10.6 |
| **Arithmetic Mean** | 3,792 | 3,404 | 2,448 | | | 18.3 | 10.3 |
| **Geometric Mean** | 3,792 | 3,404 | 2,448 | | | 18.7 | 10.6 |
| **IQR Lower** | 3,792 | 3,404 | 2,448 | | | | 12.9 |
| **80% CI, Lower Boundary** | 3,792 | 3,404 | 2,448 | | | | 15.7 |
| **90% CI, Lower Boundary** | 3,792 | 3,404 | 2,448 | | | | 17.6 |
| **Minimum** | 3,792 | 3,404 | 2,448 | | | | |

**Table 41: Redosing regimens of rivaroxaban in response to various target AUC values in subjects with mild renal impairment where the recommended dose is 20 mg rivaroxaban, 240 mg concomitant verapamil, using the exponential model.**

| **Mild Renal Impairment - 20 mg Recommended R, 240 V** | | | | | | | |
|---|---|---|---|---|---|---|---|
| | | | | | | | |

| **Exponential Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 8.5 | 7.0 | 4.0 |
| **90% CI, Upper Boundary** | 3,792 | 3,404 | 2,448 | | 12.6 | 10.4 | 5.9 |
| **80% CI, Upper Boundary** | 3,792 | 3,404 | 2,448 | | 14.0 | 11.6 | 6.6 |
| **IQR Upper** | 3,792 | 3,404 | 2,448 | | 16.6 | 13.8 | 7.8 |
| **Median** | 3,792 | 3,404 | 2,448 | | | 16.9 | 9.6 |
| **Arithmetic Mean** | 3,792 | 3,404 | 2,448 | | 19.9 | 16.5 | 9.3 |
| **Geometric Mean** | 3,792 | 3,404 | 2,448 | | | 16.9 | 9.6 |
| **IQR Lower** | 3,792 | 3,404 | 2,448 | | | | 11.7 |
| **80% CI, Lower Boundary** | 3,792 | 3,404 | 2,448 | | | | 14.2 |
| **90% CI, Lower Boundary** | 3,792 | 3,404 | 2,448 | | | | 16.0 |
| **Minimum** | 3,792 | 3,404 | 2,448 | | | | |

**Table 42: Redosing regimens of rivaroxaban in response to various target AUC values in subjects with mild renal impairment where the recommended dose is 20 mg rivaroxaban, 240 mg concomitant verapamil, using the exponential model.**

| **Mild Renal Impairment - 20 mg Recommended R, 360 V** | | | | | | | |
|---|---|---|---|---|---|---|---|
| | | | | | | | |
| **Exponential Redosing AUC** | | | | | | | |
| | | | | | | | |
| **Group** | **Target 1** | **Target 2** | **Target 3** | | **Dose 1** | **Dose 2** | **Dose 3** |
| **Maximum** | 3,792 | 3,404 | 2,448 | | 7.8 | 6.4 | 3.6 |
| **90% CI, Upper Boundary** | 3,792 | 3,404 | 2,448 | | 11.9 | 9.8 | 5.6 |
| **80% CI, Upper Boundary** | 3,792 | 3,404 | 2,448 | | 13.1 | 10.8 | 6.1 |
| **IQR Upper** | 3,792 | 3,404 | 2,448 | | 15.6 | 13.0 | 7.3 |
| **Median** | 3,792 | 3,404 | 2,448 | | 19.1 | 15.8 | 9.0 |
| **Arithmetic Mean** | 3,792 | 3,404 | 2,448 | | 18.7 | 15.5 | 8.8 |
| **Geometric Mean** | 3,792 | 3,404 | 2,448 | | 19.1 | 15.9 | 9.0 |
| **IQR Lower** | 3,792 | 3,404 | 2,448 | | | 19.4 | 11.0 |
| **80% CI, Lower Boundary** | 3,792 | 3,404 | 2,448 | | | | 13.3 |
| **90% CI, Lower Boundary** | 3,792 | 3,404 | 2,448 | | | | 15.1 |
| **Minimum** | 3,792 | 3,404 | 2,448 | | | | |

**Table 43: Redosing regimens of rivaroxaban in response to various target AUC values in subjects with moderate renal impairment where the recommended dose is 15 mg rivaroxaban, no concomitant verapamil, using the exponential model.**

| **Moderate Renal Impairment - 15 mg Recommended R, 0 V** | | | | | | | |
|---|---|---|---|---|---|---|---|
| | | | | | | | |
| **Exponential Redosing AUC** | | | | | | | |
| | | | | | | | |
| **Group** | **Target 1** | **Target 2** | **Target 3** | | **Dose 1** | **Dose 2** | **Dose 3** |
| **Maximum** | 3,792 | 3,404 | 2,448 | | | 14.1 | 8.0 |
| **90% CI, Upper Boundary** | 3,792 | 3,404 | 2,448 | | | | 12.6 |
| **80% CI, Upper Boundary** | 3,792 | 3,404 | 2,448 | | | | 14.3 |
| **IQR Upper** | 3,792 | 3,404 | 2,448 | | | | |
| **Median** | 3,792 | 3,404 | 2,448 | | | | |
| **Arithmetic Mean** | 3,792 | 3,404 | 2,448 | | | | |
| **Geometric Mean** | 3,792 | 3,404 | 2,448 | | | | |
| **IQR Lower** | 3,792 | 3,404 | 2,448 | | | | |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 44: Redosing regimens of rivaroxaban in response to various target AUC values in subjects with moderate renal impairment where the recommended dose is 15 mg rivaroxaban, 120 mg concomitant verapamil, using the exponential model.**

| **Moderate Renal Impairment - 15 mg Recommended R, 120 V** | | | | | | | |
|---|---|---|---|---|---|---|---|
| | | | | | | | |
| **Exponential Redosing AUC** | | | | | | | |
| | | | | | | | |
| **Group** | **Target 1** | **Target 2** | **Target 3** | | **Dose 1** | **Dose 2** | **Dose 3** |
| **Maximum** | 3,792 | 3,404 | 2,448 | | 9.7 | 8.1 | 4.6 |
| **90% CI, Upper Boundary** | 3,792 | 3,404 | 2,448 | | | 13.1 | 7.4 |
| **80% CI, Upper Boundary** | 3,792 | 3,404 | 2,448 | | | 14.9 | 8.4 |
| **IQR Upper** | 3,792 | 3,404 | 2,448 | | | | 10.1 |
| **Median** | 3,792 | 3,404 | 2,448 | | | | 12.3 |
| **Arithmetic Mean** | 3,792 | 3,404 | 2,448 | | | | 12.0 |
| **Geometric Mean** | 3,792 | 3,404 | 2,448 | | | | 12.4 |
| **IQR Lower** | 3,792 | 3,404 | 2,448 | | | | |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 45: Redosing regimens of rivaroxaban in response to various target AUC values in subjects with moderate renal impairment where the recommended dose is 15 mg rivaroxaban, 240 mg concomitant verapamil, using the exponential model.**

| **Moderate Renal Impairment - 15 mg Recommended R, 240 V** | | | | | | | |
|---|---|---|---|---|---|---|---|
| | | | | | | | |
| **Exponential Redosing AUC** | | | | | | | |
| | | | | | | | |
| **Group** | **Target 1** | **Target 2** | **Target 3** | | **Dose 1** | **Dose 2** | **Dose 3** |
| **Maximum** | 3,792 | 3,404 | 2,448 | | 8.7 | 7.2 | 4.1 |
| **90% CI, Upper Boundary** | 3,792 | 3,404 | 2,448 | | 14.3 | 11.8 | 6.7 |
| **80% CI, Upper Boundary** | 3,792 | 3,404 | 2,448 | | | 13.5 | 7.6 |
| **IQR Upper** | 3,792 | 3,404 | 2,448 | | | | 9.2 |
| **Median** | 3,792 | 3,404 | 2,448 | | | | 11.2 |
| **Arithmetic Mean** | 3,792 | 3,404 | 2,448 | | | | 10.9 |
| **Geometric Mean** | 3,792 | 3,404 | 2,448 | | | | 11.2 |
| **IQR Lower** | 3,792 | 3,404 | 2,448 | | | | 13.8 |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 46: Redosing regimens of rivaroxaban in response to various target AUC values in subjects with moderate renal impairment where the recommended dose is 15 mg rivaroxaban, 360 mg concomitant verapamil, using the exponential model.**

| **Moderate Renal Impairment - 15 mg Recommended R, 360 V** | | | | | | | |
|---|---|---|---|---|---|---|---|
| | | | | | | | |
| **Exponential Redosing AUC** | | | | | | | |
| | | | | | | | |
| **Group** | **Target 1** | **Target 2** | **Target 3** | | **Dose 1** | **Dose 2** | **Dose 3** |
| **Maximum** | 3,792 | 3,404 | 2,448 | | 8.1 | 6.7 | 3.8 |
| **90% CI, Upper Boundary** | 3,792 | 3,404 | 2,448 | | 13.4 | 11.1 | 6.3 |
| **80% CI, Upper Boundary** | 3,792 | 3,404 | 2,448 | | | 12.6 | 7.1 |
| **IQR Upper** | 3,792 | 3,404 | 2,448 | | | | 8.6 |
| **Median** | 3,792 | 3,404 | 2,448 | | | | 10.5 |
| **Arithmetic Mean** | 3,792 | 3,404 | 2,448 | | | | 10.3 |
| **Geometric Mean** | 3,792 | 3,404 | 2,448 | | | | 10.6 |
| **IQR Lower** | 3,792 | 3,404 | 2,448 | | | | 13.0 |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 47: Redosing regimens of rivaroxaban in response to various target AUC values in subjects with moderate renal impairment where the recommended dose is 20 mg rivaroxaban, no concomitant verapamil, using the exponential model.**

| **Moderate Renal Impairment - 20 mg Recommended R, 0 V** | | | | | | | |
|---|---|---|---|---|---|---|---|
| | | | | | | | |
| **Exp onential Redosing AU C** | | | | | | | |
| | | | | | | | |
| **Group** | **Target 1** | **Target 2** | **Target 3** | | **Dose 1** | **Dose 2** | **Dose 3** |
| **Maximum** | 3,792 | 3,404 | 2,448 | | 13.1 | 10.9 | 6.1 |
| **90% CI, Upper Boundary** | 3,792 | 3,404 | 2,448 | | | 17.0 | 9.6 |
| **80% CI, Upper Boundary** | 3,792 | 3,404 | 2,448 | | | 19.4 | 11.0 |
| **IQR Upper** | 3,792 | 3,404 | 2,448 | | | | 13.1 |
| **Median** | 3,792 | 3,404 | 2,448 | | | | 16.1 |
| **Arithmetic Mean** | 3,792 | 3,404 | 2,448 | | | | 15.8 |
| **Geometric Mean** | 3,792 | 3,404 | 2,448 | | | | 16.3 |
| **IQR Lower** | 3,792 | 3,404 | 2,448 | | | | |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 48: Redosing regimens of rivaroxaban in response to various target AUC values in subjects with moderate renal impairment where the recommended dose is 20 mg rivaroxaban, 120 mg concomitant verapamil, using the exponential model.**

| **Moderate Renal Impairment-20 mg Recommended R, 120 V** | | | | | | | |
|---|---|---|---|---|---|---|---|
| | | | | | | | |
| **Exponential Redosing AUC** | | | | | | | |
| | | | | | | | |
| **Group** | **Target 1** | **Target 2** | **Target 3** | | **Dose 1** | **Dose 2** | **Dose 3** |
| **Maximum** | 3,792 | 3,404 | 2,448 | | 7.5 | 6.2 | 3.5 |
| **90% CI, Upper Boundary** | 3,792 | 3,404 | 2,448 | | 12.1 | 10.1 | 5.7 |
| **80% CI, Upper Boundary** | 3,792 | 3,404 | 2,448 | | 13.8 | 11.5 | 6.5 |
| **IQR Upper** | 3,792 | 3,404 | 2,448 | | 16.6 | 13.8 | 7.8 |
| **Median** | 3,792 | 3,404 | 2,448 | | | 16.7 | 9.5 |
| **Arithmetic Mean** | 3,792 | 3,404 | 2,448 | | 19.7 | 16.4 | 9.3 |
| **Geometric Mean** | 3,792 | 3,404 | 2,448 | | | 16.8 | 9.5 |
| **IQR Lower** | 3,792 | 3,404 | 2,448 | | | | 11.7 |
| **80% CI, Lower Boundary** | 3,792 | 3,404 | 2,448 | | | | 14.0 |
| **90% CI, Lower Boundary** | 3,792 | 3,404 | 2,448 | | | | 15.7 |
| **Minimum** | 3,792 | 3,404 | 2,448 | | | | |

**Table 49: Redosing regimens of rivaroxaban in response to various target AUC values in subjects with moderate renal impairment where the recommended dose is 20 mg rivaroxaban, 240 mg concomitant verapamil, using the exponential model.**

| **Moderate Renal Impairment - 20 mg Recommended R, 240 V** | | | | | | | |
|---|---|---|---|---|---|---|---|
| | | | | | | | |
| **Exponential Redosing AUC** | | | | | | | |
| | | | | | | | |
| **Group** | **Target 1** | **Target 2** | **Target 3** | | **Dose 1** | **Dose 2** | **Dose 3** |
| **Maximum** | 3,792 | 3,404 | 2,448 | | 6.7 | 5.6 | 3.1 |
| **90% CI, Upper Boundary** | 3,792 | 3,404 | 2,448 | | 11.0 | 9.1 | 5.1 |
| **80% CI, Upper Boundary** | 3,792 | 3,404 | 2,448 | | 12.5 | 10.3 | 5.9 |
| **IQR Upper** | 3,792 | 3,404 | 2,448 | | 15.0 | 12.5 | 7.1 |
| **Median** | 3,792 | 3,404 | 2,448 | | 18.3 | 15.2 | 8.6 |
| **Arithmetic Mean** | 3,792 | 3,404 | 2,448 | | 17.9 | 14.8 | 8.4 |
| **Geometric Mean** | 3,792 | 3,404 | 2,448 | | 18.4 | 15.3 | 8.6 |
| **IQR Lower** | 3,792 | 3,404 | 2,448 | | | 18.7 | 10.6 |
| **80% CI, Lower Boundary** | 3,792 | 3,404 | 2,448 | | | | 12.8 |
| **90% CI, Lower Boundary** | 3,792 | 3,404 | 2,448 | | | | 14.3 |
| **Minimum** | 3,792 | 3,404 | 2,448 | | | | |

**Table 50: Redosing regimens of rivaroxaban in response to various target AUC values in subjects with moderate renal impairment where the recommended dose is 20 mg rivaroxaban, 360 mg concomitant verapamil, using the exponential model.**

| **Moderate Renal Impairment - 20 mg Recommended R, 360 V** | | | | | | | |
|---|---|---|---|---|---|---|---|
| | | | | | | | |

| **Exponential Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 6.2 | 5.2 | 2.9 |
| **90% CI, Upper Boundary** | 3,792 | 3,404 | 2,448 | | 10.3 | 8.5 | 4.8 |
| **80% CI, Upper Boundary** | 3,792 | 3,404 | 2,448 | | 11.6 | 9.6 | 5.5 |
| **IQR Upper** | 3,792 | 3,404 | 2,448 | | 14.1 | 11.7 | 6.6 |
| **Median** | 3,792 | 3,404 | 2,448 | | 17.2 | 14.3 | 8.1 |
| **Arithmetic Mean** | 3,792 | 3,404 | 2,448 | | 16.8 | 14.0 | 7.9 |
| **Geometric Mean** | 3,792 | 3,404 | 2,448 | | 17.3 | 14.3 | 8.1 |
| **IQR Lower** | 3,792 | 3,404 | 2,448 | | | 17.7 | 10.0 |
| **80% CI, Lower Boundary** | 3,792 | 3,404 | 2,448 | | | | 12.0 |
| **90% CI, Lower Boundary** | 3,792 | 3,404 | 2,448 | | | | 13.5 |
| **Minimum** | 3,792 | 3,404 | 2,448 | | | | |

**Table 51: Redosing regimens of rivaroxaban in response to various target AUC values in subjects with no renal impairment where the recommended dose is 15 mg rivaroxaban, no concomitant verapamil, using the logarithmic model.**

| **No Renal Impairment - 15 mg Recommended R, 0 V** | | | | | | | |
|---|---|---|---|---|---|---|---|
| | | | | | | | |

| **Logarithmic Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | | 14.4 | 10.4 |
| **90% CI, Upper Boundary** | 3,792 | 3,404 | 2,448 | | | | 14.6 |
| **80% CI, Upper Boundary** | 3,792 | 3,404 | 2,448 | | | | |
| **IQR Upper** | 3,792 | 3,404 | 2,448 | | | | |
| **Median** | 3,792 | 3,404 | 2,448 | | | | |
| **Arithmetic Mean** | 3,792 | 3,404 | 2,448 | | | | |
| **Geometric Mean** | 3,792 | 3,404 | 2,448 | | | | |
| **IQR Lower** | 3,792 | 3,404 | 2,448 | | | | |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 52: Redosing regimens of rivaroxaban in response to various target AUC values in subjects with no renal impairment where the recommended dose is 15 mg rivaroxaban, 120 mg concomitant verapamil, using the logarithmic model.**

| **No Renal Impairment - 15 mg Recommended R, 120 V** | | | | | | | |
|---|---|---|---|---|---|---|---|
| | | | | | | | |

| **Logarithmic Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 12.4 | 11.1 | 8.0 |
| **90% CI, Upper Boundary** | 3,792 | 3,404 | 2,448 | | | | 11.1 |
| **80% CI, Upper Boundary** | 3,792 | 3,404 | 2,448 | | | | 11.7 |
| **IQR Upper** | 3,792 | 3,404 | 2,448 | | | | 12.8 |
| **Median** | 3,792 | 3,404 | 2,448 | | | | 14.3 |
| **Arithmetic Mean** | 3,792 | 3,404 | 2,448 | | | | 14.1 |
| **Geometric Mean** | 3,792 | 3,404 | 2,448 | | | | 14.3 |
| **IQR Lower** | 3,792 | 3,404 | 2,448 | | | | |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 53: Redosing regimens of rivaroxaban in response to various target AUC values in subjects with no renal impairment where the recommended dose is 15 mg rivaroxaban, 240 mg concomitant verapamil, using the logarithmic model.**

| **No Renal Impairment - 15 mg Recommended R, 240 V** | | | | | | | |
|---|---|---|---|---|---|---|---|
| | | | | | | | |

| **Logarithmic Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 11.8 | 10.6 | 7.6 |
| **90% CI, Upper Boundary** | 3,792 | 3,404 | 2,448 | | | 14.5 | 10.4 |
| **80% CI, Upper Boundary** | 3,792 | 3,404 | 2,448 | | | | 11.0 |
| **IQR Upper** | 3,792 | 3,404 | 2,448 | | | | 12.1 |
| **Median** | 3,792 | 3,404 | 2,448 | | | | 13.5 |
| **Arithmetic Mean** | 3,792 | 3,404 | 2,448 | | | | 13.3 |
| **Geometric Mean** | 3,792 | 3,404 | 2,448 | | | | 13.5 |
| **IQR Lower** | 3,792 | 3,404 | 2,448 | | | | 15.0 |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 54: Redosing regimens of rivaroxaban in response to various target AUC values in subjects with no renal impairment where the recommended dose is 15 mg rivaroxaban, 360 mg concomitant verapamil, using the logarithmic model.**

| **No Renal Impairment - 15 mg Recommended R, 360 V** | | | | | | | |
|---|---|---|---|---|---|---|---|
| | | | | | | | |

| **Logarithmic Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 11.4 | 10.2 | 7.3 |
| **90% CI, Upper Boundary** | 3,792 | 3,404 | 2,448 | | | 13.9 | 10.0 |
| **80% CI, Upper Boundary** | 3,792 | 3,404 | 2,448 | | | 14.8 | 10.6 |
| **IQR Upper** | 3,792 | 3,404 | 2,448 | | | | 11.6 |
| **Median** | 3,792 | 3,404 | 2,448 | | | | 13.0 |
| **Arithmetic Mean** | 3,792 | 3,404 | 2,448 | | | | 12.8 |
| **Geometric Mean** | 3,792 | 3,404 | 2,448 | | | | 13.0 |
| **IQR Lower** | 3,792 | 3,404 | 2,448 | | | | 14.4 |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 55: Redosing regimens of rivaroxaban in response to various target AUC values in subjects with no renal impairment where the recommended dose is 20 mg rivaroxaban, no concomitant verapamil, using the logarithmic model.**

| **No Renal Impairment - 20 mg Recommended R, 0 V** | | | | | | | |
|---|---|---|---|---|---|---|---|
| | | | | | | | |

| **Logarithmic Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 16.0 | 14.4 | 10.4 |
| **90% CI, Upper Boundary** | 3,792 | 3,404 | 2,448 | | | | 14.6 |
| **80% CI, Upper Boundary** | 3,792 | 3,404 | 2,448 | | | | 15.5 |
| **IQR Upper** | 3,792 | 3,404 | 2,448 | | | | 16.9 |
| **Median** | 3,792 | 3,404 | 2,448 | | | | 19.0 |
| **Arithmetic Mean** | 3,792 | 3,404 | 2,448 | | | | 18.8 |
| **Geometric Mean** | 3,792 | 3,404 | 2,448 | | | | 19.1 |
| **IQR Lower** | 3,792 | 3,404 | 2,448 | | | | |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 56: Redosing regimens of rivaroxaban in response to various target AUC values in subjects with no renal impairment where the recommended dose is 20 mg rivaroxaban, 120 mg concomitant verapamil, using the logarithmic model.**

| **No Renal Impairment - 20 mg Recommended R, 120 V** | | | | | | | |
|---|---|---|---|---|---|---|---|
| | | | | | | | |

| **Logarithmic Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 12.4 | 11.1 | 8.0 |
| **90% CI, Upper Boundary** | 3,792 | 3,404 | 2,448 | | 17.1 | 15.4 | 11.1 |
| **80% CI, Upper Boundary** | 3,792 | 3,404 | 2,448 | | 18.2 | 16.3 | 11.7 |
| **IQR Upper** | 3,792 | 3,404 | 2,448 | | 19.8 | 17.8 | 12.8 |
| **Median** | 3,792 | 3,404 | 2,448 | | | 19.9 | 14.3 |
| **Arithmetic Mean** | 3,792 | 3,404 | 2,448 | | | 19.6 | 14.1 |
| **Geometric Mean** | 3,792 | 3,404 | 2,448 | | | 19.9 | 14.3 |
| **IQR Lower** | 3,792 | 3,404 | 2,448 | | | | 15.9 |
| **80% CI, Lower Boundary** | 3,792 | 3,404 | 2,448 | | | | 17.6 |
| **90% CI, Lower Boundary** | 3,792 | 3,404 | 2,448 | | | | 18.8 |
| **Minimum** | 3,792 | 3,404 | 2,448 | | | | |

**Table 57: Redosing regimens of rivaroxaban in response to various target AUC values in subjects with no renal impairment where the recommended dose is 20 mg rivaroxaban, 240 mg concomitant verapamil, using the logarithmic model.**

| **No Renal Impairment - 20 mg Recommended R, 240 V** | | | | | | | |
|---|---|---|---|---|---|---|---|
| | | | | | | | |

| **Logarithmic Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 11.8 | 10.6 | 7.6 |
| **90% CI, Upper Boundary** | 3,792 | 3,404 | 2,448 | | 16.1 | 14.5 | 10.4 |
| **80% CI, Upper Boundary** | 3,792 | 3,404 | 2,448 | | 17.1 | 15.3 | 11.0 |
| **IQR Upper** | 3,792 | 3,404 | 2,448 | | 18.7 | 16.8 | 12.1 |
| **Median** | 3,792 | 3,404 | 2,448 | | | 18.7 | 13.5 |
| **Arithmetic Mean** | 3,792 | 3,404 | 2,448 | | | 18.5 | 13.3 |
| **Geometric Mean** | 3,792 | 3,404 | 2,448 | | | 18.8 | 13.5 |
| **IQR Lower** | 3,792 | 3,404 | 2,448 | | | | 15.0 |
| **80% CI, Lower Boundary** | 3,792 | 3,404 | 2,448 | | | | 16.6 |
| **90% CI, Lower Boundary** | 3,792 | 3,404 | 2,448 | | | | 17.8 |
| **Minimum** | 3,792 | 3,404 | 2,448 | | | | |

**Table 58: Redosing regimens of rivaroxaban in response to various target AUC values in subjects with no renal impairment where the recommended dose is 20 mg rivaroxaban, 360 mg concomitant verapamil, using the logarithmic model.**

| **No Renal Impairment - 20 mg Recommended R, 360 V** | | | | | | | |
|---|---|---|---|---|---|---|---|
| | | | | | | | |

| **Logarithmic Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 11.4 | 10.2 | 7.3 |
| **90% CI, Upper Boundary** | 3,792 | 3,404 | 2,448 | | 15.5 | 13.9 | 10.0 |
| **80% CI, Upper Boundary** | 3,792 | 3,404 | 2,448 | | 16.4 | 14.8 | 10.6 |
| **IQR Upper** | 3,792 | 3,404 | 2,448 | | 18.0 | 16.2 | 11.6 |
| **Median** | 3,792 | 3,404 | 2,448 | | | 18.0 | 13.0 |
| **Arithmetic Mean** | 3,792 | 3,404 | 2,448 | | 19.8 | 17.8 | 12.8 |
| **Geometric Mean** | 3,792 | 3,404 | 2,448 | | | 18.0 | 13.0 |
| **IQR Lower** | 3,792 | 3,404 | 2,448 | | | | 14.4 |
| **80% CI, Lower Boundary** | 3,792 | 3,404 | 2,448 | | | | 16.0 |
| **90% CI, Lower Boundary** | 3,792 | 3,404 | 2,448 | | | | 17.2 |
| **Minimum** | 3,792 | 3,404 | 2,448 | | | | |

**Table 59: Redosing regimens of rivaroxaban in response to various target AUC values in subjects with mild renal impairment where the recommended dose is 15 mg rivaroxaban, no concomitant verapamil, using the logarithmic model.**

| **Mild Renal Impairment - 15 mg Recommended R, 0 V** | | | | | | | |
|---|---|---|---|---|---|---|---|
| | | | | | | | |

| **Logarithmic Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | | 14.5 | 10.4 |
| **90% CI, Upper Boundary** | 3,792 | 3,404 | 2,448 | | | | 13.7 |
| **80% CI, Upper Boundary** | 3,792 | 3,404 | 2,448 | | | | 14.5 |
| **IQR Upper** | 3,792 | 3,404 | 2,448 | | | | |
| **Median** | 3,792 | 3,404 | 2,448 | | | | |
| **Arithmetic Mean** | 3,792 | 3,404 | 2,448 | | | | |
| **Geometric Mean** | 3,792 | 3,404 | 2,448 | | | | |
| **IQR Lower** | 3,792 | 3,404 | 2,448 | | | | |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 60: Redosing regimens of rivaroxaban in response to various target AUC values in subjects with mild renal impairment where the recommended dose is 15 mg rivaroxaban, 120 mg concomitant verapamil, using the logarithmic model.**

| **Mild Renal Impairment - 15 mg Recommended R, 120 V** | | | | | | | |
|---|---|---|---|---|---|---|---|
| | | | | | | | |

| **Logarithmic Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 12.7 | 11.4 | 8.2 |
| **90% CI, Upper Boundary** | 3,792 | 3,404 | 2,448 | | | 14.2 | 10.2 |
| **80% CI, Upper Boundary** | 3,792 | 3,404 | 2,448 | | | | 10.8 |
| **IQR Upper** | 3,792 | 3,404 | 2,448 | | | | 12.0 |
| **Median** | 3,792 | 3,404 | 2,448 | | | | 13.5 |
| **Arithmetic Mean** | 3,792 | 3,404 | 2,448 | | | | 13.3 |
| **Geometric Mean** | 3,792 | 3,404 | 2,448 | | | | 13.5 |
| **IQR Lower** | 3,792 | 3,404 | 2,448 | | | | |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 61: Redosing regimens of rivaroxaban in response to various target AUC values in subjects with mild renal impairment where the recommended dose is 15 mg rivaroxaban, 240 mg concomitant verapamil, using the logarithmic model.**

| **Mild Renal Impairment - 15 mg Recommended R, 240 V** | | | | | | | |
|---|---|---|---|---|---|---|---|
| | | | | | | | |

| **Logarithmic Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 11.8 | 10.6 | 7.6 |
| **90% CI, Upper Boundary** | 3,792 | 3,404 | 2,448 | | 14.9 | 13.3 | 9.6 |
| **80% CI, Upper Boundary** | 3,792 | 3,404 | 2,448 | | | 14.2 | 10.2 |
| **IQR Upper** | 3,792 | 3,404 | 2,448 | | | | 11.3 |
| **Median** | 3,792 | 3,404 | 2,448 | | | | 12.7 |
| **Arithmetic Mean** | 3,792 | 3,404 | 2,448 | | | | 12.5 |
| **Geometric Mean** | 3,792 | 3,404 | 2,448 | | | | 12.7 |
| **IQR Lower** | 3,792 | 3,404 | 2,448 | | | | 14.2 |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 62: Redosing regimens of rivaroxaban in response to various target AUC values in subjects with mild renal impairment where the recommended dose is 15 mg rivaroxaban, 360 mg concomitant verapamil, using the logarithmic model.**

| **Mild Renal Impairment - 15 mg Recommended R, 360 V** | | | | | | | |
|---|---|---|---|---|---|---|---|
| | | | | | | | |

| **Logarithmic Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 11.3 | 10.1 | 7.3 |
| **90% CI, Upper Boundary** | 3,792 | 3,404 | 2,448 | | 14.4 | 12.9 | 9.3 |
| **80% CI, Upper Boundary** | 3,792 | 3,404 | 2,448 | | | 13.7 | 9.8 |
| **IQR Upper** | 3,792 | 3,404 | 2,448 | | | | 10.9 |
| **Median** | 3,792 | 3,404 | 2,448 | | | | 12.2 |
| **Arithmetic Mean** | 3,792 | 3,404 | 2,448 | | | | 12.1 |
| **Geometric Mean** | 3,792 | 3,404 | 2,448 | | | | 12.2 |
| **IQR Lower** | 3,792 | 3,404 | 2,448 | | | | 13.7 |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 63: Redosing regimens of rivaroxaban in response to various target AUC values in subjects with mild renal impairment where the recommended dose is 20 mg rivaroxaban, no concomitant verapamil, using the logarithmic model.**

| **Mild Renal Impairment - 20 mg Recommended R, 0 V** | | | | | | | |
|---|---|---|---|---|---|---|---|
| | | | | | | | |

| **Logarithmic Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 16.1 | 14.5 | 10.4 |
| **90% CI, Upper Boundary** | 3,792 | 3,404 | 2,448 | | | 19.0 | 13.7 |
| **80% CI, Upper Boundary** | 3,792 | 3,404 | 2,448 | | | | 14.5 |
| **IQR Upper** | 3,792 | 3,404 | 2,448 | | | | 16.3 |
| **Median** | 3,792 | 3,404 | 2,448 | | | | 18.2 |
| **Arithmetic Mean** | 3,792 | 3,404 | 2,448 | | | | 18.0 |
| **Geometric Mean** | 3,792 | 3,404 | 2,448 | | | | 18.3 |
| **IQR Lower** | 3,792 | 3,404 | 2,448 | | | | |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 64: Redosing regimens of rivaroxaban in response to various target AUC values in subjects with mild renal impairment where the recommended dose is 20 mg rivaroxaban, 120 mg concomitant verapamil, using the logarithmic model.**

| **Mild Renal Impairment - 20 mg Recommended R, 120 V** | | | | | | | |
|---|---|---|---|---|---|---|---|
| | | | | | | | |

| **Logarithmic Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 12.7 | 11.4 | 8.2 |
| **90% CI, Upper Boundary** | 3,792 | 3,404 | 2,448 | | 15.8 | 14.2 | 10.2 |
| **80% CI, Upper Boundary** | 3,792 | 3,404 | 2,448 | | 16.8 | 15.1 | 10.8 |
| **IQR Upper** | 3,792 | 3,404 | 2,448 | | 18.6 | 16.7 | 12.0 |
| **Median** | 3,792 | 3,404 | 2,448 | | | 18.7 | 13.5 |
| **Arithmetic Mean** | 3,792 | 3,404 | 2,448 | | | 18.5 | 13.3 |
| **Geometric Mean** | 3,792 | 3,404 | 2,448 | | | 18.7 | 13.5 |
| **IQR Lower** | 3,792 | 3,404 | 2,448 | | | | 15.1 |
| **80% CI, Lower Boundary** | 3,792 | 3,404 | 2,448 | | | | 16.9 |
| **90% CI, Lower Boundary** | 3,792 | 3,404 | 2,448 | | | | 18.1 |
| **Minimum** | 3,792 | 3,404 | 2,448 | | | | |

**Table 65: Redosing regimens of rivaroxaban in response to various target AUC values in subjects with mild renal impairment where the recommended dose is 20 mg rivaroxaban, 240 mg concomitant verapamil, using the logarithmic model.**

| **Mild Renal Impairment - 20 mg Recommended R, 240 V** | | | | | | | |
|---|---|---|---|---|---|---|---|
| | | | | | | | |

| **Logarithmic Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 11.8 | 10.6 | 7.6 |
| **90% CI, Upper Boundary** | 3,792 | 3,404 | 2,448 | | 14.9 | 13.3 | 9.6 |
| **80% CI, Upper Boundary** | 3,792 | 3,404 | 2,448 | | 15.8 | 14.2 | 10.2 |
| **IQR Upper** | 3,792 | 3,404 | 2,448 | | 17.5 | 15.7 | 11.3 |
| **Median** | 3,792 | 3,404 | 2,448 | | 19.7 | 17.7 | 12.7 |
| **Arithmetic Mean** | 3,792 | 3,404 | 2,448 | | 19.4 | 17.4 | 12.5 |
| **Geometric Mean** | 3,792 | 3,404 | 2,448 | | 19.7 | 17.7 | 12.7 |
| **IQR Lower** | 3,792 | 3,404 | 2,448 | | | 19.8 | 14.2 |
| **80% CI, Lower Boundary** | 3,792 | 3,404 | 2,448 | | | | 15.9 |
| **90% CI, Lower Boundary** | 3,792 | 3,404 | 2,448 | | | | 17.1 |
| **Minimum** | 3,792 | 3,404 | 2,448 | | | | |

**Table 66: Redosing regimens of rivaroxaban in response to various target AUC values in subjects with mild renal impairment where the recommended dose is 20 mg rivaroxaban, 240 mg concomitant verapamil, using the logarithmic model.**

| **Mild Renal Impairment - 20 mg Recommended R, 360 V** | | | | | | | |
|---|---|---|---|---|---|---|---|
| | | | | | | | |

| **Logarithmic Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 11.3 | 10.1 | 7.3 |
| **90% CI, Upper Boundary** | 3,792 | 3,404 | 2,448 | | 14.4 | 12.9 | 9.3 |
| **80% CI, Upper Boundary** | 3,792 | 3,404 | 2,448 | | 15.2 | 13.7 | 9.8 |
| **IQR Upper** | 3,792 | 3,404 | 2,448 | | 16.9 | 15.1 | 10.9 |
| **Median** | 3,792 | 3,404 | 2,448 | | 18.9 | 17.0 | 12.2 |
| **Arithmetic Mean** | 3,792 | 3,404 | 2,448 | | 18.7 | 16.8 | 12.1 |
| **Geometric Mean** | 3,792 | 3,404 | 2,448 | | 19.0 | 17.0 | 12.2 |
| **IQR Lower** | 3,792 | 3,404 | 2,448 | | | 19.1 | 13.7 |
| **80% CI, Lower Boundary** | 3,792 | 3,404 | 2,448 | | | | 15.4 |
| **90% CI, Lower Boundary** | 3,792 | 3,404 | 2,448 | | | | 16.5 |
| **Minimum** | 3,792 | 3,404 | 2,448 | | | | |

**Table 67: Redosing regimens of rivaroxaban in response to various target AUC values in subjects with moderate renal impairment where the recommended dose is 15 mg rivaroxaban, no concomitant verapamil, using the logarithmic model.**

| **Moderate Renal Impairment - 15 mg Recommended R, 0 V** | | | | | | | |
|---|---|---|---|---|---|---|---|
| | | | | | | | |

| **Logarithmic Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | | 13.7 | 9.8 |
| **90% CI, Upper Boundary** | 3,792 | 3,404 | 2,448 | | | | 12.8 |
| **80% CI, Upper Boundary** | 3,792 | 3,404 | 2,448 | | | | 13.8 |
| **IQR Upper** | 3,792 | 3,404 | 2,448 | | | | |
| **Median** | 3,792 | 3,404 | 2,448 | | | | |
| **Arithmetic Mean** | 3,792 | 3,404 | 2,448 | | | | |
| **Geometric Mean** | 3,792 | 3,404 | 2,448 | | | | |
| **IQR Lower** | 3,792 | 3,404 | 2,448 | | | | |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 68: Redosing regimens of rivaroxaban in response to various target AUC values in subjects with moderate renal impairment where the recommended dose is 15 mg rivaroxaban, 120 mg concomitant verapamil, using the logarithmic model.**

| **Moderate Renal Impairment - 15 mg Recommended R, 120 V** | | | | | | | |
|---|---|---|---|---|---|---|---|
| | | | | | | | |

| **Logarithmic Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 11.0 | 9.9 | 7.1 |
| **90% CI, Upper Boundary** | 3,792 | 3,404 | 2,448 | | 14.6 | 13.1 | 9.4 |
| **80% CI, Upper Boundary** | 3,792 | 3,404 | 2,448 | | | 14.1 | 10.1 |
| **IQR Upper** | 3,792 | 3,404 | 2,448 | | | | 11.3 |
| **Median** | 3,792 | 3,404 | 2,448 | | | | 12.6 |
| **Arithmetic Mean** | 3,792 | 3,404 | 2,448 | | | | 12.5 |
| **Geometric Mean** | 3,792 | 3,404 | 2,448 | | | | 12.7 |
| **IQR Lower** | 3,792 | 3,404 | 2,448 | | | | 14.2 |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 69: Redosing regimens of rivaroxaban in response to various target AUC values in subjects with moderate renal impairment where the recommended dose is 15 mg rivaroxaban, 240 mg concomitant verapamil, using the logarithmic model.**

| **Moderate Renal Impairment - 15 mg Recommended R, 240 V** | | | | | | | |
|---|---|---|---|---|---|---|---|
| | | | | | | | |

| **Logarithmic Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 10.3 | 9.3 | 6.7 |
| **90% CI, Upper Boundary** | 3,792 | 3,404 | 2,448 | | 13.7 | 12.3 | 8.9 |
| **80% CI, Upper Boundary** | 3,792 | 3,404 | 2,448 | | 14.8 | 13.3 | 9.6 |
| **IQR Upper** | 3,792 | 3,404 | 2,448 | | | 14.8 | 10.6 |
| **Median** | 3,792 | 3,404 | 2,448 | | | | 11.9 |
| **Arithmetic Mean** | 3,792 | 3,404 | 2,448 | | | | 11.8 |
| **Geometric Mean** | 3,792 | 3,404 | 2,448 | | | | 12.0 |
| **IQR Lower** | 3,792 | 3,404 | 2,448 | | | | 13.5 |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 70: Redosing regimens of rivaroxaban in response to various target AUC values in subjects with moderate renal impairment where the recommended dose is 15 mg rivaroxaban, 360 mg concomitant verapamil, using the logarithmic model.**

| **Moderate Renal Impairment - 15 mg Recommended R, 360 V** | | | | | | | |
|---|---|---|---|---|---|---|---|
| | | | | | | | |

| **Logarithmic Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 9.9 | 8.9 | 6.4 |
| **90% CI, Upper Boundary** | 3,792 | 3,404 | 2,448 | | 13.2 | 11.9 | 8.5 |
| **80% CI, Upper Boundary** | 3,792 | 3,404 | 2,448 | | 14.2 | 12.8 | 9.2 |
| **IQR Upper** | 3,792 | 3,404 | 2,448 | | | 14.3 | 10.3 |
| **Median** | 3,792 | 3,404 | 2,448 | | | | 11.5 |
| **Arithmetic Mean** | 3,792 | 3,404 | 2,448 | | | | 11.4 |
| **Geometric Mean** | 3,792 | 3,404 | 2,448 | | | | 11.5 |
| **IQR Lower** | 3,792 | 3,404 | 2,448 | | | | 13.0 |
| **80% CI, Lower Boundary** | 3,792 | 3,404 | 2,448 | | | | 14.5 |
| **90% CI, Lower Boundary** | 3,792 | 3,404 | 2,448 | | | | |
| **Minimum** | 3,792 | 3,404 | 2,448 | | | | |

**Table 71: Redosing regimens of rivaroxaban in response to various target AUC values in subjects with moderate renal impairment where the recommended dose is 20 mg rivaroxaban, no concomitant verapamil, using the logarithmic model.**

| **Moderate Renal Impairment - 20 mg Recommended R, 0 V** | | | | | | | |
|---|---|---|---|---|---|---|---|
| | | | | | | | |

| **Logarithmic Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 15.2 | 13.7 | 9.8 |
| **90% CI, Upper Boundary** | 3,792 | 3,404 | 2,448 | | 19.8 | 17.7 | 12.8 |
| **80% CI, Upper Boundary** | 3,792 | 3,404 | 2,448 | | | 19.1 | 13.8 |
| **IQR Upper** | 3,792 | 3,404 | 2,448 | | | | 15.2 |
| **Median** | 3,792 | 3,404 | 2,448 | | | | 17.2 |
| **Arithmetic Mean** | 3,792 | 3,404 | 2,448 | | | | 17.0 |
| **Geometric Mean** | 3,792 | 3,404 | 2,448 | | | | 17.3 |
| **IQR Lower** | 3,792 | 3,404 | 2,448 | | | | 19.6 |
| **80% CI, Lower Boundary** | 3,792 | 3,404 | 2,448 | | | | |
| **90% CI, Lower Boundary** | 3,792 | 3,404 | 2,448 | | | | |
| **Minimum** | 3,792 | 3,404 | 2,448 | | | | |

**Table 72: Redosing regimens of rivaroxaban in response to various target AUC values in subjects with moderate renal impairment where the recommended dose is 20 mg rivaroxaban, 120 mg concomitant verapamil, using the logarithmic model.**

| **Moderate Renal Impairment - 20 mg Recommended R, 120 V** | | | | | | | |
|---|---|---|---|---|---|---|---|
| | | | | | | | |

| **Logarithmic Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 11.0 | 9.9 | 7.1 |
| **90% CI, Upper Boundary** | 3,792 | 3,404 | 2,448 | | 14.6 | 13.1 | 9.4 |
| **80% CI, Upper Boundary** | 3,792 | 3,404 | 2,448 | | 15.7 | 14.1 | 10.1 |
| **IQR Upper** | 3,792 | 3,404 | 2,448 | | 17.5 | 15.7 | 11.3 |
| **Median** | 3,792 | 3,404 | 2,448 | | 19.5 | 17.5 | 12.6 |
| **Arithmetic Mean** | 3,792 | 3,404 | 2,448 | | 19.3 | 17.3 | 12.5 |
| **Geometric Mean** | 3,792 | 3,404 | 2,448 | | 19.6 | 17.6 | 12.7 |
| **IQR Lower** | 3,792 | 3,404 | 2,448 | | | 19.8 | 14.2 |
| **80% CI, Lower Boundary** | 3,792 | 3,404 | 2,448 | | | | 15.8 |
| **90% CI, Lower Boundary** | 3,792 | 3,404 | 2,448 | | | | 16.9 |
| **Minimum** | 3,792 | 3,404 | 2,448 | | | | |

**Table [73: Redosing regimens of rivaroxaban in response to various target AUC values in subjects with moderate renal impairment where the recommended dose is 20 mg rivaroxaban, 240 mg concomitant verapamil, using the logarithmic model.**

| **Moderate Renal Impairment - 20 mg Recommended R, 240 V** | | | | | | | |
|---|---|---|---|---|---|---|---|
| | | | | | | | |

| **Logarithmic Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 10.3 | 9.3 | 6.7 |
| **90% CI, Upper Boundary** | 3,792 | 3,404 | 2,448 | | 13.7 | 12.3 | 8.9 |
| **80% CI, Upper Boundary** | 3,792 | 3,404 | 2,448 | | 14.8 | 13.3 | 9.6 |
| **IQR Upper** | 3,792 | 3,404 | 2,448 | | 16.5 | 14.8 | 10.6 |
| **Median** | 3,792 | 3,404 | 2,448 | | 18.5 | 16.6 | 11.9 |
| **Arithmetic Mean** | 3,792 | 3,404 | 2,448 | | 18.2 | 16.4 | 11.8 |
| **Geometric Mean** | 3,792 | 3,404 | 2,448 | | 18.5 | 16.6 | 12.0 |
| **IQR Lower** | 3,792 | 3,404 | 2,448 | | | 18.7 | 13.5 |
| **80% CI, Lower Boundary** | 3,792 | 3,404 | 2,448 | | | | 15.0 |
| **90% CI, Lower Boundary** | 3,792 | 3,404 | 2,448 | | | | 16.0 |
| **Minimum** | 3,792 | 3,404 | 2,448 | | | | |

**Table 74 Redosing regimens of rivaroxaban in response to various target AUC values in subjects with moderate renal impairment where the recommended dose is 20 mg rivaroxaban, 360 mg concomitant verapamil, using the logarithmic model.**

| **Moderate Renal Impairment - 20 mg Recommended R, 360 V** | | | | | | | |
|---|---|---|---|---|---|---|---|
| | | | | | | | |

| **Logarithmic Redosing AUC** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 3,792 | 3,404 | 2,448 | | 9.9 | 8.9 | 6.4 |
| **90% CI, Upper Boundary** | 3,792 | 3,404 | 2,448 | | 13.2 | 11.9 | 8.5 |
| **80% CI, Upper Boundary** | 3,792 | 3,404 | 2,448 | | 14.2 | 12.8 | 9.2 |
| **IQR Upper** | 3,792 | 3,404 | 2,448 | | 15.9 | 14.3 | 10.3 |
| **Median** | 3,792 | 3,404 | 2,448 | | 17.8 | 16.0 | 11.5 |
| **Arithmetic Mean** | 3,792 | 3,404 | 2,448 | | 17.6 | 15.8 | 11.4 |
| **Geometric Mean** | 3,792 | 3,404 | 2,448 | | 17.9 | 16.1 | 11.5 |
| **IQR Lower** | 3,792 | 3,404 | 2,448 | | | 18.1 | 13.0 |
| **80% CI, Lower Boundary** | 3,792 | 3,404 | 2,448 | | | | 14.5 |
| **90% CI, Lower Boundary** | 3,792 | 3,404 | 2,448 | | | | 15.5 |
| **Minimum** | 3,792 | 3,404 | 2,448 | | | | |

**Table 75: Redosing regimens of rivaroxaban in response to various target steady state Cₘₐₓ values in subjects with no renal impairment where the recommended dose is 15 mg rivaroxaban, no concomitant verapamil, using the exponential model.**

| **No Renal Impairment** - **15 Recommended R, 0 V** | | | | | | | |
|---|---|---|---|---|---|---|---|
| | | | | | | | |

| **Exponential Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 11.8 | 11.4 | |
| **90% CI, Upper Boundary** | 275 | 268 | 317 | | | | |
| **80% CI, Upper Boundary** | 275 | 268 | 317 | | | | |
| IQR **Upper** | 275 | 268 | 317 | | | | |
| **Median** | 275 | 268 | 317 | | | | |
| **Arithmetic Mean** | 275 | 268 | 317 | | | | |
| **Geometric Mean** | 275 | 268 | 317 | | | | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 76: Redosing regimens of rivaroxaban in response to various target steady state steady state Cₘₐₓ values in subjects with no renal impairment where the recommended dose is 15 mg rivaroxaban, 120 mg concomitant verapamil, using the exponential model.**

| **No Renal Impairment - 15 mg Recommended R, 120 V** | | | | | | | |
|---|---|---|---|---|---|---|---|
| | | | | | | | |

| **Exponential Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 9.8 | 9.5 | 12.1 |
| **90% CI, Upper Boundary** | 275 | 268 | 317 | | 15.0 | 14.3 | |
| **80% CI, Upper Boundary** | 275 | 268 | 317 | | | | |
| IQR **Upper** | 275 | 268 | 317 | | | | |
| **Median** | 275 | 268 | 317 | | | | |
| **Arithmetic Mean** | 275 | 268 | 317 | | | | |
| **Geometric Mean** | 275 | 268 | 317 | | | | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 77: Redosing regimens of rivaroxaban in response to various target steady state steady state Cₘₐₓ values in subjects with no renal impairment where the recommended dose is 15 mg rivaroxaban, 240 mg concomitant verapamil, using the exponential model.**

| **No Renal Impairment - 15 mg Recommended R, 240 V** | | | | | | | |
|---|---|---|---|---|---|---|---|
| | | | | | | | |

| **Exponential Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 9.3 | 9.0 | 11.4 |
| **90% CI, Upper Boundary** | 275 | 268 | 317 | | 14.4 | 13.7 | |
| **80% CI, Upper Boundary** | 275 | 268 | 317 | | | | |
| **IQR Upper** | 275 | 268 | 317 | | | | |
| **Median** | 275 | 268 | 317 | | | | |
| **Arithmetic Mean** | 275 | 268 | 317 | | | | |
| **Geometric Mean** | 275 | 268 | 317 | | | | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 78: Redosing regimens of rivaroxaban in response to various target steady state steady state Cₘₐₓ values in subjects with no renal impairment where the recommended dose is 15 mg rivaroxaban, 360 mg concomitant verapamil, using the exponential model.**

| **No Renal Impairment - 15 mg Recommended R, 360 V** | | | | | | | |
|---|---|---|---|---|---|---|---|
| | | | | | | | |

| **Exponential Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 9.0 | 8.7 | 11.0 |
| **90% CI, Upper Boundary** | 275 | 268 | 317 | | 13.8 | 13.2 | |
| **80% CI, Upper Boundary** | 275 | 268 | 317 | | | 15.0 | |
| **IQR Upper** | 275 | 268 | 317 | | | | |
| **Median** | 275 | 268 | 317 | | | | |
| **Arithmetic Mean** | 275 | 268 | 317 | | | | |
| **Geometric Mean** | 275 | 268 | 317 | | | | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 79: Redosing regimens of rivaroxaban in response to various target steady state steady state Cₘₐₓ values in subjects with no renal impairment where the recommended dose is 20 mg rivaroxaban, no concomitant verapamil, using the exponential model.**

| **No Renal Impairment - 20 mg Recommended R, 0 V** | | | | | | | |
|---|---|---|---|---|---|---|---|
| | | | | | | | |

| **Exponential Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 7.8 | 7.6 | 9.4 |
| **90% CI, Upper Boundary** | 275 | 268 | 317 | | 11.6 | 11.2 | 14.8 |
| **80% CI, Upper Boundary** | 275 | 268 | 317 | | 13.1 | 12.6 | 17.0 |
| **IQR Upper** | 275 | 268 | 317 | | 15.9 | 15.2 | |
| **Median** | 275 | 268 | 317 | | | 19.2 | |
| **Arithmetic Mean** | 275 | 268 | 317 | | 19.3 | 18.3 | |
| **Geometric Mean** | 275 | 268 | 317 | | 19.9 | 18.9 | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 80: Redosing regimens of rivaroxaban in response to various target steady state steady state Cₘₐₓ values in subjects with no renal impairment where the recommended dose is 20 mg rivaroxaban, 120 mg concomitant verapamil, using the exponential model.**

| **No Renal Impairment - 20 mg Recommended R, 120 V** | | | | | | | |
|---|---|---|---|---|---|---|---|
| | | | | | | | |

| **Exponential Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 6.8 | 6.7 | 8.0 |
| **90% CI, Upper Boundary** | 275 | 268 | 317 | | 9.3 | 9.0 | 11.4 |
| **80% CI, Upper Boundary** | 275 | 268 | 317 | | 10.4 | 10.0 | 12.9 |
| **IQR Upper** | 275 | 268 | 317 | | 12.3 | 11.8 | 15.8 |
| **Median** | 275 | 268 | 317 | | 14.9 | 14.2 | 19.6 |
| **Arithmetic Mean** | 275 | 268 | 317 | | 14.4 | 13.8 | 18.8 |
| **Geometric Mean** | 275 | 268 | 317 | | 14.8 | 14.1 | 19.4 |
| **IQR Lower** | 275 | 268 | 317 | | 18.4 | 17.5 | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 81: Redosing regimens of rivaroxaban in response to various target steady state steady state Cₘₐₓ values in subjects with no renal impairment where the recommended dose is 20 mg rivaroxaban, 240 mg concomitant verapamil, using the exponential model.**

| **No Renal Impairment - 20 mg Recommended R, 240 V** | | | | | | | |
|---|---|---|---|---|---|---|---|
| | | | | | | | |

| **Exponential Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 6.6 | 6.4 | 7.6 |
| **90% CI, Upper Boundary** | 275 | 268 | 317 | | 9.0 | 8.8 | 11.0 |
| **80% CI, Upper Boundary** | 275 | 268 | 317 | | 9.9 | 9.6 | 12.3 |
| **IQR Upper** | 275 | 268 | 317 | | 11.7 | 11.3 | 14.9 |
| **Median** | 275 | 268 | 317 | | 14.1 | 13.5 | 18.4 |
| **Arithmetic Mean** | 275 | 268 | 317 | | 13.6 | 13.1 | 17.7 |
| **Geometric Mean** | 275 | 268 | 317 | | 14.0 | 13.4 | 18.2 |
| **IQR Lower** | 275 | 268 | 317 | | 17.3 | 16.5 | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 82: Redosing regimens of rivaroxaban in response to various target steady state Cₘₐₓ values in subjects with no renal impairment where the recommended dose is 20 mg rivaroxaban, 360 mg concomitant verapamil, using the exponential model.**

| **No Renal Impairment - 20 mg Recommended R, 360 V** | | | | | | | |
|---|---|---|---|---|---|---|---|
| | | | | | | | |

| **Exponential Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| Maximum | 275 | 268 | 317 | | 6.4 | 6.3 | 7.4 |
| **90% CI, Upper Boundary** | 275 | 268 | 317 | | 8.8 | 8.5 | 10.7 |
| **80% CI, Upper Boundary** | 275 | 268 | 317 | | 9.7 | 9.3 | 11.9 |
| **IQR Upper** | 275 | 268 | 317 | | 11.4 | 10.9 | 14.3 |
| **Median** | 275 | 268 | 317 | | 13.7 | 13.1 | 17.7 |
| **Arithmetic Mean** | 275 | 268 | 317 | | 13.2 | 12.6 | 17.0 |
| **Geometric Mean** | 275 | 268 | 317 | | 13.5 | 12.9 | 17.5 |
| **IQR Lower** | 275 | 268 | 317 | | 16.6 | 15.9 | |
| **80% CI, Lower Boundary** | 275 | 268 | 317 | | | 19.1 | |
| **90% CI, Lower Boundary** | 275 | 268 | 317 | | | | |
| **Minimum** | 275 | 268 | 317 | | | | |

**Table 83: Redosing regimens of rivaroxaban in response to various target steady state Cₘₐₓ values in subjects with mild renal impairment where the recommended dose is 15 mg rivaroxaban, no concomitant verapamil, using the exponential model.**

| **Mild Renal Impairment - 15 mg Recommended R, 0 V** | | | | | | | |
|---|---|---|---|---|---|---|---|
| | | | | | | | |
| **Exponential Redosing Cₘₐₓ** | | | | | | | |
| | | | | | | | |
| **Group** | **Target 1** | **Target 2** | **Target 3** | | **Dose 1** | **Dose 2** | **Dose 3** |
| **Maximum** | 275 | 268 | 317 | | 10.7 | 10.3 | 13.4 |
| **90% CI, Upper Boundary** | 275 | 268 | 317 | | | | |
| **80% CI, Upper Boundary** | 275 | 268 | 317 | | | | |
| **IQR Upper** | 275 | 268 | 317 | | | | |
| **Median** | 275 | 268 | 317 | | | | |
| **Arithmetic Mean** | 275 | 268 | 317 | | | | |
| **Geometric Mean** | 275 | 268 | 317 | | | | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 84: Redosing regimens of rivaroxaban in response to various target steady state Cₘₐₓ values in subjects with mild renal impairment where the recommended dose is 15 mg rivaroxaban, 120 mg concomitant verapamil, using the exponential model.**

| **Mild Renal Impairment - 15 mg Recommended R, 120 V** | | | | | | | |
|---|---|---|---|---|---|---|---|
| | | | | | | | |
| **Exponential R edosing C ₘₐₓ** | | | | | | | |
| | | | | | | | |
| **Group** | **Target 1** | **Target 2** | **Target 3** | | **Dose 1** | **Dose 2** | **Dose 3** |
| **Maximum** | 275 | 268 | 317 | | 9.2 | 8.9 | 11.2 |
| **90% CI, Upper Boundary** | 275 | 268 | 317 | | 14.4 | 13.8 | |
| **80% CI, Upper Boundary** | 275 | 268 | 317 | | | | |
| **IQR Upper** | 275 | 268 | 317 | | | | |
| **Median** | 275 | 268 | 317 | | | | |
| **Arithmetic Mean** | 275 | 268 | 317 | | | | |
| **Geometric Mean** | 275 | 268 | 317 | | | | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 85: Redosing regimens of rivaroxaban in response to various target steady state Cₘₐₓ values in subjects with mild renal impairment where the recommended dose is 15 mg rivaroxaban, 240 mg concomitant verapamil, using the exponential model.**

| **Mild Renal Impairment - 15 mg Recommended R, 240 V** | | | | | | | |
|---|---|---|---|---|---|---|---|
| | | | | | | | |
| **Expo nential R edosing C ₘₐₓ** | | | | | | | |
| | | | | | | | |
| **Group** | **Target 1** | **Target 2** | **Target 3** | | **Dose 1** | **Dose 2** | **Dose 3** |
| **Maximum** | 275 | 268 | 317 | | 8.9 | 8.6 | 10.8 |
| **90% CI, Upper Boundary** | 275 | 268 | 317 | | 13.8 | 13.2 | |
| **80% CI, Upper Boundary** | 275 | 268 | 317 | | | 14.5 | |
| **IQR Upper** | 275 | 268 | 317 | | | | |
| **Median** | 275 | 268 | 317 | | | | |
| **Arithmetic Mean** | 275 | 268 | 317 | | | | |
| **Geometric Mean** | 275 | 268 | 317 | | | | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 86: Redosing regimens of rivaroxaban in response to various target steady state Cₘₐₓ values in subjects with mild renal impairment where the recommended dose is 15 mg rivaroxaban, 360 mg concomitant verapamil, using the exponential model.**

| **Mild Renal Impairment - 15 mg Recommended R, 360 V** | | | | | | | |
|---|---|---|---|---|---|---|---|
| | | | | | | | |
| **Exponential R edosing C ₘₐₓ** | | | | | | | |
| | | | | | | | |
| **Group** | **Target 1** | **Target 2** | **Target 3** | | **Dose 1** | **Dose 2** | **Dose 3** |
| **Maximum** | 275 | 268 | 317 | | 8.7 | 8.5 | 10.6 |
| **90% CI, Upper Boundary** | 275 | 268 | 317 | | 13.3 | 12.7 | |
| **80% CI, Upper Boundary** | 275 | 268 | 317 | | 14.7 | 14.0 | |
| **IQR Upper** | 275 | 268 | 317 | | | | |
| **Median** | 275 | 268 | 317 | | | | |
| **Arithmetic Mean** | 275 | 268 | 317 | | | | |
| **Geometric Mean** | 275 | 268 | 317 | | | | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 87: Redosing regimens of rivaroxaban in response to various target steady state Cₘₐₓ values in subjects with mild renal impairment where the recommended dose is 20 mg rivaroxaban, no concomitant verapamil, using the exponential model.**

| **Mild Renal Impairment - 20 mg Recommended R, 0 V** | | | | | | | |
|---|---|---|---|---|---|---|---|
| | | | | | | | |
| **Expo nential R edosing Cₘₐₓ** | | | | | | | |
| | | | | | | | |
| **Group** | **Target 1** | **Target 2** | **Target 3** | | **Dose 1** | **Dose 2** | **Dose 3** |
| **Maximum** | 275 | 268 | 317 | | 7.3 | 7.1 | 8.6 |
| **90% CI, Upper Boundary** | 275 | 268 | 317 | | 11.4 | 10.9 | 14.4 |
| **80% CI, Upper Boundary** | 275 | 268 | 317 | | 12.5 | 12.0 | 16.1 |
| **IQR Upper** | 275 | 268 | 317 | | 15.1 | 14.4 | 19.9 |
| **Median** | 275 | 268 | 317 | | 19.1 | 18.1 | |
| **Arithmetic Mean** | 275 | 268 | 317 | | 18.3 | 17.4 | |
| **Geometric Mean** | 275 | 268 | 317 | | 18.9 | 18.0 | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 88: Redosing regimens of rivaroxaban in response to various target steady state Cₘₐₓ values in subjects with mild renal impairment where the recommended dose is 20 mg rivaroxaban, 120 mg concomitant verapamil, using the exponential model.**

| **Mild Renal Impairment - 20 mg Recommended R, 120 V** | | | | | | | |
|---|---|---|---|---|---|---|---|
| | | | | | | | |
| **Expo nential Redosing C ₘₐₓ** | | | | | | | |
| | | | | | | | |
| **Group** | **Target 1** | **Target 2** | **Target 3** | | **Dose 1** | **Dose 2** | **Dose 3** |
| Maximum | 275 | 268 | 317 | | 6.5 | 6.3 | 7.5 |
| **90% CI, Upper Boundary** | 275 | 268 | 317 | | 9.1 | 8.8 | 11.1 |
| **80% CI, Upper Boundary** | 275 | 268 | 317 | | 9.8 | 9.5 | 12.2 |
| **IQR Upper** | 275 | 268 | 317 | | 11.5 | 11.1 | 14.6 |
| **Median** | 275 | 268 | 317 | | 14.1 | 13.4 | 18.3 |
| **Arithmetic Mean** | 275 | 268 | 317 | | 13.6 | 13.0 | 17.6 |
| **Geometric Mean** | 275 | 268 | 317 | | 13.9 | 13.3 | 18.2 |
| **IQR Lower** | 275 | 268 | 317 | | 17.4 | 16.6 | |
| **80% CI, Lower Boundary** | 275 | 268 | 317 | | | 19.9 | |
| **90% CI, Lower Boundary** | 275 | 268 | 317 | | | | |
| **Minimum** | 275 | 268 | 317 | | | | |

**Table 89: Redosing regimens of rivaroxaban in response to various target steady state Cₘₐₓ values in subjects with mild renal impairment where the recommended dose is 20 mg rivaroxaban, 240 mg concomitant verapamil, using the exponential model.**

| **Mild Renal Impairment - 20 mg Recommended R, 240 V** | | | | | | | |
|---|---|---|---|---|---|---|---|
| | | | | | | | |
| **Expo nential R edosing C ₘₐₓ** | | | | | | | |
| | | | | | | | |
| **Group** | **Target 1** | **Target 2** | **Target 3** | | **Dose 1** | **Dose 2** | **Dose 3** |
| **Maximum** | 275 | 268 | 317 | | 6.4 | 6.2 | 7.3 |
| **90% CI, Upper Boundary** | 275 | 268 | 317 | | 8.8 | 8.5 | 10.6 |
| **80% CI, Upper Boundary** | 275 | 268 | 317 | | 9.4 | 9.1 | 11.6 |
| **IQR Upper** | 275 | 268 | 317 | | 11.0 | 10.6 | 13.8 |
| **Median** | 275 | 268 | 317 | | 13.3 | 12.8 | 17.2 |
| **Arithmetic Mean** | 275 | 268 | 317 | | 12.9 | 12.4 | 16.6 |
| **Geometric Mean** | 275 | 268 | 317 | | 13.2 | 12.7 | 17.1 |
| **IQR Lower** | 275 | 268 | 317 | | 16.4 | 15.6 | |
| **80% CI, Lower Boundary** | 275 | 268 | 317 | | 19.8 | 18.8 | |
| **90% CI, Lower Boundary** | 275 | 268 | 317 | | | | |
| **Minimum** | 275 | 268 | 317 | | | | |

**Table 90: Redosing regimens of rivaroxaban in response to various target steady state Cₘₐₓ values in subjects with mild renal impairment where the recommended dose is 20 mg rivaroxaban, 240 mg concomitant verapamil, using the exponential model.**

| **Mild Renal Impairment - 20 mg Recommended R, 360 V** | | | | | | | |
|---|---|---|---|---|---|---|---|
| | | | | | | | |
| **Expo nential Redosing C ₘₐₓ** | | | | | | | |
| | | | | | | | |
| **Group** | **Target 1** | **Target 2** | **Target 3** | | **Dose 1** | **Dose 2** | **Dose 3** |
| **Maximum** | 275 | 268 | 317 | | 6.3 | 6.1 | 7.2 |
| **90% CI, Upper Boundary** | 275 | 268 | 317 | | 8.6 | 8.3 | 10.3 |
| **80% CI, Upper Boundary** | 275 | 268 | 317 | | 9.2 | 8.9 | 11.2 |
| **IQR Upper** | 275 | 268 | 317 | | 10.6 | 10.3 | 13.3 |
| **Median** | 275 | 268 | 317 | | 12.9 | 12.4 | 16.6 |
| **Arithmetic Mean** | 275 | 268 | 317 | | 12.5 | 12.0 | 16.0 |
| **Geometric Mean** | 275 | 268 | 317 | | 12.8 | 12.3 | 16.4 |
| **IQR Lower** | 275 | 268 | 317 | | 15.8 | 15.0 | |
| **80% CI, Lower Boundary** | 275 | 268 | 317 | | 18.9 | 18.0 | |
| **90% CI, Lower Boundary** | 275 | 268 | 317 | | | | |
| **Minimum** | 275 | 268 | 317 | | | | |

**Table 91: Redosing regimens of rivaroxaban in response to various target steady state Cₘₐₓ values in subjects with moderate renal impairment where the recommended dose is 15 mg rivaroxaban, no concomitant verapamil, using the exponential model.**

| **Moderate Renal Impairment** - **15 mg Recommended R, 0 V** | | | | | | | |
|---|---|---|---|---|---|---|---|
| | | | | | | | |

| **Expo nential Re edosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 10.0 | 9.7 | 12.4 |
| **90% CI, Upper Boundary** | 275 | 268 | 317 | | | | |
| **80% CI, Upper Boundary** | 275 | 268 | 317 | | | | |
| **IQR Upper** | 275 | 268 | 317 | | | | |
| **Median** | 275 | 268 | 317 | | | | |
| **Arithmetic Mean** | 275 | 268 | 317 | | | | |
| **Geometric Mean** | 275 | 268 | 317 | | | | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 92: Redosing regimens of rivaroxaban in response to various target steady state Cₘₐₓ values in subjects with moderate renal impairment where the recommended dose is 15 mg rivaroxaban, 120 mg concomitant verapamil, using the exponential model.**

| **Moderate R enal Impairment - 15 mg Rec ommended R, 120 V** | | | | | | | |
|---|---|---|---|---|---|---|---|
| | | | | | | | |

| **Expo nential Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 8.1 | 7.9 | 9.8 |
| **90% CI, Upper Boundary** | 275 | 268 | 317 | | 13.8 | 13.2 | |
| **80% CI, Upper Boundary** | 275 | 268 | 317 | | | 14.9 | |
| **IQR Upper** | 275 | 268 | 317 | | | | |
| **Median** | 275 | 268 | 317 | | | | |
| **Arithmetic Mean** | 275 | 268 | 317 | | | | |
| **Geometric Mean** | 275 | 268 | 317 | | | | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 93: Redosing regimens of rivaroxaban in response to various target steady state Cₘₐₓ values in subjects with moderate renal impairment where the recommended dose is 15 mg rivaroxaban, 240 mg concomitant verapamil, using the exponential model.**

| **Moderate R enal Impairment - 15 mg Recom mended R, 240 V** | | | | | | | |
|---|---|---|---|---|---|---|---|
| | | | | | | | |

| **Expo nential Redosing** Cₘₐₓ | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 7.8 | 7.6 | 9.3 |
| **90% CI, Upper Boundary** | 275 | 268 | 317 | | 13.2 | 12.6 | |
| **80% CI, Upper Boundary** | 275 | 268 | 317 | | 14.6 | 14.0 | |
| **IQR Upper** | 275 | 268 | 317 | | | | |
| **Median** | 275 | 268 | 317 | | | | |
| **Arithmetic Mean** | 275 | 268 | 317 | | | | |
| **Geometric Mean** | 275 | 268 | 317 | | | | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 94: Redosing regimens of rivaroxaban in response to various target steady state Cₘₐₓ values in subjects with moderate renal impairment where the recommended dose is 15 mg rivaroxaban, 360 mg concomitant verapamil, using the exponential model.**

| **Moderate R enal Impairment - 15 mg Recommended R, 360 V** | | | | | | | |
|---|---|---|---|---|---|---|---|
| | | | | | | | |

| **Expo nential R edosing C ₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| Maximum | 275 | 268 | 317 | | 7.6 | 7.4 | 9.1 |
| **90% CI, Upper Boundary** | 275 | 268 | 317 | | 12.7 | 12.2 | |
| **80% CI, Upper Boundary** | 275 | 268 | 317 | | 14.1 | 13.4 | |
| **IQR Upper** | 275 | 268 | 317 | | | | |
| **Median** | 275 | 268 | 317 | | | | |
| **Arithmetic Mean** | 275 | 268 | 317 | | | | |
| **Geometric Mean** | 275 | 268 | 317 | | | | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 95: Redosing regimens of rivaroxaban in response to various target steady state Cₘₐₓ values in subjects with moderate renal impairment where the recommended dose is 20 mg rivaroxaban, no concomitant verapamil, using the exponential model.**

| **Moderate Renal Imp airment - 20 mg Recommended R, 0 V** | | | | | | | |
|---|---|---|---|---|---|---|---|
| | | | | | | | |

| **Expo nential R edosing C ₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| Maximum | 275 | 268 | 317 | | 6.9 | 6.8 | 8.1 |
| **90% CI, Upper Boundary** | 275 | 268 | 317 | | 11.1 | 10.7 | 13.9 |
| **80% CI, Upper Boundary** | 275 | 268 | 317 | | 12.2 | 11.7 | 15.6 |
| **IQR Upper** | 275 | 268 | 317 | | 14.6 | 14.0 | 19.2 |
| **Median** | 275 | 268 | 317 | | 18.2 | 17.3 | |
| **Arithmetic Mean** | 275 | 268 | 317 | | 17.5 | 16.7 | |
| **Geometric Mean** | 275 | 268 | 317 | | 18.0 | 17.1 | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 96: Redosing regimens of rivaroxaban in response to various target steady state Cₘₐₓ values in subjects with moderate renal impairment where the recommended dose is 20 mg rivaroxaban, 120 mg concomitant verapamil, using the exponential model.**

| **Moderate R enal Impairment - 20 mg Recommended R, 120 V** | | | | | | | |
|---|---|---|---|---|---|---|---|
| | | | | | | | |

| **Expo nential R edosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| Maximum | 275 | 268 | 317 | | 6.0 | 5.8 | 6.8 |
| **90% CI, Upper Boundary** | 275 | 268 | 317 | | 8.8 | 8.5 | 10.7 |
| **80% CI, Upper Boundary** | 275 | 268 | 317 | | 9.6 | 9.3 | 11.8 |
| **IQR Upper** | 275 | 268 | 317 | | 11.1 | 10.7 | 13.9 |
| **Median** | 275 | 268 | 317 | | 13.3 | 12.8 | 17.2 |
| **Arithmetic Mean** | 275 | 268 | 317 | | 12.9 | 12.4 | 16.6 |
| **Geometric Mean** | 275 | 268 | 317 | | 13.2 | 12.7 | 17.1 |
| **IQR Lower** | 275 | 268 | 317 | | 16.0 | 15.2 | |
| **80% CI, Lower Boundary** | 275 | 268 | 317 | | 19.7 | 18.6 | |
| **90% CI, Lower Boundary** | 275 | 268 | 317 | | | | |
| **Minimum** | 275 | 268 | 317 | | | | |

**Table 97: Redosing regimens of rivaroxaban in response to various target steady state Cₘₐₓ values in subjects with moderate renal impairment where the recommended dose is 20 mg rivaroxaban, 240 mg concomitant verapamil, using the exponential model.**

| **Moderate R enal Impairment - 20 mg Re commended R, 240 V** | | | | | | | |
|---|---|---|---|---|---|---|---|
| | | | | | | | |

| **Expo nential R edosing C ₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| Maximum | 275 | 268 | 317 | | 5.8 | 5.7 | 6.6 |
| **90% CI, Upper Boundary** | 275 | 268 | 317 | | 8.5 | 8.2 | 10.2 |
| **80% CI, Upper Boundary** | 275 | 268 | 317 | | 9.2 | 8.9 | 11.2 |
| **IQR Upper** | 275 | 268 | 317 | | 10.6 | 10.2 | 13.2 |
| **Median** | 275 | 268 | 317 | | 12.7 | 12.1 | 16.2 |
| **Arithmetic Mean** | 275 | 268 | 317 | | 12.3 | 11.8 | 15.7 |
| **Geometric Mean** | 275 | 268 | 317 | | 12.6 | 12.1 | 16.1 |
| **IQR Lower** | 275 | 268 | 317 | | 15.1 | 14.4 | 19.9 |
| **80% CI, Lower Boundary** | 275 | 268 | 317 | | 18.4 | 17.4 | |
| **90% CI, Lower Boundary** | 275 | 268 | 317 | | | 19.7 | |
| **Minimum** | 275 | 268 | 317 | | | | |

**Table 98: Redosing regimens of rivaroxaban in response to various target steady state Cₘₐₓ values in subjects with moderate renal impairment where the recommended dose is 20 mg rivaroxaban, 360 mg concomitant verapamil, using the exponential model.**

| **Moderate R enal Impairment - 20 mg Recom mended R, 360 V** | | | | | | | |
|---|---|---|---|---|---|---|---|
| | | | | | | | |

| **Expo nential Redosing C ₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| Maximum | 275 | 268 | 317 | | 5.7 | 5.6 | 6.4 |
| **90% CI, Upper Boundary** | 275 | 268 | 317 | | 8.3 | 8.0 | 9.9 |
| **80% CI, Upper Boundary** | 275 | 268 | 317 | | 8.9 | 8.6 | 10.8 |
| **IQR Upper** | 275 | 268 | 317 | | 10.3 | 9.9 | 12.8 |
| **Median** | 275 | 268 | 317 | | 12.3 | 11.8 | 15.7 |
| **Arithmetic Mean** | 275 | 268 | 317 | | 11.9 | 11.4 | 15.1 |
| **Geometric Mean** | 275 | 268 | 317 | | 12.2 | 11.7 | 15.5 |
| **IQR Lower** | 275 | 268 | 317 | | 14.6 | 13.9 | 19.1 |
| **80% CI, Lower Boundary** | 275 | 268 | 317 | | 17.7 | 16.8 | |
| **90% CI, Lower Boundary** | 275 | 268 | 317 | | 19.9 | 18.9 | |
| **Minimum** | 275 | 268 | 317 | | | | |

**Table 99: Redosing regimens of rivaroxaban in response to various target steady state Cₘₐₓ values in subjects with no renal impairment where the recommended dose is 15 mg rivaroxaban, no concomitant verapamil, using the logarithmic model.**

| **No Renal Impairment** - **15 mg Recommended R, 0 V** | | | | | | | |
|---|---|---|---|---|---|---|---|
| | | | | | | | |

| **Logarithmic Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 11.8 | 11.4 | |
| **90% CI, Upper Boundary** | 275 | 268 | 317 | | | | |
| **80% CI, Upper Boundary** | 275 | 268 | 317 | | | | |
| **IQR Upper** | 275 | 268 | 317 | | | | |
| **Median** | 275 | 268 | 317 | | | | |
| **Arithmetic Mean** | 275 | 268 | 317 | | | | |
| **Geometric Mean** | 275 | 268 | 317 | | | | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 100: Redosing regimens of rivaroxaban in response to various target steady state Cₘₐₓ values in subjects with no renal impairment where the recommended dose is 15 mg rivaroxaban, 120 mg concomitant verapamil, using the logarithmic model.**

| **No Renal Impairment - 15 mg Recommended R, 120 V** | | | | | | | |
|---|---|---|---|---|---|---|---|
| | | | | | | | |

| **Logarithmic Redosing Cmax** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 9.8 | 9.5 | 12.1 |
| **90% CI, Upper Boundary** | 275 | 268 | 317 | | 15.0 | 14.3 | |
| **80% CI, Upper Boundary** | 275 | 268 | 317 | | | | |
| **IQR Upper** | 275 | 268 | 317 | | | | |
| **Median** | 275 | 268 | 317 | | | | |
| **Arithmetic Mean** | 275 | 268 | 317 | | | | |
| **Geometric Mean** | 275 | 268 | 317 | | | | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 101: Redosing regimens of rivaroxaban in response to various target steady state Cₘₐₓ values in subjects with no renal impairment where the recommended dose is 15 mg rivaroxaban, 240 mg concomitant verapamil, using the logarithmic model.**

| **No Renal Impairment - 15 mg Recommended R, 240 V** | | | | | | | |
|---|---|---|---|---|---|---|---|
| | | | | | | | |

| **Logarithmic Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 9.3 | 9.0 | 11.4 |
| **90% CI, Upper Boundary** | 275 | 268 | 317 | | 14.4 | 13.7 | |
| **80% CI, Upper Boundary** | 275 | 268 | 317 | | | | |
| **IQR Upper** | 275 | 268 | 317 | | | | |
| **Median** | 275 | 268 | 317 | | | | |
| **Arithmetic Mean** | 275 | 268 | 317 | | | | |
| **Geometric Mean** | 275 | 268 | 317 | | | | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 102: Redosing regimens of rivaroxaban in response to various target steady state Cₘₐₓ values in subjects with no renal impairment where the recommended dose is 15 mg rivaroxaban, 360 mg concomitant verapamil, using the logarithmic model.**

| **No Renal Impairment - 15 mg Recommended R, 360 V** | | | | | | | |
|---|---|---|---|---|---|---|---|
| | | | | | | | |

| **Logarithmic Redosing Cₘₐₓ.** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 9.0 | 8.7 | 11.0 |
| **90% CI, Upper Boundary** | 275 | 268 | 317 | | 13.8 | 13.2 | |
| **80% CI, Upper Boundary** | 275 | 268 | 317 | | | 15.0 | |
| **IQR Upper** | 275 | 268 | 317 | | | | |
| **Median** | 275 | 268 | 317 | | | | |
| **Arithmetic Mean** | 275 | 268 | 317 | | | | |
| **Geometric Mean** | 275 | 268 | 317 | | | | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 103: Redosing regimens of rivaroxaban in response to various target steady state Cₘₐₓ values in subjects with no renal impairment where the recommended dose is 20 mg rivaroxaban, no concomitant verapamil, using the logarithmic model.**

| **No Renal Impairment - 20 mg Recommended R, 0 V** | | | | | | | |
|---|---|---|---|---|---|---|---|
| | | | | | | | |

| **Logarithmic Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 7.8 | 7.6 | 9.4 |
| **90% CI, Upper Boundary** | 275 | 268 | 317 | | 11.6 | 11.2 | 14.8 |
| **80% CI, Upper Boundary** | 275 | 268 | 317 | | 13.1 | 12.6 | 17.0 |
| **IQR Upper** | 275 | 268 | 317 | | 15.9 | 15.2 | |
| **Median** | 275 | 268 | 317 | | | 19.2 | |
| **Arithmetic Mean** | 275 | 268 | 317 | | 19.3 | 18.3 | |
| **Geometric Mean** | 275 | 268 | 317 | | 19.9 | 18.9 | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 104: Redosing regimens of rivaroxaban in response to various target steady state Cₘₐₓ values in subjects with no renal impairment where the recommended dose is 20 mg rivaroxaban, 120 mg concomitant verapamil, using the logarithmic model.**

| **No Renal Impairment - 20 mg Recommended R, 120 V** | | | | | | | |
|---|---|---|---|---|---|---|---|
| | | | | | | | |

| **Logarithmic Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 6.8 | 6.7 | 8.0 |
| **90% CI, Upper Boundary** | 275 | 268 | 317 | | 9.3 | 9.0 | 11.4 |
| **80% CI, Upper Boundary** | 275 | 268 | 317 | | 10.4 | 10.0 | 12.9 |
| **IQR Upper** | 275 | 268 | 317 | | 12.3 | 11.8 | 15.8 |
| **Median** | 275 | 268 | 317 | | 14.9 | 14.2 | 19.6 |
| **Arithmetic Mean** | 275 | 268 | 317 | | 14.4 | 13.8 | 18.8 |
| **Geometric Mean** | 275 | 268 | 317 | | 14.8 | 14.1 | 19.4 |
| **IQR Lower** | 275 | 268 | 317 | | 18.4 | 17.5 | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 105: Redosing regimens of rivaroxaban in response to various target steady state Cₘₐₓ values in subjects with no renal impairment where the recommended dose is 20 mg rivaroxaban, 240 mg concomitant verapamil, using the logarithmic model.**

| **No Renal Impairment - 20 mg Recommended R, 240 V** | | | | | | | |
|---|---|---|---|---|---|---|---|
| | | | | | | | |

| **Logarithmic Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 6.6 | 6.4 | 7.6 |
| **90% CI, Upper Boundary** | 275 | 268 | 317 | | 9.0 | 8.8 | 11.0 |
| **80% CI, Upper Boundary** | 275 | 268 | 317 | | 9.9 | 9.6 | 12.3 |
| **IQR Upper** | 275 | 268 | 317 | | 11.7 | 11.3 | 14.9 |
| **Median** | 275 | 268 | 317 | | 14.1 | 13.5 | 18.4 |
| **Arithmetic Mean** | 275 | 268 | 317 | | 13.6 | 13.1 | 17.7 |
| **Geometric Mean** | 275 | 268 | 317 | | 14.0 | 13.4 | 18.2 |
| **IQR Lower** | 275 | 268 | 317 | | 17.3 | 16.5 | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 106: Redosing regimens of rivaroxaban in response to various target steady state Cₘₐₓ values in subjects with no renal impairment where the recommended dose is 20 mg rivaroxaban, 360 mg concomitant verapamil, using the logarithmic model.**

| **No Renal Impairment - 20 mg Recommended R, 360 V** | | | | | | | |
|---|---|---|---|---|---|---|---|
| | | | | | | | |

| **Logarithmic Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 6.4 | 6.3 | 7.4 |
| **90% CI, Upper Boundary** | 275 | 268 | 317 | | 8.8 | 8.5 | 10.7 |
| **80% CI, Upper Boundary** | 275 | 268 | 317 | | 9.7 | 9.3 | 11.9 |
| **IQR Upper** | 275 | 268 | 317 | | 11.4 | 10.9 | 14.3 |
| **Median** | 275 | 268 | 317 | | 13.7 | 13.1 | 17.7 |
| **Arithmetic Mean** | 275 | 268 | 317 | | 13.2 | 12.6 | 17.0 |
| **Geometric Mean** | 275 | 268 | 317 | | 13.5 | 12.9 | 17.5 |
| **IQR Lower** | 275 | 268 | 317 | | 16.6 | 15.9 | |
| **80% CI, Lower Boundary** | 275 | 268 | 317 | | | 19.1 | |
| **90% CI, Lower Boundary** | 275 | 268 | 317 | | | | |
| **Minimum** | 275 | 268 | 317 | | | | |

**Table 107: Redosing regimens of rivaroxaban in response to various target steady state Cₘₐₓ values in subjects with mild renal impairment where the recommended dose is 15 mg rivaroxaban, no concomitant verapamil, using the logarithmic model.**

| **Mild Renal Impairment - 15 mg Recommended R, 0 V** | | | | | | | |
|---|---|---|---|---|---|---|---|
| | | | | | | | |

| **Logarithmic Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 10.7 | 10.3 | 13.4 |
| **90% CI, Upper Boundary** | 275 | 268 | 317 | | | | |
| **80% CI, Upper Boundary** | 275 | 268 | 317 | | | | |
| **IQR Upper** | 275 | 268 | 317 | | | | |
| **Median** | 275 | 268 | 317 | | | | |
| **Arithmetic Mean** | 275 | 268 | 317 | | | | |
| **Geometric Mean** | 275 | 268 | 317 | | | | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 108: Redosing regimens of rivaroxaban in response to various target steady state Cₘₐₓ values in subjects with mild renal impairment where the recommended dose is 15 mg rivaroxaban, 120 mg concomitant verapamil, using the logarithmic model.**

| **Mild Renal Impairment - 15 mg Recommended R, 120 V** | | | | | | | |
|---|---|---|---|---|---|---|---|
| | | | | | | | |

| **Logarithmic Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 9.2 | 8.9 | 11.2 |
| **90% CI, Upper Boundary** | 275 | 268 | 317 | | 14.4 | 13.8 | |
| **80% CI, Upper Boundary** | 275 | 268 | 317 | | | | |
| **IQR Upper** | 275 | 268 | 317 | | | | |
| **Median** | 275 | 268 | 317 | | | | |
| **Arithmetic Mean** | 275 | 268 | 317 | | | | |
| **Geometric Mean** | 275 | 268 | 317 | | | | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 109: Redosing regimens of rivaroxaban in response to various target steady state Cₘₐₓ values in subjects with mild renal impairment where the recommended dose is 15 mg rivaroxaban, 240 mg concomitant verapamil, using the logarithmic model.**

| **Mild Renal Impairment - 15 mg Recommended R, 240 V** | | | | | | | |
|---|---|---|---|---|---|---|---|
| | | | | | | | |

| **Logarithmic Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 8.9 | 8.6 | 10.8 |
| **90% CI, Upper Boundary** | 275 | 268 | 317 | | 13.8 | 13.2 | |
| **80% CI, Upper Boundary** | 275 | 268 | 317 | | | 14.5 | |
| **IQR Upper** | 275 | 268 | 317 | | | | |
| **Median** | 275 | 268 | 317 | | | | |
| **Arithmetic Mean** | 275 | 268 | 317 | | | | |
| **Geometric Mean** | 275 | 268 | 317 | | | | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 110: Redosing regimens of rivaroxaban in response to various target steady state Cₘₐₓ values in subjects with mild renal impairment where the recommended dose is 15 mg rivaroxaban, 360 mg concomitant verapamil, using the logarithmic model.**

| **Mild Renal Impairment - 15 mg Recommended R, 360 V** | | | | | | | |
|---|---|---|---|---|---|---|---|
| | | | | | | | |

| **Logarithmic Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 8.7 | 8.5 | 10.6 |
| **90% CI, Upper Boundary** | 275 | 268 | 317 | | 13.3 | 12.7 | |
| **80% CI, Upper Boundary** | 275 | 268 | 317 | | 14.7 | 14.0 | |
| **IQR Upper** | 275 | 268 | 317 | | | | |
| **Median** | 275 | 268 | 317 | | | | |
| **Arithmetic Mean** | 275 | 268 | 317 | | | | |
| **Geometric Mean** | 275 | 268 | 317 | | | | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 111: Redosing regimens of rivaroxaban in response to various target steady state Cₘₐₓ values in subjects with mild renal impairment where the recommended dose is 20 mg rivaroxaban, no concomitant verapamil, using the logarithmic model.**

| **Mild Renal Impairment - 20 mg Recommended R, 0 V** | | | | | | | |
|---|---|---|---|---|---|---|---|
| | | | | | | | |

| **Logarithmic Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 7.3 | 7.1 | 8.6 |
| **90% CI, Upper Boundary** | 275 | 268 | 317 | | 11.4 | 10.9 | 14.4 |
| **80% CI, Upper Boundary** | 275 | 268 | 317 | | 12.5 | 12.0 | 16.1 |
| **IQR Upper** | 275 | 268 | 317 | | 15.1 | 14.4 | 19.9 |
| **Median** | 275 | 268 | 317 | | 19.1 | 18.1 | |
| **Arithmetic Mean** | 275 | 268 | 317 | | 18.3 | 17.4 | |
| **Geometric Mean** | 275 | 268 | 317 | | 18.9 | 18.0 | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 112: Redosing regimens of rivaroxaban in response to various target steady state Cₘₐₓ values in subjects with mild renal impairment where the recommended dose is 20 mg rivaroxaban, 120 mg concomitant verapamil, using the logarithmic model.**

| **Mild Renal Impairment - 20 mg Recommended R, 120 V** | | | | | | | |
|---|---|---|---|---|---|---|---|
| | | | | | | | |

| **Logarithmic Redosing Cmax** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 6.5 | 6.3 | 7.5 |
| **90% CI, Upper Boundary** | 275 | 268 | 317 | | 9.1 | 8.8 | 11.1 |
| **80% CI, Upper Boundary** | 275 | 268 | 317 | | 9.8 | 9.5 | 12.2 |
| **IQR Upper** | 275 | 268 | 317 | | 11.5 | 11.1 | 14.6 |
| **Median** | 275 | 268 | 317 | | 14.1 | 13.4 | 18.3 |
| **Arithmetic Mean** | 275 | 268 | 317 | | 13.6 | 13.0 | 17.6 |
| **Geometric Mean** | 275 | 268 | 317 | | 13.9 | 13.3 | 18.2 |
| **IQR Lower** | 275 | 268 | 317 | | 17.4 | 16.6 | |
| **80% CI, Lower Boundary** | 275 | 268 | 317 | | | 19.9 | |
| **90% CI, Lower Boundary** | 275 | 268 | 317 | | | | |
| **Minimum** | 275 | 268 | 317 | | | | |

**Table 113: Redosing regimens of rivaroxaban in response to various target steady state Cₘₐₓ values in subjects with mild renal impairment where the recommended dose is 20 mg rivaroxaban, 240 mg concomitant verapamil, using the logarithmic model.**

| **Mild Renal Impairment - 20 mg Recommended R, 240 V** | | | | | | | |
|---|---|---|---|---|---|---|---|
| | | | | | | | |

| **Logarithmic Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 6.4 | 6.2 | 7.3 |
| **90% CI, Upper Boundary** | 275 | 268 | 317 | | 8.8 | 8.5 | 10.6 |
| **80% CI, Upper Boundary** | 275 | 268 | 317 | | 9.4 | 9.1 | 11.6 |
| **IQR Upper** | 275 | 268 | 317 | | 11.0 | 10.6 | 13.8 |
| **Median** | 275 | 268 | 317 | | 13.3 | 12.8 | 17.2 |
| **Arithmetic Mean** | 275 | 268 | 317 | | 12.9 | 12.4 | 16.6 |
| **Geometric Mean** | 275 | 268 | 317 | | 13.2 | 12.7 | 17.1 |
| **IQR Lower** | 275 | 268 | 317 | | 16.4 | 15.6 | |
| **80% CI, Lower Boundary** | 275 | 268 | 317 | | 19.8 | 18.8 | |
| **90% CI, Lower Boundary** | 275 | 268 | 317 | | | | |
| **Minimum** | 275 | 268 | 317 | | | | |

**Table 114: Redosing regimens of rivaroxaban in response to various target steady state Cₘₐₓ values in subjects with mild renal impairment where the recommended dose is 20 mg rivaroxaban, 240 mg concomitant verapamil, using the logarithmic model.**

| **Mild Renal Impairment - 20 mg Recommended R, 360 V** | | | | | | | |
|---|---|---|---|---|---|---|---|
| | | | | | | | |

| **Logarithmic Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 6.3 | 6.1 | 7.2 |
| **90% CI, Upper Boundary** | 275 | 268 | 317 | | 8.6 | 8.3 | 10.3 |
| **80% CI, Upper Boundary** | 275 | 268 | 317 | | 9.2 | 8.9 | 11.2 |
| **IQR Upper** | 275 | 268 | 317 | | 10.6 | 10.3 | 13.3 |
| **Median** | 275 | 268 | 317 | | 12.9 | 12.4 | 16.6 |
| **Arithmetic Mean** | 275 | 268 | 317 | | 12.5 | 12.0 | 16.0 |
| **Geometric Mean** | 275 | 268 | 317 | | 12.8 | 12.3 | 16.4 |
| **IQR Lower** | 275 | 268 | 317 | | 15.8 | 15.0 | |
| **80% CI, Lower Boundary** | 275 | 268 | 317 | | 18.9 | 18.0 | |
| **90% CI, Lower Boundary** | 275 | 268 | 317 | | | | |
| **Minimum** | 275 | 268 | 317 | | | | |

**Table 115: Redosing regimens of rivaroxaban in response to various target steady state Cₘₐₓ values in subjects with moderate renal impairment where the recommended dose is 15 mg rivaroxaban, no concomitant verapamil, using the logarithmic model.**

| **Moderate Renal Impairment - 15 mg Recommended R, 0 V** | | | | | | | |
|---|---|---|---|---|---|---|---|
| | | | | | | | |

| **Logarithmic Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 10.0 | 9.7 | 12.4 |
| **90% CI, Upper Boundary** | 275 | 268 | 317 | | | | |
| **80% CI, Upper Boundary** | 275 | 268 | 317 | | | | |
| **IQR Upper** | 275 | 268 | 317 | | | | |
| **Median** | 275 | 268 | 317 | | | | |
| **Arithmetic Mean** | 275 | 268 | 317 | | | | |
| **Geometric Mean** | 275 | 268 | 317 | | | | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 116: Redosing regimens of rivaroxaban in response to various target steady state Cₘₐₓ values in subjects with moderate renal impairment where the recommended dose is 15 mg rivaroxaban, 120 mg concomitant verapamil, using the logarithmic model.**

| **Moderate Renal Impairment - 15 mg Recommended R, 120 V** | | | | | | | |
|---|---|---|---|---|---|---|---|
| | | | | | | | |

| **Logarithmic Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 8.1 | 7.9 | 9.8 |
| **90% CI, Upper Boundary** | 275 | 268 | 317 | | 13.8 | 13.2 | |
| **80% CI, Upper Boundary** | 275 | 268 | 317 | | | 14.9 | |
| **IQR Upper** | 275 | 268 | 317 | | | | |
| **Median** | 275 | 268 | 317 | | | | |
| **Arithmetic Mean** | 275 | 268 | 317 | | | | |
| **Geometric Mean** | 275 | 268 | 317 | | | | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 117: Redosing regimens of rivaroxaban in response to various target steady state Cₘₐₓ values in subjects with moderate renal impairment where the recommended dose is 15 mg rivaroxaban, 240 mg concomitant verapamil, using the logarithmic model.**

| **Moderate Renal Impairment - 15 mg Recommended R, 240 V** | | | | | | | |
|---|---|---|---|---|---|---|---|
| | | | | | | | |

| **Logarithmic Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 7.8 | 7.6 | 9.3 |
| **90% CI, Upper Boundary** | 275 | 268 | 317 | | 13.2 | 12.6 | |
| **80% CI, Upper Boundary** | 275 | 268 | 317 | | 14.6 | 14.0 | |
| **IQR Upper** | 275 | 268 | 317 | | | | |
| **Median** | 275 | 268 | 317 | | | | |
| **Arithmetic Mean** | 275 | 268 | 317 | | | | |
| **Geometric Mean** | 275 | 268 | 317 | | | | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 118: Redosing regimens of rivaroxaban in response to various target steady state Cₘₐₓ values in subjects with moderate renal impairment where the recommended dose is 15 mg rivaroxaban, 360 mg concomitant verapamil, using the logarithmic model.**

| **Moderate Renal Impairment - 15 mg Recommended R, 360 V** | | | | | | | |
|---|---|---|---|---|---|---|---|
| | | | | | | | |

| **Logarithmic Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 7.6 | 7.4 | 9.1 |
| **90% CI, Upper Boundary** | 275 | 268 | 317 | | 12.7 | 12.2 | |
| **80% CI, Upper Boundary** | 275 | 268 | 317 | | 14.1 | 13.4 | |
| **IQR Upper** | 275 | 268 | 317 | | | | |
| **Median** | 275 | 268 | 317 | | | | |
| **Arithmetic Mean** | 275 | 268 | 317 | | | | |
| **Geometric Mean** | 275 | 268 | 317 | | | | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 119: Redosing regimens of rivaroxaban in response to various target steady state Cₘₐₓ values in subjects with moderate renal impairment where the recommended dose is 20 mg rivaroxaban, no concomitant verapamil, using the logarithmic model.**

| **Moderate Renal Impairment - 20 mg Recommended R, 0 V** | | | | | | | |
|---|---|---|---|---|---|---|---|
| | | | | | | | |

| **Logarithmic Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 6.9 | 6.8 | 8.1 |
| **90% CI, Upper Boundary** | 275 | 268 | 317 | | 11.1 | 10.7 | 13.9 |
| **80% CI, Upper Boundary** | 275 | 268 | 317 | | 12.2 | 11.7 | 15.6 |
| **IQR Upper** | 275 | 268 | 317 | | 14.6 | 14.0 | 19.2 |
| **Median** | 275 | 268 | 317 | | 18.2 | 17.3 | |
| **Arithmetic Mean** | 275 | 268 | 317 | | 17.5 | 16.7 | |
| **Geometric Mean** | 275 | 268 | 317 | | 18.0 | 17.1 | |
| **IQR Lower** | 275 | 268 | 317 | | | | |
| **80% CI, Lower Boundary** | 275 | 268 | 317 | | | | |
| **90% CI, Lower Boundary** | 275 | 268 | 317 | | | | |
| **Minimum** | 275 | 268 | 317 | | | | |

**Table 120: Redosing regimens of rivaroxaban in response to various target steady state Cₘₐₓ values in subjects with moderate renal impairment where the recommended dose is 20 mg rivaroxaban, 120 mg concomitant verapamil, using the logarithmic model.**

| **Moderate Renal Impairment - 20 mg Recommended R, 120 V** | | | | | | | |
|---|---|---|---|---|---|---|---|
| | | | | | | | |

| **Logarithmic Redosing Cₘₐₓ.** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 6.0 | 5.8 | 6.8 |
| **90% CI, Upper Boundary** | 275 | 268 | 317 | | 8.8 | 8.5 | 10.7 |
| **80% CI, Upper Boundary** | 275 | 268 | 317 | | 9.6 | 9.3 | 11.8 |
| **IQR Upper** | 275 | 268 | 317 | | 11.1 | 10.7 | 13.9 |
| **Median** | 275 | 268 | 317 | | 13.3 | 12.8 | 17.2 |
| **Arithmetic Mean** | 275 | 268 | 317 | | 12.9 | 12.4 | 16.6 |
| **Geometric Mean** | 275 | 268 | 317 | | 13.2 | 12.7 | 17.1 |
| **IQR Lower** | 275 | 268 | 317 | | 16.0 | 15.2 | |
| **80% CI, Lower Boundary** | 275 | 268 | 317 | | 19.7 | 18.6 | |
| **90% CI, Lower Boundary** | 275 | 268 | 317 | | | | |
| **Minimum** | 275 | 268 | 317 | | | | |

**Table 121: Redosing regimens of rivaroxaban in response to various target steady state Cₘₐₓ values in subjects with moderate renal impairment where the recommended dose is 20 mg rivaroxaban, 240 mg concomitant verapamil, using the logarithmic model.**

| **Moderate Renal Impairment - 20 mg Recommended R, 240 V** | | | | | | | |
|---|---|---|---|---|---|---|---|
| | | | | | | | |

| **Logarithmic Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 5.8 | 5.7 | 6.6 |
| **90% CI, Upper Boundary** | 275 | 268 | 317 | | 8.5 | 8.2 | 10.2 |
| **80% CI, Upper Boundary** | 275 | 268 | 317 | | 9.2 | 8.9 | 11.2 |
| **IQR Upper** | 275 | 268 | 317 | | 10.6 | 10.2 | 13.2 |
| **Median** | 275 | 268 | 317 | | 12.7 | 12.1 | 16.2 |
| **Arithmetic Mean** | 275 | 268 | 317 | | 12.3 | 11.8 | 15.7 |
| **Geometric Mean** | 275 | 268 | 317 | | 12.6 | 12.1 | 16.1 |
| **IQR Lower** | 275 | 268 | 317 | | 15.1 | 14.4 | 19.9 |
| **80% CI, Lower Boundary** | 275 | 268 | 317 | | 18.4 | 17.4 | |
| **90% CI, Lower Boundary** | 275 | 268 | 317 | | | 19.7 | |
| **Minimum** | 275 | 268 | 317 | | | | |

**Table 122: Redosing regimens of rivaroxaban in response to various target steady state Cₘₐₓ values in subjects with moderate renal impairment where the recommended dose is 20 mg rivaroxaban, 360 mg concomitant verapamil, using the logarithmic model.**

| **Moderate Renal Impairment - 20 mg Recommended R, 360 V** | | | | | | | |
|---|---|---|---|---|---|---|---|
| | | | | | | | |

| **Logarithmic Redosing Cₘₐₓ** | | | | | | | |
|---|---|---|---|---|---|---|---|
| | | | | | | | |

| **Group** | **Target 1** | **Target 2** | **Target 3** | | **Dose 1** | **Dose 2** | **Dose 3** |
|---|---|---|---|---|---|---|---|
| **Maximum** | 275 | 268 | 317 | | 5.7 | 5.6 | 6.4 |
| **90% CI, Upper Boundary** | 275 | 268 | 317 | | 8.3 | 8.0 | 9.9 |
| **80% CI, Upper Boundary** | 275 | 268 | 317 | | 8.9 | 8.6 | 10.8 |
| **IQR Upper** | 275 | 268 | 317 | | 10.3 | 9.9 | 12.8 |
| **Median** | 275 | 268 | 317 | | 12.3 | 11.8 | 15.7 |
| **Arithmetic Mean** | 275 | 268 | 317 | | 11.9 | 11.4 | 15.1 |
| **Geometric Mean** | 275 | 268 | 317 | | 12.2 | 11.7 | 15.5 |
| **IQR Lower** | 275 | 268 | 317 | | 14.6 | 13.9 | 19.1 |
| **80% CI, Lower Boundary** | 275 | 268 | 317 | | 17.7 | 16.8 | |
| **90% CI, Lower Boundary** | 275 | 268 | 317 | | 19.9 | 18.9 | |
| **Minimum** | 275 | 268 | 317 | | | | |

Additionally, the model system was used to iteratively calculate dosage levels where ranges from 97.5-75% of subjects would have steady state AUC and Cmax levels below the defined safety target levels. These results are included in Tables 123 and 124 below.

**Table 123: Redosing regimens of rivaroxaban, by percentage of patients below the target steady state AUC values in subjects with no to moderate renal impairment, for varying levels of concomitant verapamil.**

| **PBPK Calculated Dosing by Percentile Matching Safety Target** | | | | | |
|---|---|---|---|---|---|
| Renal State State | Verapamil Dose (mg) | Percentile | Target Levels | | |
| | | | **3,972** | **3,404** | **2,448** |
| Normal | 0 | 75.0% | | | 15 |
| | 0 | 90.0% | | | 13 |
| | 0 | 95.0% | | | 12 |
| | 0 | 97.5% | | 17 | 11 |
| | 120 | 75.0% | 18 | 16 | 10 |
| | 120 | 90.0% | 16 | 14 | 9 |
| | 120 | 95.0% | 15 | 13 | 9 |
| | 120 | 97.5% | 14 | 12 | 8 |
| | 240 | 75.0% | 17 | 14 | 10 |
| | 240 | 90.0% | 15 | 13 | 9 |
| | 240 | 95.0% | 14 | 12 | 8 |
| | 240 | 97.5% | 13 | 11 | 8 |
| | 360 | 75.0% | 16 | 14 | 9 |
| | 360 | 90.0% | 14 | 12 | 8 |
| | 360 | 95.0% | 13 | 12 | 8 |
| | 360 | 97.5% | 12 | 11 | 7 |
| Mild Impairment | 0 | 75.0% | | | 14 |
| | 0 | 90.0% | | | 12 |
| | 0 | 95.0% | | 17 | 11 |
| | 0 | 97.5% | 18 | 15 | 10 |
| | 120 | 75.0% | 16 | 14 | 10 |
| | 120 | 90.0% | 14 | 13 | 9 |
| | 120 | 95.0% | 13 | 12 | 8 |
| | 120 | 97.5% | 13 | 11 | 8 |
| | 240 | 75.0% | 15 | 13 | 9 |
| | 240 | 90.0% | 13 | 12 | 8 |
| | 240 | 95.0% | 12 | 11 | 8 |
| | 240 | 97.5% | 12 | 10 | 7 |
| | 360 | 75.0% | 15 | 13 | 9 |
| | 360 | 90.0% | 13 | 11 | 8 |
| | 360 | 95.0% | 12 | 11 | 7 |
| | 360 | 97.5% | 11 | 10 | 7 |
| Moderate Impairment | 0 | 75.0% | | | 13 |
| | 0 | 90.0% | | 17 | 11 |
| | 0 | 95.0% | 18 | 16 | 10 |
| | 0 | 97.5% | 16 | 14 | 10 |
| | 120 | 75.0% | 15 | 13 | 9 |
| | 120 | 90.0% | 13 | 12 | 8 |
| | 120 | 95.0% | 12 | 11 | 7 |
| | 120 | 97.5% | 11 | 10 | 7 |
| | 240 | 75.0% | 14 | 12 | 9 |
| | 240 | 90.0% | 12 | 11 | 8 |
| | 240 | 95.0% | 11 | 10 | 7 |
| | 240 | 97.5% | 11 | 9 | 7 |
| | 360 | 75.0% | 14 | 12 | 8 |
| | 360 | 90.0% | 12 | 10 | 7 |
| | 360 | 95.0% | 11 | 10 | 7 |
| | 360 | 97.5% | 10 | 9 | 6 |

**Table 124: Redosing regimens of rivaroxaban, by percentage of patients below the target steady state Cₘₐₓ values in subjects with no to moderate renal impairment, for varying levels of concomitant verapamil**

| **PBPK Calculated Dosing by Percentile Matching Safety Target** | | | | | |
|---|---|---|---|---|---|
| Renal State State | Verapamil Dose (mg) | Percentile | Target Levels | | |
| | | | **275** | **268** | **317** |
| Normal | 0 | 75.0% | | 18 | |
| | 0 | 90.0% | 16 | 15 | |
| | 0 | 95.0% | 15 | 14 | 18 |
| | 0 | 97.5% | 14 | 13 | 16 |
| | 120 | 75.0% | 15 | 15 | |
| | 120 | 90.0% | 13 | 13 | 16 |
| | 120 | 95.0% | 12 | 12 | 14 |
| | 120 | 97.5% | 11 | 11 | 13 |
| | 240 | 75.0% | 15 | 14 | 18 |
| | 240 | 90.0% | 13 | 12 | 15 |
| | 240 | 95.0% | 12 | 11 | 14 |
| | 240 | 97.5% | 11 | 11 | 13 |
| | 360 | 75.0% | 14 | 14 | 17 |
| | 360 | 90.0% | 12 | 12 | 15 |
| | 360 | 95.0% | 11 | 11 | 13 |
| | 360 | 97.5% | 11 | 10 | 13 |
| Mild Impairment | 0 | 75.0% | 18 | 17 | |
| | 0 | 90.0% | 15 | 15 | |
| | 0 | 95.0% | 14 | 14 | 17 |
| | 0 | 97.5% | 13 | 13 | 16 |
| | 120 | 75.0% | 14 | 14 | 17 |
| | 120 | 90.0% | 13 | 12 | 15 |
| | 120 | 95.0% | 12 | 12 | 14 |
| | 120 | 97.5% | 11 | 11 | 13 |
| | 240 | 75.0% | 14 | 13 | 16 |
| | 240 | 90.0% | 12 | 12 | 14 |
| | 240 | 95.0% | 11 | 11 | 14 |
| | 240 | 97.5% | 11 | 11 | 13 |
| | 360 | 75.0% | 13 | 13 | 16 |
| | 360 | 90.0% | 12 | 11 | 14 |
| | 360 | 95.0% | 11 | 11 | 13 |
| | 360 | 97.5% | 11 | 10 | 12 |
| Moderate Impairment | 0 | 75.0% | 17 | 17 | |
| | 0 | 90.0% | 15 | 14 | 18 |
| | 0 | 95.0% | 14 | 13 | 17 |
| | 0 | 97.5% | 13 | 13 | 16 |
| | 120 | 75.0% | 14 | 13 | 17 |
| | 120 | 90.0% | 12 | 12 | 15 |
| | 120 | 95.0% | 11 | 11 | 13 |
| | 120 | 97.5% | 11 | 10 | 13 |
| | 240 | 75.0% | 13 | 13 | 16 |
| | 240 | 90.0% | 12 | 11 | 14 |
| | 240 | 95.0% | 11 | 11 | 13 |
| | 240 | 97.5% | 10 | 10 | 12 |
| | 360 | 75.0% | 13 | 13 | 16 |
| | 360 | 90.0% | 11 | 11 | 14 |
| | 360 | 95.0% | 11 | 10 | 13 |
| | 360 | 97.5% | 10 | 10 | 12 |

Each of the rivaroxaban redosing values specified in the above tables is an aspect of the methods of the present disclosure, wherein a patient having the specified renal status, and concomitantly (or not as indicated) administered verapamil, may be re-dosed at the indicated level (or to a level rounded to the nearest mg or 0.5 mg).

## Claims

1. Rivaroxaban for use in a method of reducing the risk of stroke and systemic embolism in a patient with nonvalvular atrial fibrillation comprising:
(a) administering about 100 to about 480 mg verapamil daily to the patient; and
(b) administering a dose of rivaroxaban to the patient which is less than the dose recommended for that patient if the patient was not concomitantly administered verapamil, wherein the dose of rivaroxaban recommended for an otherwise identical patient who is not concomitantly administered verapamil is 20 mg and the method comprises administering a dose of about 10 mg, about 12.5 mg or about 15 mg rivaroxaban to the patient;
wherein the patient has a CL_{Cr} of 50-79 mL/min; and
wherein after administering the rivaroxaban, the patient exhibits one or more of the following:
a steady state rivaroxaban AUC of no more than about 3792 µg•hr/L;
a steady state Cₘₐₓ of no more than about 317 ng/mL;
a risk of major bleeding of no more than about 4.5%; or
a prothrombin time of 20-30 seconds.

2. Rivaroxaban for use in a method of reducing the risk of stroke and systemic embolism in a patient with nonvalvular atrial fibrillation comprising:
(a) administering about 100 to about 480 mg verapamil daily to the patient; and
(b) administering a dose of rivaroxaban to the patient which is less than the dose recommended for that patient if the patient was not concomitantly administered verapamil, wherein the dose of rivaroxaban recommended for an otherwise identical patient who is not concomitantly administered verapamil is 15 mg and the method comprises administering a dose of about 7.5 mg, about 10 mg or about 12.5 mg rivaroxaban to the patient;
wherein the patient has a CL_{Cr} of 15-49 mL/min; and
wherein after administering the rivaroxaban, the patient exhibits one or more of the following:
a steady state rivaroxaban AUC of no more than about 3792 µg•hr/L;
a steady state Cₘₐₓ of no more than about 317 ng/mL;
a risk of major bleeding of no more than about 4.5%; or
a prothrombin time of 20-30 seconds.

3. Rivaroxaban for the use of any one of the preceding claims, wherein after administering the rivaroxaban, the patient exhibits the following:
a steady state rivaroxaban AUC of no more than about 3792 µg•hr/L;
a steady state Cₘₐₓ of no more than about 317 ng/mL;
a risk of major bleeding of no more than about 4.5%; and
a prothrombin time of 20-30 seconds.

4. Rivaroxaban for the use of any one of the preceding claims, wherein the method comprises administering a daily verapamil dose of 120, 240, 360, or 480 mg verapamil to the patient.

## Patentansprüche

1. Rivaroxaban zur Anwendung in einem Verfahren zur Reduktion des Risikos von Schlaganfall und systemischer Embolie bei einem Patienten mit nichtvalvulärem Vorhofflimmern, umfassend:
(a) Verabreichung von etwa 100 bis etwa 480 mg Verapamil täglich an den Patienten; und
(b) Verabreichung einer Rivaroxaban-Dosis an den Patienten, die geringer ist als die für diesen Patienten empfohlene Dosis, wenn dem Patienten nicht gleichzeitig Verapamil verabreicht würde, wobei die Rivaroxaban-Dosis, die einem ansonsten identischen Patienten empfohlen wird, dem nicht gleichzeitig Verapamil verabreicht wird, 20 mg beträgt und das Verfahren die Verabreichung einer Dosis von etwa 10 mg, etwa 12,5 mg oder etwa 15 mg Rivaroxaban an den Patienten umfasst;
wobei der Patient eine CL_{Cr} von 50-79 ml/min aufweist; und
wobei der Patient nach der Verabreichung des Rivaroxaban eines oder mehrere der Folgenden zeigt:
eine Rivaroxaban-AUC im Steady-State von höchstens etwa 3792 µg·h/l;
eine Cₘₐₓ im Steady-State von höchstens etwa 317 ng/ml;
ein Risiko schwererer Blutungen von höchstens etwa 4,5%; oder
eine Prothrombinzeit von 20-30 Sekunden.

2. Rivaroxaban zur Anwendung in einem Verfahren zur Reduktion des Risikos von Schlaganfall und systemischer Embolie bei einem Patienten mit nichtvalvulärem Vorhofflimmern, umfassend:
(a) Verabreichung von etwa 100 bis etwa 480 mg Verapamil täglich an den Patienten; und
(b) Verabreichung einer Rivaroxaban-Dosis an den Patienten, die geringer ist als die für diesen Patienten empfohlene Dosis, wenn dem Patienten nicht gleichzeitig Verapamil verabreicht würde, wobei die Rivaroxaban-Dosis, die einem ansonsten identischen Patienten empfohlen wird, dem nicht gleichzeitig Verapamil verabreicht wird, 15 mg beträgt und das Verfahren die Verabreichung einer Dosis von etwa 7,5 mg, etwa 10 mg oder etwa 12,5 mg Rivaroxaban an den Patienten umfasst;
wobei der Patient eine CL_{Cr} von 15-49 ml/min aufweist; und
wobei der Patient nach der Verabreichung des Rivaroxaban eines oder mehrere der Folgenden zeigt:
eine Rivaroxaban-AUC im Steady-State von höchstens etwa 3792 µg·h/l;
eine Cₘₐₓ im Steady-State von höchstens etwa 317 ng/ml;
ein Risiko schwererer Blutungen von höchstens etwa 4,5%; oder
eine Prothrombinzeit von 20-30 Sekunden.

3. Rivaroxaban zur Anwendung nach einem der vorhergehenden Ansprüche, wobei der Patient nach Verabreichung des Rivaroxaban Folgendes zeigt:
eine Rivaroxaban-AUC im Steady-State von höchstens etwa 3792 µg·h/l;
eine Cₘₐₓ im Steady-State von höchstens etwa 317 ng/ml;
ein Risiko schwererer Blutungen von höchstens etwa 4,5%; und
eine Prothrombinzeit von 20-30 Sekunden.

4. Rivaroxaban zur Anwendung nach einem der vorhergehenden Ansprüche, wobei das Verfahren die Verabreichung einer täglichen Verapamil-Dosis von 120, 240, 360 oder 480 mg Verapamil an den Patienten umfasst.

## Revendications

1. Rivaroxaban destiné à être utilisé dans une méthode de réduction du risque d'accident vasculaire cérébral et d'embolie systémique chez un patient présentant une fibrillation atriale non valvulaire comprenant :
(a) l'administration d'environ 100 à environ 480 mg de vérapamil quotidiennement au patient ; et
(b) l'administration au patient d'une dose de rivaroxaban qui est inférieure à la dose recommandée pour ce patient si du vérapamil n'était pas administré de manière concomitante au patient, la dose de rivaroxaban recommandée pour un patient par ailleurs identique auquel du vérapamil n'est pas administré de manière concomitante étant de 20 mg et la méthode comprenant l'administration d'une dose d'environ 10 mg, d'environ 12,5 mg ou d'environ 15 mg de rivaroxaban au patient ;
le patient ayant une CL_{Cr} de 50-79 ml/min ; et
après l'administration du rivaroxaban, le patient présentant ce qui suit :
une AUC du rivaroxaban à l'état stationnaire de pas plus d'environ 3792 µg•h/l;
une Cₘₐₓ à l'état stationnaire de pas plus d'environ 317 ng/ml ;
un risque de saignement majeur de pas plus d'environ 4,5 % ; et/ou
un temps de prothrombine de 20-30 secondes.

2. Rivaroxaban destiné à être utilisé dans une méthode de réduction du risque d'accident vasculaire cérébral et d'embolie systémique chez un patient présentant une fibrillation atriale non valvulaire comprenant :
(a) l'administration d'environ 100 à environ 480 mg de vérapamil quotidiennement au patient ; et
(b) l'administration au patient d'une dose de rivaroxaban qui est inférieure à la dose recommandée pour ce patient si du vérapamil n'était pas administré de manière concomitante au patient, la dose de rivaroxaban recommandée pour un patient par ailleurs identique auquel du vérapamil n'est pas administré de manière concomitante étant de 15 mg et la méthode comprenant l'administration d'une dose d'environ 7,5 mg, d'environ 10 mg ou d'environ 12,5 mg de rivaroxaban au patient ;
le patient ayant une CL_{Cr} de 15-49 ml/min ; et
après l'administration du rivaroxaban, le patient présentant ce qui suit :
une AUC du rivaroxaban à l'état stationnaire de pas plus d'environ 3792 µg•h/l ;
une Cₘₐₓ à l'état stationnaire de pas plus d'environ 317 ng/ml ;
un risque de saignement majeur de pas plus d'environ 4,5 % ; et/ou
un temps de prothrombine de 20-30 secondes.

3. Rivaroxaban destiné à être utilisé selon l'une quelconque des revendications précédentes, le patient, après l'administration du rivaroxaban, présentant ce qui suit :
une AUC du rivaroxaban à l'état stationnaire de pas plus d'environ 3792 µg•h/l ;
une Cₘₐₓ à l'état stationnaire de pas plus d'environ 317 ng/ml ;
un risque de saignement majeur de pas plus d'environ 4,5 % ; et
un temps de prothrombine de 20-30 secondes.

4. Rivaroxaban destiné à être utilisé selon l'une quelconque des revendications précédentes, la méthode comprenant l'administration d'une dose quotidienne de vérapamil de 120, 240, 360 ou 480 mg de vérapamil au patient.
